(19) Europäisches Patentamt
European Patent Office
Office européen des brevets

(11) EP 3 760 720 A1

# (12) EUROPEAN PATENT APPLICATION

(43) Date of publication:
**06.01.2021 Bulletin 2021/01**

(21) Application number: **20182238.4**

(22) Date of filing: **14.03.2014**

(51) Int Cl.:
***C12N 15/113*** *(2010.01)* ***A61K 31/7088*** *(2006.01)*
***A61P 21/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.03.2013 US 201361782706 P**

(62) Document number(s) of the earlier application(s) in accordance with Art. 76 EPC:
**18215621.6 / 3 495 485**
**14723583.2 / 2 970 964**

(71) Applicant: **Sarepta Therapeutics, Inc.**
**Cambridge, MA 02142 (US)**

(72) Inventors:
- **BESTWICK, Richard, K.**
  **Bend, OR 97707 (US)**
- **FRANK, Diane, Elizabeth**
  **Seattle, WA 98115 (US)**

(74) Representative: **D Young & Co LLP**
**120 Holborn**
**London EC1N 2DY (GB)**

Remarks:
- This application was filed on 25-06-2020 as a divisional application to the application mentioned under INID code 62.
- Claims filed after the date of filing of the application (Rule 68(4) EPC).

## (54) EXON SKIPPING COMPOSITIONS FOR TREATING MUSCULAR DYSTROPHY

(57) Antisense molecules capable of binding to a selected target site in the human dystrophin gene to induce exon 53 skipping are described.

EP 3 760 720 A1

## Description

### RELATED APPLICATIONS

This patent application claims the benefit of U.S. Provisional Patent Application Serial No. 61/782,706, filed March 14, 2013. The entire contents of the above-referenced provisional patent application are incorporate herein by reference.

### FIELD OF THE INVENTION

The present invention relates to novel antisense compounds and compositions suitable for facilitating exon skipping in the human dystrophin gene. It also provides methods for inducing exon skipping using the novel antisense compositions adapted for use in the methods of the invention.

### BACKGROUND OF THE INVENTION

Antisense technologies are being developed using a range of chemistries to affect gene expression at a variety of different levels (transcription, splicing, stability, translation). Much of that research has focused on the use of antisense compounds to correct or compensate for abnormal or disease-associated genes in a wide range of indications. Antisense molecules are able to inhibit gene expression with specificity, and because of this, many research efforts concerning oligonucleotides as modulators of gene expression have focused on inhibiting the expression of targeted genes or the function of cis-acting elements. The antisense oligonucleotides are typically directed against RNA, either the sense strand (e.g., mRNA), or minus-strand in the case of some viral RNA targets. To achieve a desired effect of specific gene down-regulation, the oligonucleotides generally either promote the decay of the targeted mRNA, block translation of the mRNA or block the function of cis-acting RNA elements, thereby effectively preventing either *de novo* synthesis of the target protein or replication of the viral RNA.

However, such techniques are not useful where the object is to up-regulate production of the native protein or compensate for mutations that induce premature termination of translation, such as nonsense or frame-shifting mutations. In these cases, the defective gene transcript should not be subjected to targeted degradation or steric inhibition, so the antisense oligonucleotide chemistry should not promote target mRNA decay or block translation.

In a variety of genetic diseases, the effects of mutations on the eventual expression of a gene can be modulated through a process of targeted exon skipping during the splicing process. The splicing process is directed by complex multi-component machinery that brings adjacent exon-intron junctions in pre-mRNA into close proximity and performs cleavage of phosphodiester bonds at the ends of the introns with their subsequent reformation between exons that are to be spliced together. This complex and highly precise process is mediated by sequence motifs in the pre-mRNA that are relatively short, semi-conserved RNA segments to which various nuclear splicing factors that are then involved in the splicing reactions bind. By changing the way the splicing machinery reads or recognizes the motifs involved in pre-mRNA processing, it is possible to create differentially spliced mRNA molecules. It has now been recognized that the majority of human genes are alternatively spliced during normal gene expression, although the mechanisms involved have not been identified. Bennett et al. (U.S. Patent No. 6,210,892) describe antisense modulation of wild-type cellular mRNA processing using antisense oligonucleotide analogs that do not induce RNAse H-mediated cleavage of the target RNA. This finds utility in being able to generate alternatively spliced mRNAs that lack specific exons (e.g., as described by (Sazani, Kole, et al. 2007) for the generation of soluble TNF superfamily receptors that lack exons encoding membrane spanning domains.

In cases where a normally functional protein is prematurely terminated because of mutations therein, a means for restoring some functional protein production through antisense technology has been shown to be possible through intervention during the splicing processes, and that if exons associated with disease-causing mutations can be specifically deleted from some genes, a shortened protein product can sometimes be produced that has similar biological properties of the native protein or has sufficient biological activity to ameliorate the disease caused by mutations associated with the exon (see e.g., Sierakowska, Sambade et al. 1996; Wilton, Lloyd et al. 1999; van Deutekom, Bremmer-Bout et al. 2001; Lu, Mann et al. 2003; Aartsma-Rus, Janson et al. 2004). Kole *et al.* (U.S. Patent Nos. 5,627,274; 5,916,808; 5,976,879; and 5,665,593) disclose methods of combating aberrant splicing using modified antisense oligonucleotide analogs that do not promote decay of the targeted pre-mRNA. Bennett et al. (U.S. Patent No. 6,210,892) describe antisense modulation of wild-type cellular mRNA processing also using antisense oligonucleotide analogs that do not induce RNAse H-mediated cleavage of the target RNA.

The process of targeted exon skipping is likely to be particularly useful in long genes where there are many exons and introns, where there is redundancy in the genetic constitution of the exons or where a protein is able to function without one or more particular exons. Efforts to redirect gene processing for the treatment of genetic diseases associated

with truncations caused by mutations in various genes have focused on the use of antisense oligonucleotides that either: (1) fully or partially overlap with the elements involved in the splicing process; or (2) bind to the pre-mRNA at a position sufficiently close to the element to disrupt the binding and function of the splicing factors that would normally mediate a particular splicing reaction which occurs at that element.

Duchenne muscular dystrophy (DMD) is caused by a defect in the expression of the protein dystrophin. The gene encoding the protein contains 79 exons spread out over more than 2 million nucleotides of DNA. Any exonic mutation that changes the reading frame of the exon, or introduces a stop codon, or is characterized by removal of an entire out of frame exon or exons, or duplications of one or more exons, has the potential to disrupt production of functional dystrophin, resulting in DMD.

A less severe form of muscular dystrophy, Becker muscular dystrophy (BMD) has been found to arise where a mutation, typically a deletion of one or more exons, results in a correct reading frame along the entire dystrophin transcript, such that translation of mRNA into protein is not prematurely terminated. If the joining of the upstream and downstream exons in the processing of a mutated dystrophin pre-mRNA maintains the correct reading frame of the gene, the result is an mRNA coding for a protein with a short internal deletion that retains some activity, resulting in a Becker phenotype.

For many years it has been known that deletions of an exon or exons which do not alter the reading frame of a dystrophin protein would give rise to a BMD phenotype, whereas an exon deletion that causes a frame-shift will give rise to DMD (Monaco, Bertelson et al. 1988). In general, dystrophin mutations including point mutations and exon deletions that change the reading frame and thus interrupt proper protein translation result in DMD. It should also be noted that some BMD and DMD patients have exon deletions covering multiple exons.

Modulation of mutant dystrophin pre-mRNA splicing with antisense oligoribonucleotides has been reported both *in vitro* and *in vivo* (see *e.g.,* Matsuo, Masumura et al. 1991; Takeshima, Nishio et al. 1995; Pramono, Takeshima et al. 1996; Dunckley, Eperon et al. 1997; Dunckley, Manoharan et al. 1998; Errington, Mann et al. 2003).

The first example of specific and reproducible exon skipping in the *mdx* mouse model was reported by Wilton *et al.* (Wilton, Lloyd et al. 1999). By directing an antisense molecule to the donor splice site, consistent and efficient exon 23 skipping was induced in the dystrophin mRNA within 6 hours of treatment of the cultured cells. Wilton *et al.* also describe targeting the acceptor region of the mouse dystrophin pre-mRNA with longer antisense oligonucleotides. While the first antisense oligonucleotide directed at the intron 23 donor splice site induced consistent exon skipping in primary cultured myoblasts, this compound was found to be much less efficient in immortalized cell cultures expressing higher levels of dystrophin. However, with refined targeting and antisense oligonucleotide design, the efficiency of specific exon removal was increased by almost an order of magnitude (Mann, Honeyman et al. 2002).

Recent studies have begun to address the challenge of achieving sustained dystrophin expression accompanied by minimal adverse effects in tissues affected by the absence of dystrophin. Intramuscular injection of an antisense oligonucleotide targeted to exon 51 (PRO051) into the tibialis anterior muscle in four patients with DMD resulted in specific skipping of exon 51 without any clinically apparent adverse effects (Mann, Honeyman et al. 2002; van Deutekom, Janson et al. 2007). Studies looking at systemic delivery of an antisense phosphorodiamidate morpholino oligomer conjugated to a cell-penetrating peptide (PPMO) targeted to exon 23 in *mdx* mice produced high and sustained dystrophin protein production in skeletal and cardiac muscles without detectable toxicity (Jearawiriyapaisarn, Moulton et al. 2008; Wu, Moulton et al. 2008; Yin, Moulton et al. 2008).

Recent clinical trials testing the safety and efficacy of splice switching oligonucleotides (SSOs) for the treatment of DMD are based on SSO technology to induce alternative splicing of pre-mRNAs by steric blockade of the spliceosome (Cirak *et al.*, 2011; Goemans *et al.*, 2011; Kinali *et al.*, 2009; van Deutekom *et al.*, 2007).

Despite these successes, there remains a need for improved antisense oligomers targeted to multiple dystrophin exons and improved delivery compositions and methods for DMD therapeutic applications.

## SUMMARY OF THE INVENTION

In one aspect, the invention provides an antisense oligomer of 20-50 nucleotides in length capable of binding a selected target to induce exon skipping in the human dystrophin gene, wherein the antisense oligomer comprises a sequence of bases that specifically hybridizes to an exon 53 target region selected from the group consisting of H53A(+36+60), H53A(+30+57), H53A (+30+56), H53A(+30+55) and H53A(+33+57), wherein the bases of the oligomer are linked to morpholino ring structures, and wherein the morpholino ring structures are joined by phosphorous-containing intersubunit linkages joining a morpholino nitrogen of one ring structure to a 5' exocyclic carbon of an adjacent ring structure. In one embodiment, the antisense oligomer comprises a sequence of bases designated as SEQ ID NOs: 1-5. In another embodiment, the antisense oligomer is about 20 to 30 nucleotides in length or about 25 to 28 nucleotides in length. In yet another embodiment, the sequence is 25 nucleotides and consists of a sequence selected from SEQ ID NOs: 1-5. In one embodiment, the invention provides an antisense oligomer that does not activate RNase H.

In another aspect, the invention provides an antisense oligomer that is chemically linked to one or more moieties

or conjugates that enhance the activity, cellular distribution, or cellular uptake of the antisense oligomer, such as, for example a polyethylene glycol molecule. In other embodiments, the antisense oligomer is conjugated to an arginine-rich peptide such as a sequence selected from SEQ ID NOS: 16-31.

In yet another aspect, the invention provides an antisense oligomer of 20-50 nucleotides in length capable of binding a selected target to induce exon skipping in the human dystrophin gene, wherein the antisense oligomer comprises a sequence of bases that specifically hybridizes to an exon 53 target region selected from the group consisting of H53A(+36+60), H53A(+30+57), H53A (+30+56), H53A(+30+55) and H53A(+33+57), wherein the bases of the oligomer are linked to morpholino ring structures, and wherein the morpholino ring structures are joined by substantially uncharged phosphorus-containing intersubunit linkages joining a morpholino nitrogen of one ring structure to a 5' exocyclic carbon of an adjacent ring structure. In one embodiment, 5%-35% of the linkages of the antisense oligomer are positively charged. In another embodiment, the intersubunit linkages of the antisense oligomer are uncharged and interspersed with linkages that are positively charged at physiological pH, wherein the total number of positively charged linkages is between 2 and no more than half of the total number of linkages. In some embodiments, the antisense oligomer comprises morpholino ring structures and phosphorodiamidate intersubunit linkages. In some embodiments, the antisense oligomer is modified with a pendant cationic group.

In another aspect, the invention provides an antisense oligomer, wherein the oligomer has

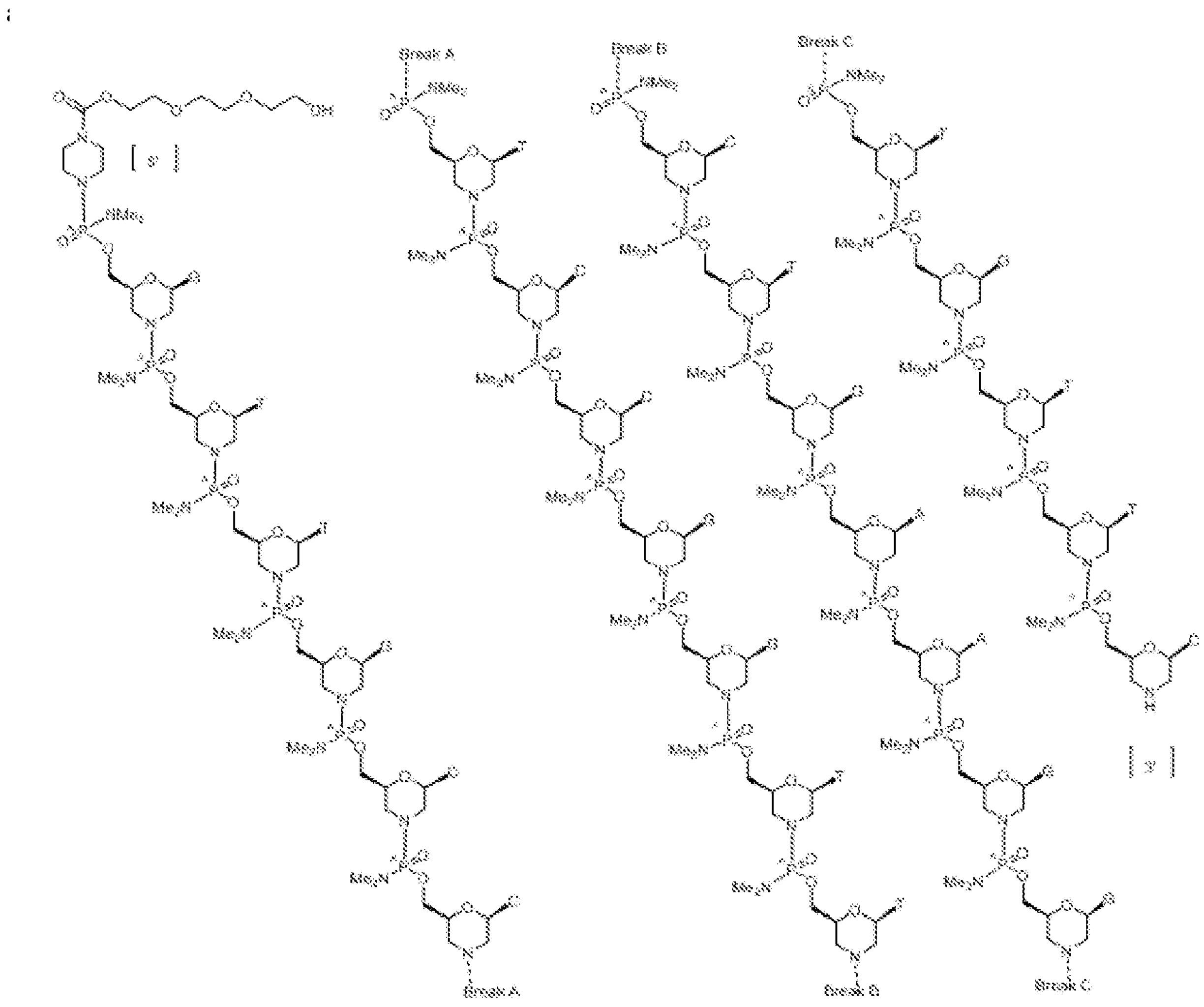

H53A(+36+60),

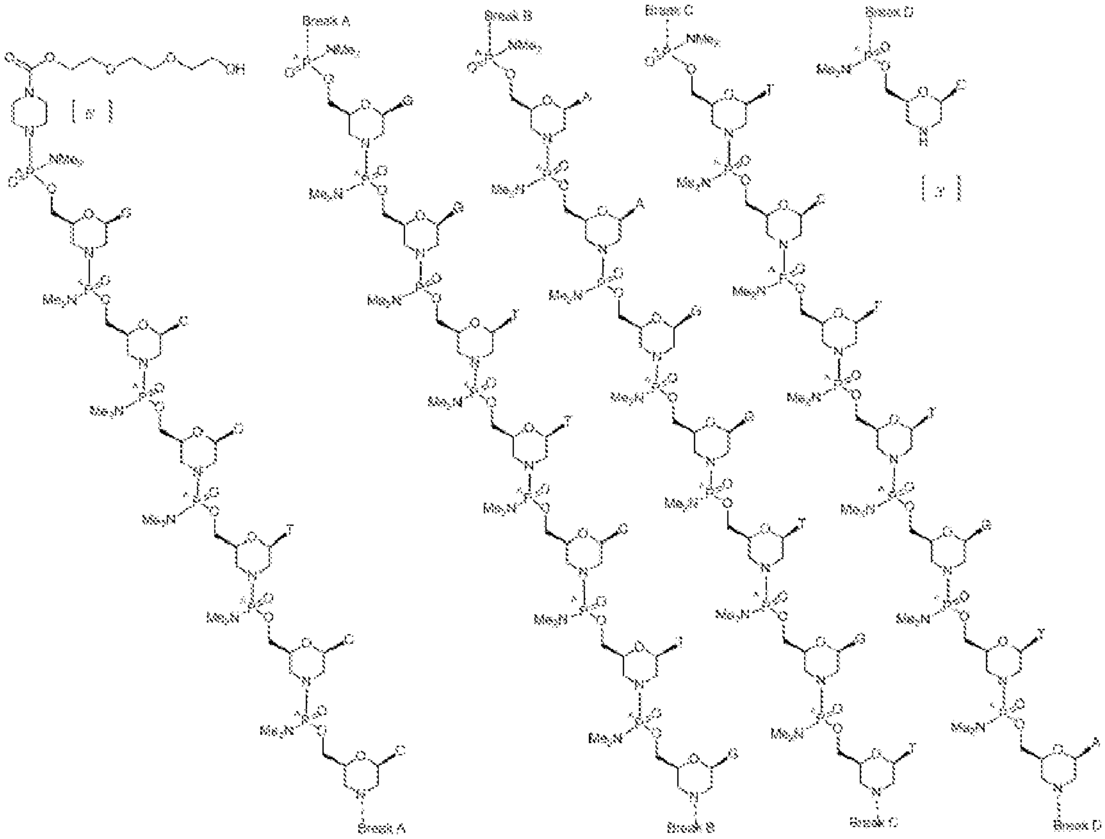

H53A(+30+57),

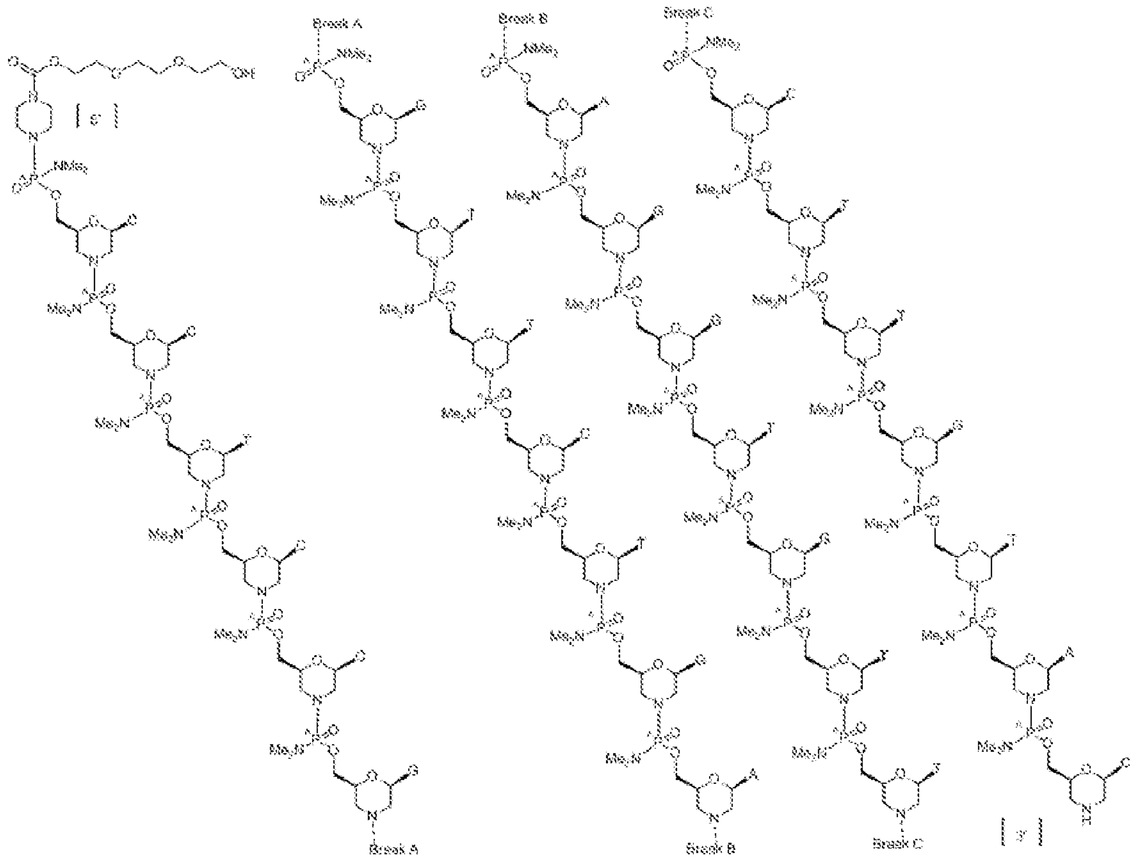

H53A(+30+56),

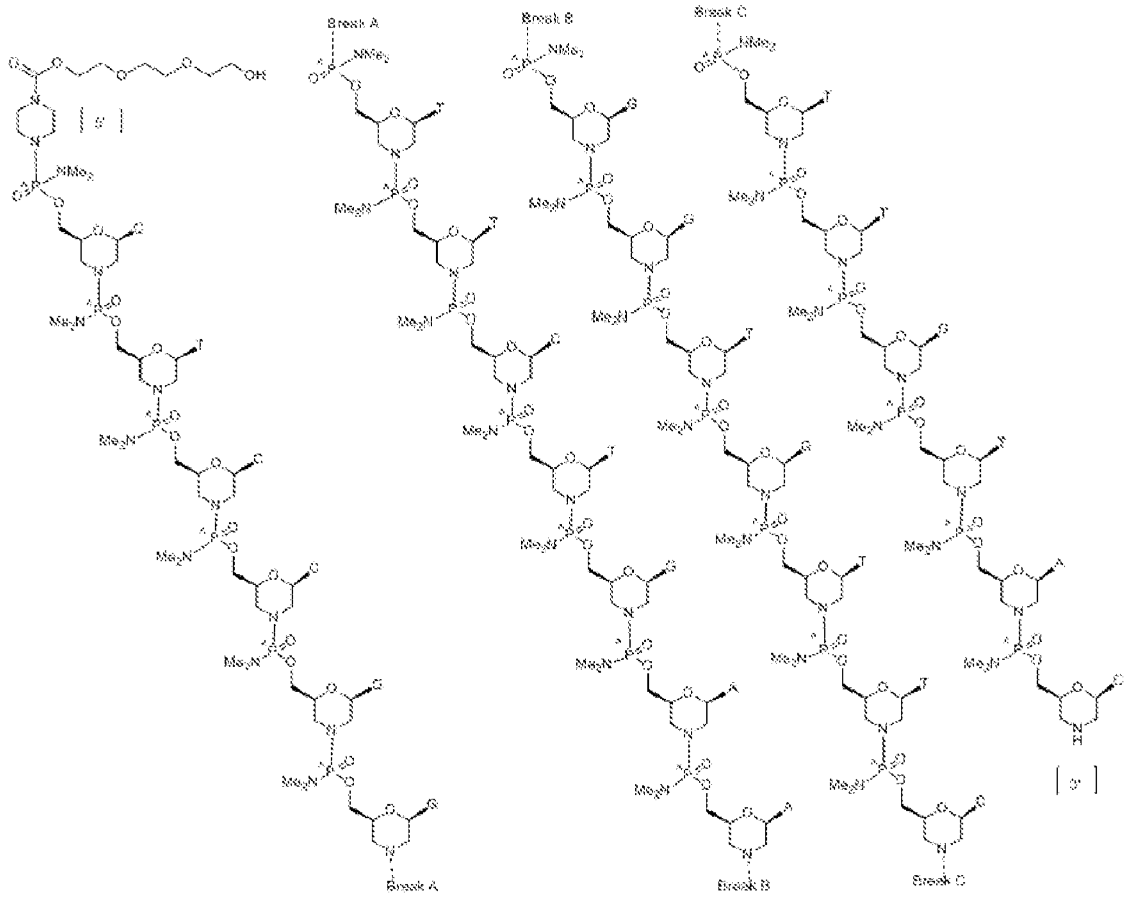

H53A(+30+55),

and

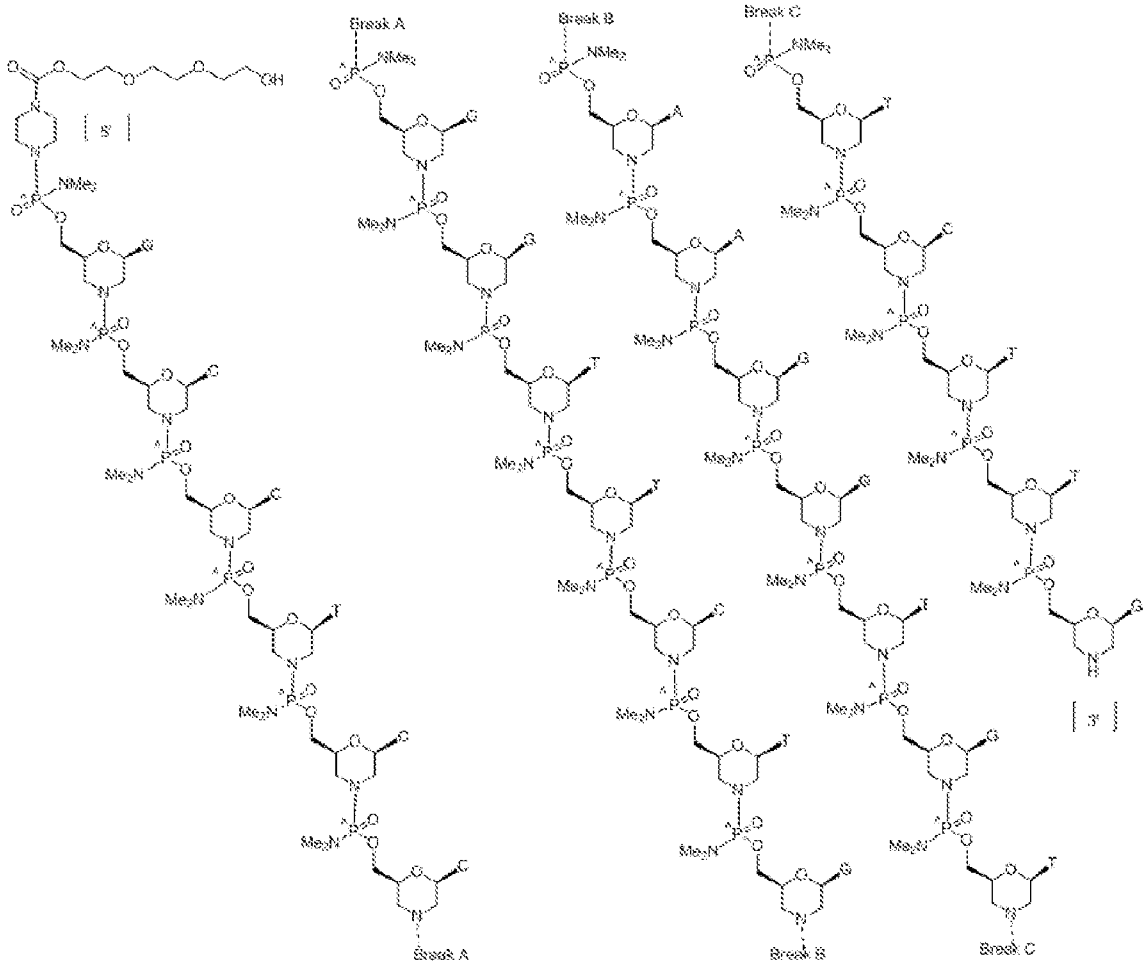

H53A(+33+57),

wherein

A= 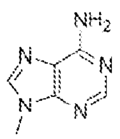 , C= 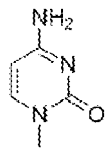 , G= 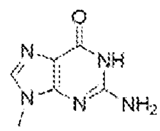 , and T=m$^5$U= 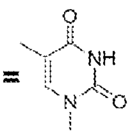 ,

and

wherein ^ = the stereochemistry of the phosphorous center is not defined.

Optionally, uracil bases can be substituted for thymine bases.

In one aspect, the antisense compound is composed of phosphorus-containing intersubunit linkages joining a morpholino nitrogen of one subunit to a 5' exocyclic carbon of an adjacent subunit, wherein the linkages are phosphorodiamidate linkages, in accordance with the structure:

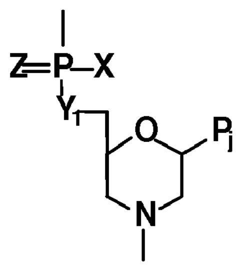

where $Y_1$=O, Z=O, Pj is a purine or pyrimidine base-pairing moiety effective to bind, by base-specific hydrogen bonding, to a base in a polynucleotide, and X is alkyl, alkoxy, thioalkoxy, or alkyl amino e.g., wherein X=$NR_2$, where each R is independently hydrogen or methyl. The above intersubunit linkages, which are uncharged, may be interspersed with linkages that are positively charged at physiological pH, where the total number of positively charged linkages is between 1 and up to all of the total number of intersubunit linkages.

In another exemplary embodiment, the compound is comprised of intersubunit linkage and terminal modifications as described in US Application No:13/118,298, which is incorporated herein in its entirety.

According to one aspect, the invention provides antisense molecules capable of binding to a selected target in human dystrophin pre-mRNA to induce exon skipping. In another aspect, the invention provides two or more antisense oligonucleotides which are used together to induce single or multiple exon skipping. For example, exon skipping of a single or multiple exons can be achieved by linking together two or more antisense oligonucleotide molecules.

In another embodiment, the invention relates to an isolated antisense oligonucleotide of 20 to 50 nucleotides in length, including at least 10, 12, 15, 17, 20 or 25 consecutive nucleotides complementary to an exon 53 target region of the dystrophin gene designated as an annealing site selected from the group consisting of: H53(+36+60), H53A(+30+57), H53A (+30+56), H53A(+30+55) and H53A(+33+57), wherein the antisense oligonucleotide specifically hybridizes to the annealing site inducing exon 53 skipping. In one embodiment, the antisense oligonucleotide is 20 to 30 or 25 to 28 nucleotides in length.

In another aspect, the invention relates to an isolated antisense oligonucleotide of 20 to 50 nucleotides in length, including at least 10, 12, 15, 17, 20, or 25 consecutive nucleotides of a nucleotide sequence selected from the group consisting of: SEQ ID NOs: 1-5, wherein the oligonucleotide specifically hybridizes to an exon 53 target region of the Dystrophin gene and induces exon 53 skipping. In one embodiment, the antisense oligonucleotide is 20 to 30 or 25 to 28 nucleotides in length. In another embodiment, thymine bases in SEQ ID NOs: 1-5 are optionally uracil.

The present invention includes exemplary antisense sequences targeted to exon 53, such as those identified below.

H53A(+36+60): 5'-GTTGCCTCCGGTTCTGAAGGTGTTC-3' (SEQ ID NO:1)
H53A(+30+57): 5'-GCCTCCGGTTCTGAAGGTGTTCTTGTAC-3' (SEQ ID NO: 2)
H53A(+30+56): 5'-CCTCCGGTTCTGAAGGTGTTCTTGTAC-3' (SEQ ID NO: 3)
H53A(+30+55): 5'-CTCCGGTTCTGAAGGTGTTCTTGTAC-3' (SEQ ID NO: 4)
H53A(+33+57): 5'-GCCTCCGGTTCTGAAGGTGTTCTTG-3' (SEQ ID NO: 5)

In one embodiment, the antisense oligomer specifically hybridizes to annealing site H53A(+30+56), such as SEQ ID NO: 3. In yet another embodiment, the antisense oligomer specifically hybridizes to annealing site H53A(+30+57), such as SEQ ID NO: 2. In yet another embodiment, the antisense oligomer specifically hybridizes to annealing site H53A(+30+55), such as SEQ ID NO: 4. In yet another embodiment, the antisense oligomer specifically hybridizes to annealing site H53A(+33+57), such as SEQ ID NO: 5. In yet another embodiment, the antisense oligomer specifically hybridizes to annealing site H53A(+36+60), such as SEQ ID NO: 1.

In some embodiments, the antisense oligonucleotides of the invention contain one or more modifications to minimize or prevent cleavage by RNase H. In some embodiments, the antisense oligonucleotides of the invention do not activate RNase H. In some embodiments, the antisense oligonucleotides comprise a non-natural backbone. In some embodiments, the sugar moieties of the oligonucleotide backbone are replaced with non-natural moieties, such as morpholinos. In some embodiments, the antisense oligonucleotides have the inter-nucleotide linkages of the oligonucleotide backbone replaced with non-natural inter-nucleotide linkages, such as modified phosphates. Exemplary modified phosphates include methyl phosphonates, methyl phosphorothioates, phosphoromorpholidates, phosophropiperazidates, and phosphoroamidates. In some embodiments, the antisense oligonucleotide is a 2'-O-methyl-oligoribonucleotide or a peptide nucleic acid.

In some embodiments, the antisense oligonucleotides contain base modifications or substitutions. For example, certain nucleo-bases may be selected to increase the binding affinity of the antisense oligonucleotides described herein.

These include 5-substituted pyrimidines, 6-azapyrimidines and N-2, N-6 and O-6 substituted purines, including 2-aminopropyladenine, 5-propynyluracil, 5-propynylcytosine and 2, 6-diaminopurine. 5-methylcytosine substitutions have been shown to increase nucleic acid duplex stability by 0.6-1.2°C, and may be incorporated into the antisense oligonucleotides described herein. In one embodiment, at least one pyrimidine base of the oligonucleotide comprises a 5-substituted pyrimidine base, wherein the pyrimidine base is selected from the group consisting of cytosine, thymine and uracil. In one embodiment, the 5-substituted pyrimidine base is 5-methylcytosine. In another embodiment, at least one purine base of the oligonucleotide comprises an N-2, N-6 substituted purine base. In one embodiment, the N-2, N-6 substituted purine base is 2, 6-diaminopurine.

In one embodiment, the antisense oligonucleotide includes one or more 5-methylcytosine substitutions alone or in combination with another modification, such as 2'-O-methoxyethyl sugar modifications. In yet another embodiment, the antisense oligonucleotide includes one or more 2, 6-diaminopurine substitutions alone or in combination with another modification.

In some embodiments, the antisense oligonucleotide is chemically linked to one or more moieties, such as a polyethylene glycol moiety, or conjugates, such as a arginine-rich cell penetrating peptide (e.g., SEQ ID NOs:16-31), that enhance the activity, cellular distribution, or cellular uptake of the antisense oligonucleotide. In one exemplary embodiment, the arginine-rich polypeptide is covalently coupled at its N-terminal or C-terminal residue to the 3' or 5' end of the antisense compound. Also in an exemplary embodiment, the antisense compound is composed of morpholino subunits and phosphorus-containing intersubunit linkages joining a morpholino nitrogen of one subunit to a 5' exocyclic carbon of an adjacent subunit.

In another aspect, the invention provides expression vectors that incorporate the antisense oligonucleotides described above, e.g., the antisense oligonucleotides of SEQ ID NOs: 1-5. In some embodiments, the expression vector is a modified retrovirus or non-retroviral vector, such as a adeno-associated viral vector.

In another aspect, the invention provides pharmaceutical compositions that include the antisense oligonucleotides described above, and a saline solution that includes a phosphate buffer.

In another aspect, the invention provides antisense molecules selected and or adapted to aid in the prophylactic or therapeutic treatment of a genetic disorder comprising at least an antisense molecule in a form suitable for delivery to a patient.

In another aspect, the invention provides a method for treating a patient suffering from a genetic disease wherein there is a mutation in a gene encoding a particular protein and the affect of the mutation can be abrogated by exon skipping, comprising the steps of: (a) selecting an antisense molecule in accordance with the methods described herein; and (b) administering the molecule to a patient in need of such treatment. The invention also addresses the use of purified and isolated antisense oligonucleotides of the invention, for the manufacture of a medicament for treatment of a genetic disease.

In another aspect, the invention provides a method of treating a condition characterized by muscular dystrophy, such as Duchenne muscular dystrophy or Becker muscular dystrophy, which includes administering to a patient an effective amount of an appropriately designed antisense oligonucleotide of the invention, relevant to the particular genetic lesion in that patient. Further, the invention provides a method for prophylactically treating a patient to prevent or minimize muscular dystrophy, such as Duchene muscular dystrophy or Becker muscular dystrophy, by administering to the patient an effective amount of an antisense oligonucleotide or a pharmaceutical composition comprising one or more of these biological molecules.

In another aspect, the invention also provides kits for treating a genetic disease, which kits comprise at least an antisense oligonucleotide of the present invention, packaged in a suitable container and instructions for its use.

These and other objects and features will be more fully understood when the following detailed description of the invention is read in conjunction with the figures.

## BRIEF DESCRIPTION OF THE FIGURES

**FIG. 1A** shows an exemplary morpholino oligomer structure with a phosphorodiamidate linkage.

**FIG. 1B** shows a conjugate of an arginine-rich peptide and an antisense oligomer, in accordance with an embodiment of the invention.

**FIG. 1C** shows a conjugate as in FIG. 1B, wherein the backbone linkages contain one or more positively charged groups.

**FIGs. 1D-G** show the repeating subunit segment of exemplary morpholino oligonucleotides, designated D through G.

**FIGs. 2A-2B** depicts a reaction scheme for the preparation of a linker for solid phase synthesis and a solid support for oligomer synthesis.

**FIG. 3** depicts a graph showing relative activities of exemplary antisense oligomers for inducing exon 53 skipping

in cultured primary myoblasts. RNA isolated from primary myoblasts treated with the indicated oligomers were subjected to exon 53-specific nested RT-PCR amplification, followed by gel electrophoresis and band intensity quantification. Data are plotted as % exon skipping as assessed by PCR, i.e., the band intensity of the exon-skipped product relative to the full-length PCR product.

**FIG. 4** depicts a graph showing relative activities of exemplary antisense oligomers for inducing exon 53 skipping in cultured human rhabdomyosarcoma cells. RNA isolated from rhabdomyosarcoma cells treated with the indicated oligomers were subjected to exon 53-specific nested RT-PCR amplification, followed by gel electrophoresis and band intensity quantification. Data are plotted as % exon skipping as assessed by PCR, i.e., the band intensity of the exon-skipped product relative to the full-length PCR product.

**FIG. 5A-B** depicts graphs showing relative activities of exemplary antisense oligomers for inducing exon 53 skipping in cultured primary myoblasts. RNA isolated from primary myoblasts treated with the indicated oligomers were subjected to exon 53-specific nested RT-PCR amplification, followed by gel electrophoresis and band intensity quantification. Data are plotted as % exon skipping as assessed by PCR, i.e., the band intensity of the exon-skipped product relative to the full-length PCR product.

## DETAILED DESCRIPTION OF THE INVENTION

Embodiments of the present invention relate generally to improved antisense compounds, and methods of use thereof, which are specifically designed to induce exon skipping in the human dystrophin gene. Dystrophin plays a vital role in muscle function, and various muscle-related diseases are characterized by mutated forms of this gene. Hence, in certain embodiments, the improved antisense compounds described herein induce exon skipping in mutated forms of the human dystrophin gene, such as the mutated dystrophin genes found in Duchenne muscular dystrophy (DMD) and Becker muscular dystrophy (BMD).

Due to aberrant mRNA splicing events caused by mutations, these mutated human dystrophin genes either express defective dystrophin protein or express no measurable dystrophin at all, a condition that leads to various forms of muscular dystrophy. To remedy this condition, the antisense compounds of the present invention hybridize to selected regions of a pre-processed RNA of a mutated human dystrophin gene, induce exon skipping and differential splicing in that otherwise aberrantly spliced dystrophin mRNA, and thereby allow muscle cells to produce an mRNA transcript that encodes a functional dystrophin protein. In certain embodiments, the resulting dystrophin protein is not necessarily the "wild-type" form of dystrophin, but is rather a truncated, yet functional or semi-functional, form of dystrophin.

By increasing the levels of functional dystrophin protein in muscle cells, these and related embodiments are useful in the prophylaxis and treatment of muscular dystrophy, especially those forms of muscular dystrophy, such as DMD and BMD, that are characterized by the expression of defective dystrophin proteins due to aberrant mRNA splicing. The specific oligomers described herein further provide improved, dystrophin-exon-specific targeting over other oligomers in use, and thereby offer significant and practical advantages over alternate methods of treating relevant forms of muscular dystrophy.

Thus, the invention relates to an antisense oligomer of 20-50 nucleotides in length capable of binding a selected target to induce exon skipping in the human dystrophin gene, wherein the antisense oligomer comprises a sequence of bases that specifically hybridizes to an exon 53 target region selected from the group consisting of H53A(+36+60), H53A(+30+57), H53A (+30+56), H53A(+30+55) and H53A(+33+57), wherein the bases of the oligomer are linked to morpholino ring structures, and wherein the morpholino ring structures are joined by phosphorous-containing intersubunit linkages joining a morpholino nitrogen of one ring structure to a 5' exocyclic carbon of an adjacent ring structure. In one embodiment, the antisense oligomer comprises a sequence of bases designated as SEQ ID NO: 1-5. In another embodiment, the antisense oligomer is about 20 to 30 nucleotides in length or about 25 to 28 nucleotides in length. In yet another embodiment, the sequence is 25 nucleotides and consists of a sequence selected from SEQ ID NO: 1-5.

The invention also relates to antisense oligonucleotides of 20 to 50 nucleotides in length and including at least 10, 12, 15, 17, 20 or more, consecutive nucleotides complementary to an exon 53 target region of the dystrophin gene designated as an annealing site selected from the group consisting of: H53(+36+60), H53A(+30+57), H53A (+30+56), H53A(+30+55) and H53A(+33+57).

Other antisense oligonucleotides of the invention are 20 to 50 nucleotides in length and include at least 10, 12, 15, 17, 20 or more, consecutive nucleotides of SEQ ID NOs: 1-5. In some embodiments, thymine bases in SEQ ID NOs: 1-5 are optionally uracil.

Exemplary antisense oligomers of the invention are set forth below:

H53A(+36+60): 5'-GTTGCCTCCGGTTCTGAAGGTGTTC-3' (SEQ ID NO: 1)
H53A(+30+57): 5'-GCCTCCGGTTCTGAAGGTGTTCTTGTAC-3' (SEQ ID NO: 2)
H53A(+30+56): 5'-CCTCCGGTTCTGAAGGTGTTCTTGTAC-3' (SEQ ID NO: 3)
H53A(+30+55): 5'-CTCCGGTTCTGAAGGTGTTCTTGTAC-3' (SEQ ID NO: 4)

H53A(+33+57): 5'-GCCTCCGGTTCTGAAGGTGTTCTTG-3' (SEQ ID NO: 5)

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

## I. Definitions

By "about" is meant a quantity, level, value, number, frequency, percentage, dimension, size, amount, weight or length that varies by as much as 30, 25, 20, 15, 10, 9, 8, 7, 6, 5, 4, 3, 2 or 1% to a reference quantity, level, value, number, frequency, percentage, dimension, size, amount, weight or length.

The terms "complementary" and "complementarity" refer to polynucleotides (i.e., a sequence of nucleotides) related by base-pairing rules. For example, the sequence "T-G-A (5'-3')," is complementary to the sequence "T-C-A (5'-3')." Complementarity may be "partial," in which only some of the nucleic acids' bases are matched according to base pairing rules. Or, there may be "complete" or "total" complementarity between the nucleic acids. The degree of complementarity between nucleic acid strands has significant effects on the efficiency and strength of hybridization between nucleic acid strands. While perfect complementarity is often desired, some embodiments can include one or more but preferably 6, 5, 4, 3, 2, or 1 mismatches with respect to the target RNA. Variations at any location within the oligomer are included. In certain embodiments, variations in sequence near the termini of an oligomer are generally preferable to variations in the interior, and if present are typically within about 6, 5, 4, 3, 2, or 1 nucleotides of the 5' and/or 3' terminus.

The terms "cell penetrating peptide" and "CPP" are used interchangeably and refer to cationic cell penetrating peptides, also called transport peptides, carrier peptides, or peptide transduction domains. The peptides, as shown herein, have the capability of inducing cell penetration within 100% of cells of a given cell culture population and allow macromolecular translocation within multiple tissues *in vivo* upon systemic administration. A preferred CPP embodiment is an arginine-rich peptide as described further below.

The terms "antisense oligomer" and "antisense compound" and "antisense oligonucleotide" are used interchangeably and refer to a sequence of cyclic subunits, each bearing a base-pairing moiety, linked by intersubunit linkages that allow the base-pairing moieties to hybridize to a target sequence in a nucleic acid (typically an RNA) by Watson-Crick base pairing, to form a nucleic acid:oligomer heteroduplex within the target sequence. The cyclic subunits are based on ribose or another pentose sugar or, in a preferred embodiment, a morpholino group (see description of morpholino oligomers below). The oligomer may have exact or near sequence complementarity to the target sequence; variations in sequence near the termini of an oligomer are generally preferable to variations in the interior.

Such an antisense oligomer can be designed to block or inhibit translation of mRNA or to inhibit natural pre-mRNA splice processing, and may be said to be "directed to" or "targeted against" a target sequence with which it hybridizes. The target sequence is typically a region including an AUG start codon of an mRNA, a Translation Suppressing Oligomer, or splice site of a pre-processed mRNA, a Splice Suppressing Oligomer (SSO). The target sequence for a splice site may include an mRNA sequence having its 5' end 1 to about 25 base pairs downstream of a normal splice acceptor junction in a preprocessed mRNA. A preferred target sequence is any region of a preprocessed mRNA that includes a splice site or is contained entirely within an exon coding sequence or spans a splice acceptor or donor site. An oligomer is more generally said to be "targeted against" a biologically relevant target, such as a protein, virus, or bacteria, when it is targeted against the nucleic acid of the target in the manner described above.

The terms "morpholino oligomer" or "PMO" (phosphoramidate- or phosphorodiamidate morpholino oligomer) refer to an oligonucleotide analog composed of morpholino subunit structures, where (i) the structures are linked together by phosphorus-containing linkages, one to three atoms long, preferably two atoms long, and preferably uncharged or cationic, joining the morpholino nitrogen of one subunit to a 5' exocyclic carbon of an adjacent subunit, and (ii) each morpholino ring bears a purine or pyrimidine base-pairing moiety effective to bind, by base specific hydrogen bonding, to a base in a polynucleotide. See, for example, the structure in Figure 1A, which shows a preferred phosphorodiamidate linkage type. The term "morpholino ring structure" may be used inetrchangably with the term "morpholino subunit." Variations can be made to this linkage as long as they do not interfere with binding or activity. For example, the oxygen attached to phosphorus may be substituted with sulfur (thiophosphorodiamidate). The 5' oxygen may be substituted with amino or lower alkyl substituted amino. The pendant nitrogen attached to phosphorus may be unsubstituted, monosubstituted, or disubstituted with (optionally substituted) lower alkyl. The purine or pyrimidine base pairing moiety is typically adenine, cytosine, guanine, uracil, thymine or inosine. The synthesis, structures, and binding characteristics of morpholino oligomers are detailed in U.S. Patent Nos. 5,698,685, 5,217,866, 5,142,047, 5,034,506, 5,166,315, 5,521,063, 5,506,337, 8,076,476, 8,299,206 and 7,943,762 (cationic linkages), all of which are incorporated herein by reference. Modified intersubunit linkages and terminal groups are detailed in PCT application US2011/038459 and publication WO/2011/150408 which are incorporated herein by reference in their entirety.

An "amino acid subunit" or "amino acid residue" can refer to an α-amino acid residue (-CO-CHR-NH-) or a β- or other amino acid residue (*e.g.*-CO-$(CH_2)_n$CHR-NH-), where R is a side chain (which may include hydrogen) and n is 1 to 6, preferably 1 to 4.

The term "naturally occurring amino acid" refers to an amino acid present in proteins found in nature. The term "non-natural amino acids" refers to those amino acids not present in proteins found in nature, examples include beta-alanine (β-Ala), 6-aminohexanoic acid (Ahx) and 6-aminopentanoic acid.

An "exon" refers to a defined section of nucleic acid that encodes for a protein, or a nucleic acid sequence that is represented in the mature form of an RNA molecule after either portions of a pre-processed (or precursor) RNA have been removed by splicing. The mature RNA molecule can be a messenger RNA (mRNA) or a functional form of a non-coding RNA, such as rRNA or tRNA. The human dystrophin gene has about 79 exons.

An "intron" refers to a nucleic acid region (within a gene) that is not translated into a protein. An intron is a non-coding section that is transcribed into a precursor mRNA (pre-mRNA), and subsequently removed by splicing during formation of the mature RNA.

An "effective amount" or "therapeutically effective amount" refers to an amount of therapeutic compound, such as an antisense oligomer, administered to a mammalian subject, either as a single dose or as part of a series of doses, which is effective to produce a desired therapeutic effect. For an antisense oligomer, this effect is typically brought about by inhibiting translation or natural splice-processing of a selected target sequence.

"Exon skipping" refers generally to the process by which an entire exon, or a portion thereof, is removed from a given pre-processed RNA, and is thereby excluded from being present in the mature RNA, such as the mature mRNA that is translated into a protein. Hence, the portion of the protein that is otherwise encoded by the skipped exon is not present in the expressed form of the protein, typically creating an altered, though still functional, form of the protein. In certain embodiments, the exon being skipped is an aberrant exon from the human dystrophin gene, which may contain a mutation or other alteration in its sequence that otherwise causes aberrant splicing. In certain embodiments, the exon being skipped is any one or more of exons 1-79 of the dystrophin gene, though exon 53 of the human dystrophin gene is preferred.

"Dystrophin" is a rod-shaped cytoplasmic protein, and a vital part of the protein complex that connects the cytoskeleton of a muscle fiber to the surrounding extracellular matrix through the cell membrane. Dystrophin contains multiple functional domains. For instance, dystrophin contains an actin binding domain at about amino acids 14-240 and a central rod domain at about amino acids 253-3040. This large central domain is formed by 24 spectrin-like triple-helical elements of about 109 amino acids, which have homology to alpha-actinin and spectrin. The repeats are typically interrupted by four proline-rich non-repeat segments, also referred to as hinge regions. Repeats 15 and 16 are separated by an 18 amino acid stretch that appears to provide a major site for proteolytic cleavage of dystrophin. The sequence identity between most repeats ranges from 10-25%. One repeat contains three alpha-helices: 1, 2 and 3. Alpha-helices 1 and 3 are each formed by 7 helix turns, probably interacting as a coiled-coil through a hydrophobic interface. Alpha-helix 2 has a more complex structure and is formed by segments of four and three helix turns, separated by a Glycine or Proline residue. Each repeat is encoded by two exons, typically interrupted by an intron between amino acids 47 and 48 in the first part of alpha-helix 2. The other intron is found at different positions in the repeat, usually scattered over helix-3. Dystrophin also contains a cysteine-rich domain at about amino acids 3080-3360), including a cysteine-rich segment (i.e., 15 Cysteines in 280 amino acids) showing homology to the C-terminal domain of the slime mold (Dictyostelium discoideum) alpha-actinin. The carboxy-terminal domain is at about amino acids 3361-3685.

The amino-terminus of dystrophin binds to F-actin and the carboxy-terminus binds to the dystrophin-associated protein complex (DAPC) at the sarcolemma. The DAPC includes the dystroglycans, sarcoglycans, integrins and caveolin, and mutations in any of these components cause autosomally inherited muscular dystrophies. The DAPC is destabilized when dystrophin is absent, which results in diminished levels of the member proteins, and in turn leads to progressive fibre damage and membrane leakage. In various forms of muscular dystrophy, such as Duchenne's muscular dystrophy (DMD) and Becker's muscular dystrophy (BMD), muscle cells produce an altered and functionally defective form of dystrophin, or no dystrophin at all, mainly due to mutations in the gene sequence that lead to incorrect splicing. The predominant expression of the defective dystrophin protein, or the complete lack of dystrophin or a dystrophin-like protein, leads to rapid progression of muscle degeneration, as noted above. In this regard, a "defective" dystrophin protein may be characterized by the forms of dystrophin that are produced in certain subjects with DMD or BMD, as known in the art, or by the absence of detectable dystrophin.

As used herein, the terms "function" and "functional" and the like refer to a biological, enzymatic, or therapeutic function.

A "functional" dystrophin protein refers generally to a dystrophin protein having sufficient biological activity to reduce the progressive degradation of muscle tissue that is otherwise characteristic of muscular dystrophy, typically as compared to the altered or "defective" form of dystrophin protein that is present in certain subjects with DMD or BMD. In certain embodiments, a functional dystrophin protein may have about 10%, 20%, 30%, 40%, 50%, 60%, 70%, 80%, 90%, or 100% (including all integers in between) of the in vitro or in vivo biological activity of wild-type dystrophin, as

measured according to routine techniques in the art. As one example, dystrophin-related activity in muscle cultures in vitro can be measured according to myotube size, myofibril organization (or disorganization), contractile activity, and spontaneous clustering of acetylcholine receptors (see, e.g., Brown et al., Journal of Cell Science. 112:209-216, 1999). Animal models are also valuable resources for studying the pathogenesis of disease, and provide a means to test dystrophin-related activity. Two of the most widely used animal models for DMD research are the mdx mouse and the golden retriever muscular dystrophy (GRMD) dog, both of which are dystrophin negative (see, e.g., Collins & Morgan, Int J Exp Pathol 84: 165-172, 2003). These and other animal models can be used to measure the functional activity of various dystrophin proteins. Included are truncated forms of dystrophin, such as those forms that are produced by certain of the exon-skipping antisense compounds of the present invention.

By "isolated" is meant material that is substantially or essentially free from components that normally accompany it in its native state. For example, an "isolated polynucleotide," as used herein, may refer to a polynucleotide that has been purified or removed from the sequences that flank it in a naturally-occurring state, e.g., a DNA fragment that has been removed from the sequences that are normally adjacent to the fragment.

As used herein, "sufficient length" refers to an antisense oligonucleotide that is complementary to at least 8, more typically 8-30, contiguous nucleobases in a target dystrophin pre-mRNA. In some embodiments, an antisense of sufficient length includes at least 8, 9, 10, 11, 12, 13, 14, or 15 contiguous nucleobases in the target dystrophin pre-mRNA. In other embodiments an antisense of sufficient length includes at least 16, 17, 18, 19, 20, 21, 22, 23, 24, or 25 contiguous nucleobases in the target dystrophin pre-mRNA. An antisense oligonucleotide of sufficient length has at least a minimal number of nucleotides to be capable of specifically hybridizing to exon 53. Preferably an oligonucleotide of sufficient length is from about 10 to about 50 nucleotides in length, including oligonucleotides of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 and 40 or more nucleotides. In one embodiment, an oligonucleotide of sufficient length is from 10 to about 30 nucleotides in length. In another embodiment, an oligonucleotide of sufficient length is from 15 to about 25 nucleotides in length. In yet another embodiment, an oligonucleotide of sufficient length is from 20 to 30, or 20 to 50, nucleotides in length. In yet another embodiment, an oligonucleotide of sufficient length is from 22 to 28, 25 to 28, 24 to 29 or 25 to 30 nucleotides in length.

By "enhance" or "enhancing," or "increase" or "increasing," or "stimulate" or "stimulating," refers generally to the ability of one or antisense compounds or compositions to produce or cause a greater physiological response (i.e., downstream effects) in a cell or a subject, as compared to the response caused by either no antisense compound or a control compound. A measurable physiological response may include increased expression of a functional form of a dystrophin protein, or increased dystrophin-related biological activity in muscle tissue, among other responses apparent from the understanding in the art and the description herein. Increased muscle function can also be measured, including increases or improvements in muscle function by about 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100%. The percentage of muscle fibres that express a functional dystrophin can also be measured, including increased dystrophin expression in about 1%, 2%, %, 15%, 16%, 17%, 18%, 19%, 20%, 25%, 30%, 35%, 40%, 45%, 50%, 55%, 60%, 65%, 70%, 75%, 80%, 85%, 90%, 95%, or 100% of muscle fibres. For instance, it has been shown that around 40% of muscle function improvement can occur if 25-30% of fibers express dystrophin (see, e.g., DelloRusso et al, Proc Natl Acad Sci USA 99: 12979-12984, 2002). An "increased" or "enhanced" amount is typically a "statistically significant" amount, and may include an increase that is 1.1, 1.2, 2, 3, 4, 5, 6, 7, 8, 9, 10, 15, 20, 30, 40, 50 or more times (e.g., 500, 1000 times) (including all integers and decimal points in between and above 1), e.g., 1.5, 1.6, 1.7, 1.8, etc.) the amount produced by no antisense compound (the absence of an agent) or a control compound.

The term "reduce" or "inhibit" may relate generally to the ability of one or more antisense compounds of the invention to "decrease" a relevant physiological or cellular response, such as a symptom of a disease or condition described herein, as measured according to routine techniques in the diagnostic art. Relevant physiological or cellular responses (*in vivo* or *in vitro*) will be apparent to persons skilled in the art, and may include reductions in the symptoms or pathology of muscular dystrophy, or reductions in the expression of defective forms of dystrophin, such as the altered forms of dystrophin that are expressed in individuals with DMD or BMD. A "decrease" in a response may be statistically significant as compared to the response produced by no antisense compound or a control composition, and may include a 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% decrease, including all integers in between.

Also included are vector delivery systems that are capable of expressing the oligomeric, dystrophin-targeting sequences of the present invention, such as vectors that express a polynucleotide sequence comprising any one or more of SEQ ID NOs: 1 and 9-18, as described herein. By "vector" or "nucleic acid construct" is meant a polynucleotide molecule, preferably a DNA molecule derived, for example, from a plasmid, bacteriophage, yeast or virus, into which a polynucleotide can be inserted or cloned. A vector preferably contains one or more unique restriction sites and can be capable of autonomous replication in a defined host cell including a target cell or tissue or a progenitor cell or tissue thereof, or be integrable with the genome of the defined host such that the cloned sequence is reproducible. Accordingly,

the vector can be an autonomously replicating vector, i.e., a vector that exists as an extra-chromosomal entity, the replication of which is independent of chromosomal replication, e.g., a linear or closed circular plasmid, an extra-chromosomal element, a mini-chromosome, or an artificial chromosome. The vector can contain any means for assuring self-replication. Alternatively, the vector can be one which, when introduced into the host cell, is integrated into the genome and replicated together with the chromosome(s) into which it has been integrated.

"Treatment" of an individual (e.g. a mammal, such as a human) or a cell is any type of intervention used in an attempt to alter the natural course of the individual or cell. Treatment includes, but is not limited to, administration of a pharmaceutical composition, and may be performed either prophylactically or subsequent to the initiation of a pathologic event or contact with an etiologic agent. Treatment includes any desirable effect on the symptoms or pathology of a disease or condition associated with the dystrophin protein, as in certain forms of muscular dystrophy, and may include, for example, minimal changes or improvements in one or more measurable markers of the disease or condition being treated. Also included are "prophylactic" treatments, which can be directed to reducing the rate of progression of the disease or condition being treated, delaying the onset of that disease or condition, or reducing the severity of its onset. "Treatment" or "prophylaxis" does not necessarily indicate complete eradication, cure, or prevention of the disease or condition, or associated symptoms thereof.

Hence, included are methods of treating muscular dystrophy, such as DMD and BMD, by administering one or more antisense oligomers of the present invention (e.g., SEQ ID NOs: 1-5, and variants thereof), optionally as part of a pharmaceutical formulation or dosage form, to a subject in need thereof. Also included are methods of inducing exon-skipping in a subject by administering one or more antisense oligomers, in which the exon is exon 53 from the dystrophin gene, preferably the human dystrophin gene. A "subject," as used herein, includes any animal that exhibits a symptom, or is at risk for exhibiting a symptom, which can be treated with an antisense compound of the invention, such as a subject that has or is at risk for having DMD or BMD, or any of the symptoms associated with these conditions (e.g., muscle fibre loss). Suitable subjects (patients) include laboratory animals (such as mouse, rat, rabbit, or guinea pig), farm animals, and domestic animals or pets (such as a cat or dog). Non-human primates and, preferably, human patients, are included.

"Alkyl" or "alkylene" both refer to a saturated straight or branched chain hydrocarbon radical containing from 1 to 18 carbons. Examples include without limitation methyl, ethyl, propyl, iso-propyl, butyl, iso-butyl, tert-butyl, n-pentyl and n-hexyl. The term "lower alkyl" refers to an alkyl group, as defined herein, containing between 1 and 8 carbons.

"Alkenyl" refers to an unsaturated straight or branched chain hydrocarbon radical containing from 2 to 18 carbons and comprising at least one carbon to carbon double bond. Examples include without limitation ethenyl, propenyl, iso-propenyl, butenyl, iso-butenyl, tert-butenyl, n-pentenyl and n-hexenyl. The term "lower alkenyl" refers to an alkenyl group, as defined herein, containing between 2 and 8 carbons.

"Alkynyl" refers to an unsaturated straight or branched chain hydrocarbon radical containing from 2 to 18 carbons comprising at least one carbon to carbon triple bond. Examples include without limitation ethynyl, propynyl, iso-propynyl, butynyl, iso-butynyl, tert-butynyl, pentynyl and hexynyl. The term "lower alkynyl" refers to an alkynyl group, as defined herein, containing between 2 and 8 carbons.

"Cycloalkyl" refers to a mono- or poly-cyclic alkyl radical. Examples include without limitation cyclobutyl, cyclopentyl, cyclohexyl, cycloheptyl and cyclooctyl.

"Aryl" refers to a cyclic aromatic hydrocarbon moiety containing from to 18 carbons having one or more closed ring(s). Examples include without limitation phenyl, benzyl, naphthyl, anthracenyl, phenanthracenyl and biphenyl.

"Aralkyl" refers to a radical of the formula RaRb where Ra is an alkylene chain as defined above and Rb is one or more aryl radicals as defined above, for example, benzyl, diphenylmethyl and the like.

"Thioalkoxy" refers to a radical of the formula -SRc where Rc is an alkyl radical as defined herein. The term "lower thioalkoxy" refers to an alkoxy group, as defined herein, containing between 1 and 8 carbons.

"Alkoxy" refers to a radical of the formula -ORda where Rd is an alkyl radical as defined herein. The term "lower alkoxy" refers to an alkoxy group, as defined herein, containing between 1 and 8 carbons. Examples of alkoxy groups include, without limitation, methoxy and ethoxy.

"Alkoxyalkyl" refers to an alkyl group substituted with an alkoxy group.

"Carbonyl" refers to the C(=O) - radical.

"Guanidynyl" refers to the $H_2N(C=NH_2)$-NH- radical.

"Amidinyl" refers to the $H_2N(C=NH_2)CH$- radical.

"Amino" refers to the $NH_2$ radical.

"Alkylamino" refers to a radical of the formula -NHRd or -NRdRd where each Rd is, independently, an alkyl radical as defined herein. The term "lower alkylamino" refers to an alkylamino group, as defined herein, containing between 1 and 8 carbons.

"Heterocycle" means a 5- to 7-membered monocyclic, or 7- to 10-membered bicyclic, heterocyclic ring which is either saturated, unsaturated, or aromatic, and which contains from 1 to 4 heteroatoms independently selected from nitrogen, oxygen and sulfur, and wherein the nitrogen and sulfur heteroatoms may be optionally oxidized, and the nitrogen

heteroatom may be optionally quaternized, including bicyclic rings in which any of the above heterocycles are fused to a benzene ring. The heterocycle may be attached via any heteroatom or carbon atom. Heterocycles include heteroaryls as defined below. Thus, in addition to the heteroaryls listed below, heterocycles also include morpholinyl, pyrrolidinonyl, pyrrolidinyl, piperidinyl, piperizinyl, hydantoinyl, valerolactamyl, oxiranyl, oxetanyl, tetrahydrofuranyl, tetrahydropyranyl, tetrahydropyridinyl, tetrahydrothiophenyl, tetrahydrothiopyranyl, tetrahydropyrimidinyl, tetrahydrothiopyranyl, and the like.

"Heteroaryl" means an aromatic heterocycle ring of 5- to 10 members and having at least one heteroatom selected from nitrogen, oxygen and sulfur, and containing at least 1 carbon atom, including both mono- and bicyclic ring systems. Representative heteroaryls are pyridyl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl, and quinazolinyl.

The terms "optionally substituted alkyl", "optionally substituted alkenyl", "optionally substituted alkoxy", "optionally substituted thioalkoxy", "optionally substituted alkyl amino", "optionally substituted lower alkyl", "optionally substituted lower alkenyl", "optionally substituted lower alkoxy", "optionally substituted lower thioalkoxy", "optionally substituted lower alkyl amino" and "optionally substituted heterocyclyl" mean that, when substituted, at least one hydrogen atom is replaced with a substituent. In the case of an oxo substituent (=O) two hydrogen atoms are replaced. In this regard, substituents include: deuterium, optionally substituted alkyl, optionally substituted alkenyl, optionally substituted alkynyl, optionally substituted aryl, optionally substituted heterocycle, optionally substituted cycloalkyl, oxo, halogen, -CN, -ORx, NRxRy, NRxC(=O)Ry, NRxSO2Ry, -NRxC(=O)NRxRy, C(=O)Rx, C(=O)ORx, C(=O)NRxRy, -SOmRx and -SOmNRxRy, wherein m is 0, 1 or 2, Rx and Ry are the same or different and independently hydrogen, optionally substituted alkyl, optionally substituted alkenyl, optionally substituted alkynyl, optionally substituted aryl, optionally substituted heterocycle or optionally substituted cycloalkyl and each of said optionally substituted alkyl, optionally substituted alkenyl, optionally substituted alkynyl, optionally substituted aryl, optionally substituted heterocycle and optionally substituted cycloalkyl substituents may be further substituted with one or more of oxo, halogen, - CN, -ORx, NRxRy, NRxC(=O)Ry, NRxSO2Ry, -NRxC(=O)NRxRy, C(=O)Rx, C(=O)ORx, C(=O)NRxRy, -SOmRx and -SOmNRxRy.

An antisense molecule nomenclature system was proposed and published to distinguish between the different antisense molecules (see Mann et al., (2002) J Gen Med 4, 644-654). This nomenclature became especially relevant when testing several slightly different antisense molecules, all directed at the same target region, as shown below:

$$H\#A/D(x:y).$$

The first letter designates the species (e.g. H: human, M: murine, C: canine). "#" designates target dystrophin exon number. "A/D" indicates acceptor or donor splice site at the beginning and end of the exon, respectively, (x y) represents the annealing coordinates where "-" or "+" indicate intronic or exonic sequences respectively. For example, A(-6+18) would indicate the last 6 bases of the intron preceding the target exon and the first 18 bases of the target exon. The closest splice site would be the acceptor so these coordinates would be preceded with an "A". Describing annealing coordinates at the donor splice site could be D(+2-18) where the last 2 exonic bases and the first 18 intronic bases correspond to the annealing site of the antisense molecule. Entirely exonic annealing coordinates that would be represented by A(+65+85), that is the site between the 65th and 85th nucleotide from the start of that exon.

## II. Antisense Oligonucleotides

When antisense molecule(s) are targeted to nucleotide sequences involved in splicing of exons within pre-mRNA sequences, normal splicing of the exon may be inhibited, causing the splicing machinery to by-pass the entire targeted exon from the mature mRNA. In many genes, deletion of an entire exon would lead to the production of a non-functional protein through the loss of important functional domains or the disruption of the reading frame. In some proteins, however, it is possible to shorten the protein by deleting one or more exons from within the protein, without disrupting the reading frame, and without seriously altering the biological activity of the protein. Typically, such proteins have a structural role and/or possess functional domains at their ends. Duchenne muscular dystrophy arises from mutations that preclude the synthesis of a functional dystrophin gene product, typically by disrupting the reading frame. Antisense oligonucleotides that induce exon skipping of the region of the dystrophin gene containing the mutation can allow muscle cells to produce a mature mRNA transcript that encodes a functional dystrophin protein. The resulting dystrophin protein is not necessarily the "wild-type" form of dystrophin, but is rather a truncated, yet functional or semi-functional, form of dystrophin. The present invention describes antisense molecules capable of binding to specified dystrophin pre-mRNA targets in exon 53, and re-directing processing of that gene.

In particular, the invention relates to isolated antisense oligonucleotides of 20 to 50 nucleotides in length, including at least 10, 12, 15, 17, 20, 25 or more, consecutive nucleotides complementary to an exon 53 target region of

the dystrophin gene designated as an annealing site selected from the following: H53A(+36+60), H53A(+30+57), H53A (+30+56), H53A(+30+55) and H53A(+33+57). Antisense oligonucleotides specifically hybridize to the annealing site, inducing exon 53 skipping.

The antisense oligonucleotide and the target RNA are complementary to each other when a sufficient number of corresponding positions in each molecule are occupied by nucleotides which can hydrogen bond with each other, such that stable and specific binding occurs between the oligonucleotide and the target. Thus, "specifically hybridizable" and "complementary" are terms which are used to indicate a sufficient degree of complementarity or precise pairing such that stable and specific binding occurs between the oligonucleotide and the target. It is understood in the art that the sequence of an antisense molecule need not be 100% complementary to that of its target sequence to be specifically hybridizable. An antisense molecule is specifically hybridizable when binding of the oligonucleotide to the target molecule interferes with the normal function of the target RNA, and there is a sufficient degree of complementarity to avoid non-specific binding of the antisense oligonucleotide to non-target sequences under conditions in which specific binding is desired, i.e., under physiological conditions in the case of in vivo assays or therapeutic treatment, and in the case of in vitro assays, under conditions in which the assays are performed.

The length of an antisense molecule may vary so long as it is capable of binding selectively to the intended location within the pre-mRNA molecule. The length of such sequences can be determined in accordance with selection procedures described herein. Generally, the antisense molecule will be from about 10 nucleotides in length up to about 50 nucleotides in length. It will be appreciated however that any length of nucleotides within this range may be used in the method. Preferably, the length of the antisense molecule is between 10-30 nucleotides in length.

In one embodiment, oligonucleotides of the invention are 20 to 50 nucleotides in length and include at least 10, 12, 15, 17, 20 or more, nucleotides of any of SEQ ID NOs: 1-5. In some embodiments, thymine bases in SEQ ID NOs: 1-5 are optionally uracil.

The exon deletion should not lead to a reading frame shift in the shortened transcribed mRNA. Thus, if in a linear sequence of three exons the end of the first exon encodes two of three nucleotides in a codon and the next exon is deleted then the third exon in the linear sequence must start with a single nucleotide that is capable of completing the nucleotide triplet for a codon. If the third exon does not commence with a single nucleotide there will be a reading frame shift that would lead to the generation of truncated or a non-functional protein.

It will be appreciated that the codon arrangements at the end of exons in structural proteins may not always break at the end of a codon, consequently there may be a need to delete more than one exon from the pre-mRNA to ensure in-frame reading of the mRNA. In such circumstances, a plurality of antisense oligonucleotides may need to be selected by the method of the invention wherein each is directed to a different region responsible for inducing splicing in the exons that are to be deleted.

In some embodiments, the antisense oligonucleotides have the chemical composition of a naturally occurring nucleic acid molecule, *i.e.,* the antisense oligonucleotides do not include a modified or substituted base, sugar, or intersubunit linkage. In a preferred embodiment, the antisense oligonucleotides of the present invention are non-naturally occurring nucleic acid molecules. For example, non-naturally occurring nucleic acids can include one or more non-natural base, sugar, and/or intersubunit linkage, *e.g.,* a base, sugar, and/or linkage that has been modified or substituted with respect to that found in a naturally occurring nucleic acid molecule. Exemplary modifications are described below. In some embodiments, non-naturally occurring nucleic acids include more than one type of modification, e.g., sugar and base modifications, sugar and linkage modifications, base and linkage modifications, or base, sugar, and linkage modifications. For example, in some embodiments, the antisense oligonucleotides contain a non-natural (e.g., modified or substituted) base. In some embodiments, the antisense oligonucleotides contain a non-natural (e.g., modified or substituted) sugar. In some embodiments, the antisense oligonucleotides contain a non-natural (e.g., modified or substituted) intersubunit linkage. In some embodiments, the antisense oligonucleotides contain more than one type of modification or substitution, e.g., a non-natural base and/or a non-natural sugar, and/or a non-natural intersubuint linkage.

To avoid degradation of pre-mRNA during duplex formation with the antisense molecules, the antisense molecules may be adapted to minimize or prevent cleavage by endogenous RNase H. This property is highly preferred as the treatment of the RNA with the unmethylated oligonucleotides either intracellularly or in crude extracts that contain RNase H leads to degradation of the pre-mRNA: antisense oligonucleotide duplexes. Any form of modified antisense molecule that is capable of by-passing or not inducing such degradation may be used in the present method. An example of antisense molecules which when duplexed with RNA are not cleaved by cellular RNase H is 2'-O-methyl derivatives. 2'-O-methyl-oligoribonucleotides are very stable in a cellular environment and in animal tissues, and their duplexes with RNA have higher Tm values than their ribo- or deoxyribo-counterparts. Methylation of the 2' hydroxyribose position and the incorporation of a phosphorothioate backbone is a common strategy for producing molecules that superficially resemble RNA but that are much more resistant to nuclease degradation.

Antisense molecules that do not activate RNase H can be made in accordance with known techniques (see, e.g., U.S. Pat. No. 5,149,797). Such antisense molecules, which may be deoxyribonucleotide or ribonucleotide sequences, simply contain any structural modification which sterically hinders or prevents binding of RNase H to a duplex molecule

containing the oligonucleotide as one member thereof, which structural modification does not substantially hinder or disrupt duplex formation. Because the portions of the oligonucleotide involved in duplex formation are substantially different from those portions involved in RNase H binding thereto, numerous antisense molecules that do not activate RNase H are available. For example, such antisense molecules may be oligonucleotides wherein at least one, or all, of the inter-nucleotide bridging phosphate residues are modified phosphates, such as methyl phosphonates, methyl phosphorothioates, phosphoromorpholidates, phosphoropiperazidates and phosphoramidates. For example, every other one of the internucleotide bridging phosphate residues may be modified as described. In another non-limiting example, such antisense molecules are molecules wherein at least one, or all, of the nucleotides contain a 2' lower alkyl moiety (e.g., $C_1$-$C_4$, linear or branched, saturated or unsaturated alkyl, such as methyl, ethyl, ethenyl, propyl, 1-propenyl, 2-propenyl, and isopropyl). For example, every other one of the nucleotides may be modified as described.

Specific examples of antisense oligonucleotides useful in this invention include oligonucleotides containing modified backbones or non-natural intersubunit linkages. Oligonucleotides having modified backbones include those that retain a phosphorus atom in the backbone and those that do not have a phosphorus atom in the backbone. Modified oligonucleotides that do not have a phosphorus atom in their inter-nucleoside backbone can also be considered to be oligonucleosides.

In other antisense molecules, both the sugar and the inter-nucleoside linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone.

Modified oligonucleotides may also contain one or more substituted sugar moieties.

Oligonucleotides may also include nucleobase (often referred to in the art simply as "base") modifications or substitutions. Oligonucleotides containing a modified or substituted base include oligonucleotides in which one or more purine or pyrimidine bases most commonly found in nucleic acids are replaced with less common or non-natural bases.

Purine bases comprise a pyrimidine ring fused to an imidazole ring, as described by the general formula:

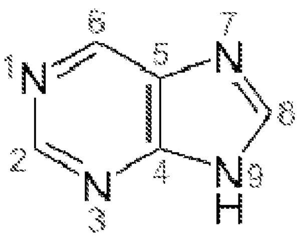

Purine

Adenine and guanine are the two purine nucleobases most commonly found in nucleic acids. These may be substituted with other naturally-occurring purines, including but not limited to $N^6$-methyladenine, $N^2$-methylguanine, hypoxanthine, and 7-methylguanine.

Pyrimidine bases comprise a six-membered pyrimidine ring as described by the general formula:

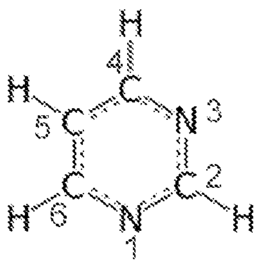

Pyrimidine

Cytosine, uracil, and thymine are the pyrimidine bases most commonly found in nucleic acids. These may be substituted with other naturally-occurring pyrimidines, including but not limited to 5-methylcytosine, 5-hydroxymethylcytosine, pseudouracil, and 4-thiouracil. In one embodiment, the oligonucleotides described herein contain thymine bases in place of

uracil.

Other modified or substituted bases include, but are not limited to, 2,6-diaminopurine, orotic acid, agmatidine, lysidine, 2-thiopyrimidine (e.g. 2-thiouracil, 2-thiothymine), G-clamp and its derivatives, 5-substituted pyrimidine (e.g. 5-halouracil, 5-propynyluracil, 5-propynylcytosine, 5-aminomethyluracil, 5-hydroxymethyluracil, 5-aminomethylcytosine, 5-hydroxymethylcytosine, Super T), 7-deazaguanine, 7-deazaadenine, 7-aza-2,6-diaminopurine, 8-aza-7-deazaguanine, 8-aza-7-deazaadenine, 8-aza-7-deaza-2,6-diaminopurine, Super G, Super A, and N4-ethylcytosine, or derivatives thereof; $N^2$-cyclopentylguanine (cPent-G), $N^2$-cyclopentyl-2-aminopurine (cPent-AP), and $N^2$-propyl-2-aminopurine (Pr-AP), pseudouracil or derivatives thereof; and degenerate or universal bases, like 2,6-difluorotoluene or absent bases like abasic sites (e.g. 1-deoxyribose, 1,2-dideoxyribose, l-deoxy-2-O-methylribose; or pyrrolidine derivatives in which the ring oxygen has been replaced with nitrogen (azaribose)). Examples of derivatives of Super A, Super G and Super T can be found in U.S. Patent 6,683, 173 (Epoch Biosciences), which is incorporated here entirely by reference. cPent-G, cPent-AP and Pr-AP were shown to reduce immunostimulatory effects when incorporated in siRNA (Peacock H. et al. J. Am. Chem. Soc. 2011, 133, 9200). Pseudouracil is a naturally occuring isomerized version of uracil, with a C-glycoside rather than the regular N-glycoside as in uridine. Pseudouridine-containing synthetic mRNA may have an improved safety profile compared to uridine-containing mPvNA (WO 2009127230, incorporated here in its entirety by reference).

Certain modified or substituted nucleo-bases are particularly useful for increasing the binding affinity of the antisense oligonucleotides of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2°C and are presently preferred base substitutions, even more particularly when combined with 2'-O-methoxyethyl sugar modifications.

In some embodiments, modified or substituted nucleo-bases are useful for facilitating purification of antisense oligonucleotides. For example, in certain embodiments, antisense oligonucleotides may contain three or more *(e.g.,* 3, 4, 5, 6 or more) consecutive guanine bases. In certain antisense oligonucleotides, a string of three or more consecutive guanine bases can result in aggregation of the oligonucleotides, complicating purification. In such antisense oligonucleotides, one or more of the consecutive guanines can be substituted with inosine. The substitution of inosine for one or more guanines in a string of three or more consecutive guanine bases can reduce aggregation of the antisense oligonucleotide, thereby facilitating purification.

In one embodiment, another modification of the antisense oligonucleotides involves chemically linking to the oligonucleotide one or more moieties or conjugates that enhance the activity, cellular distribution or cellular uptake of the oligonucleotide. Such moieties include but are not limited to lipid moieties such as a cholesterol moiety, cholic acid, a thioether, e.g., hexyl-5-tritylthiol, a thiocholesterol, an aliphatic chain, e.g., dodecandiol or undecyl residues, a phospholipid, e.g., di-hexadecyl-rac-glycerol or triethylammonium 1,2-di-O-hexadecyl-rac-glycero-3-H-phosphonate, a polyamine or a polyethylene glycol chain, or adamantane acetic acid, a palmityl moiety, or an octadecylamine or hexylamino-carbonyl-oxycholesterol moiety.

It is not necessary for all positions in a given compound to be uniformly modified, and in fact more than one of the aforementioned modifications may be incorporated in a single compound or even at a single nucleoside within an oligonucleotide. The present invention also includes antisense oligonucleotides that are chimeric compounds. "Chimeric" antisense compounds or "chimeras," in the context of this invention, are antisense molecules, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the increased resistance to nuclease degradation, increased cellular uptake, and an additional region for increased binding affinity for the target nucleic acid.

The antisense molecules used in accordance with this invention may be conveniently and routinely made through the well-known technique of solid phase synthesis. Equipment for such synthesis is sold by several vendors including, for example, Applied Biosystems (Foster City, Calif.). One method for synthesising oligonucleotides on a modified solid support is described in U.S. Pat. No. 4,458,066.

Any other means for such synthesis known in the art may additionally or alternatively be employed. It is well known to use similar techniques to prepare oligonucleotides such as the phosphorothioates and alkylated derivatives. In one such automated embodiment, diethyl-phosphoramidites are used as starting materials and may be synthesized as described by Beaucage, et al., (1981) Tetrahedron Letters, 22:1859-1862.

The antisense molecules of the invention are synthesised in vitro and do not include antisense compositions of biological origin. The molecules of the invention may also be mixed, encapsulated, conjugated or otherwise associated with other molecules, molecule structures or mixtures of compounds, as for example, liposomes, receptor targeted molecules, oral, rectal, topical or other formulations, for assisting in uptake, distribution and/or absorption.

### *A. Morpholino Oligomers*

Exemplary embodiments of the invention relate to morpholino oligomers having phosphorus-containing backbone linkages are illustrated in Figs. 1A-1C. One embodiment is a phosphorodiamidate-linked morpholino oligomer such as shown in Fig. 1C, which is modified, in accordance with one aspect of the present invention, to contain positively charged groups at preferably 10%-50% of its backbone linkages. Morpholino oligomers with uncharged backbone linkages, including antisense oligonucleotides, are detailed, for example, in (Summerton and Weller 1997) and in co-owned U.S. Patent Nos. 5,698,685, 5,217,866, 5,142,047, 5,034,506, 5,166,315, 5,185, 444, 5,521,063, 5,506,337, 8,076,476, 8,299,206 and 7,943,762 all of which are expressly incorporated by reference herein. Morpholino oligomers with modified linkages including charged linkages can be found in US application number 13/118,298, incorporated herein in its entirety by reference.

Important properties of the morpholino-based subunits include: 1) the ability to be linked in a oligomeric form by stable, uncharged or positively charged backbone linkages; 2) the ability to support a nucleotide base (e.g. adenine, cytosine, guanine, thymidine, uracil and inosine) such that the polymer formed can hybridize with a complementary-base target nucleic acid, including target RNA, Tm values above about 45°C in relatively short oligonucleotides *(e.g.,* 10-15 bases); 3) the ability of the oligonucleotide to be actively or passively transported into mammalian cells; and 4) the ability of the antisense oligonucleotide:RNA heteroduplex to resist RNAse and RNase H degradation, respectively.

Exemplary backbone structures for antisense oligonucleotides of the claimed subject matter include the morpholino subunit types shown in Figs. 1D-G, each linked by an uncharged or positively charged, phosphorus-containing subunit linkage. Fig. 1D shows a phosphorus-containing linkage which forms the five atom repeating-unit backbone, where the morpholino rings are linked by a 1-atom phosphoamide linkage. Fig. IE shows a linkage which produces a 6-atom repeating-unit backbone. In this structure, the atom Y linking the 5' morpholino carbon to the phosphorus group may be sulfur, nitrogen, carbon or, preferably, oxygen. The X moiety pendant from the phosphorus may be fluorine, an alkyl or substituted alkyl, an alkoxy or substituted alkoxy, a thioalkoxy or substituted thioalkoxy, or unsubstituted, monosubstituted, or disubstituted nitrogen, including cyclic structures, such as morpholines or piperidines. Alkyl, alkoxy and thioalkoxy preferably include 1-6 carbon atoms. The Z moieties are sulfur or oxygen, and are preferably oxygen.

The linkages shown in Figs. 1F and 1G are designed for 7-atom unit-length backbones. In structure IF, the X moiety is as in Structure IE, and the Y moiety may be methylene, sulfur, or, preferably, oxygen. In Structure 1G, the X and Y moieties are as in Structure IE. Particularly preferred morpholino oligonucleotides include those composed of morpholino subunit structures of the form shown in Fig. IE, where X=$NH_2$, $N(CH_3)_2$, or 1-piperazine or other charged group, Y=O, and Z=O.

A substantially uncharged oligonucleotide may be modified, in accordance with an aspect of the invention, to include charged linkages, *e.g.,* up to about 1 per every 2-5 uncharged linkages, such as about 4-5 per every 10 uncharged linkages. In certain embodiments, optimal improvement in antisense activity may be seen when about 25% of the backbone linkages are cationic. In certain embodiments, enhancement may be seen with a small number *e.g.,* 10-20% cationic linkages, or where the number of cationic linkages are in the range 50-80%, such as about 60%.

Oligomers having any number of cationic linkages are provided, including fully cationic-linked oligomers. Preferably, however, the oligomers are partially charged, having, for example, 10%-80%. In preferred embodiments, about 10% to 60%, and preferably 20% to 50% of the linkages are cationic.

In one embodiment, the cationic linkages are interspersed along the backbone. The partially charged oligomers preferably contain at least two consecutive uncharged linkages; that is, the oligomer preferably does not have a strictly alternating pattern along its entire length.

Also considered are oligomers having blocks of cationic linkages and blocks of uncharged linkages; for example, a central block of uncharged linkages may be flanked by blocks of cationic linkages, or vice versa. In one embodiment, the oligomer has approximately equal-length 5', 3' and center regions, and the percentage of cationic linkages in the center region is greater than about 50%, preferably greater than about 70%.

In certain embodiments, the antisense compounds can be prepared by stepwise solid-phase synthesis, employing methods detailed in the references cited above, and below with respect to the synthesis of oligonucleotides having a mixture or uncharged and cationic backbone linkages. In some cases, it may be desirable to add additional chemical moieties to the antisense compound, *e.g.,* to enhance pharmacokinetics or to facilitate capture or detection of the compound. Such a moiety may be covalently attached, according to standard synthetic methods. For example, addition of a polyethylene glycol moiety or other hydrophilic polymer, *e.g.,* one having 1-100 monomeric subunits, may be useful in enhancing solubility.

A reporter moiety, such as fluorescein or a radiolabeled group, may be attached for purposes of detection. Alternatively, the reporter label attached to the oligomer may be a ligand, such as an antigen or biotin, capable of binding a labeled antibody or streptavidin. In selecting a moiety for attachment or modification of an antisense compound, it is generally of course desirable to select chemical compounds of groups that are biocompatible and likely to be tolerated by a subject without undesirable side effects.

Oligomers for use in antisense applications generally range in length from about 10 to about 50 subunits, more preferably about 10 to 30 subunits, and typically 15-25 bases. For example, an oligomer of the invention having 19-20 subunits, a useful length for an antisense compound, may ideally have two to ten, *e.g.,* four to eight, cationic linkages, and the remainder uncharged linkages. An oligomer having 14-15 subunits may ideally have two to seven, *e.g.,* 3, 4, or 5, cationic linkages and the remainder uncharged linkages. In a preferred embodiment, the oligomers have 25 to 28 subunits.

Each morpholino ring structure supports a base pairing moiety, to form a sequence of base pairing moieties which is typically designed to hybridize to a selected antisense target in a cell or in a subject being treated. The base pairing moiety may be a purine or pyrimidine found in native DNA or RNA *(e.g.,* A, G, C, T or U) or an analog, such as hypoxanthine (the base component of the nucleoside inosine) or 5-methyl cytosine.

As noted above, certain embodiments are directed to oligomers comprising novel intersubunit linkages, including PMO-X oligomers and those having modified terminal groups. In some embodiments, these oligomers have higher affinity for DNA and RNA than do the corresponding unmodified oligomers and demonstrate improved cell delivery, potency, and/or tissue distribution properties compared to oligomers having other intersubunit linkages. The structural features and properties of the various linkage types and oligomers are described in more detail in the following discussion. The synthesis of these and related oligomers is described in co-owned U.S. Application No. 13/118,298, which is incorporated by reference in its entirety.

In certain embodiments, the invention provides for an oligonucleotide having a sequence complementary to the target sequence which is associated with a human disease, and comprises a sequence of nucleotides having a formula:

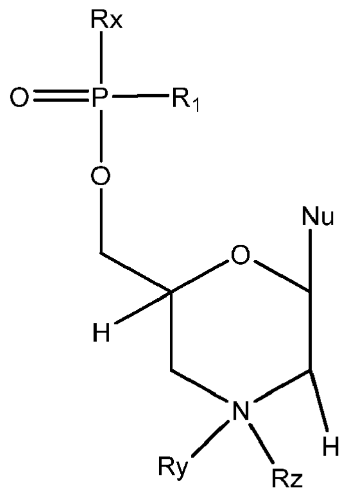

wherein Nu is a nucleobase;

$R_1$ has the formula

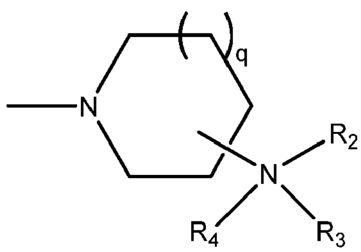

q is 0, 1, or 2;

$R_2$ is selected from the group consisting of hydrogen, $C_1$-$C_5$ alkyl, $C_1$-$C_5$ aralkyl, and a formamidinyl group, and

$R_3$ is selected from the group consisting of hydrogen, $C_1$-$C_{10}$ acyl, $C_1$-$C_{10}$ aminoacyl, acyl moiety of a natural or unnatural alpha or beta amino acid, $C_1$-$C_{10}$ aralkyl, and $C_1$-$C_{10}$ alkyl, or

$R_2$ and $R_3$ are joined to form a 5-7 membered ring where the ring may be optionally substituted with a substituent selected from the group consisting of $C_1$-$C_{10}$ alkyl, phenyl, halogen, and $C_1$-$C_{10}$ aralkyl;

$R_4$ is selected from the group consisting of an electron pair, hydrogen, a $C_1$-$C_6$ alkyl and $C_1$-$C_6$ aralkyl;

Rx is selected from the group consisting of sarcosinamide, hydroxyl, a nucleotide, a cell penetrating peptide moiety, and piperazinyl;

Ry is selected from the group consisting of hydrogen, a $C_1$-$C_6$ alkyl, a nucleotide a cell penetrating peptide moiety, an amino acid, a formamidinyl group, and $C_1$-$C_6$ acyl; and,

Rz is selected from the group consisting of an electron pair, hydrogen, a $C_1$-$C_6$ alkyl, and $C_1$-$C_6$ acyl pharmaceutically acceptable salts thereof.

Nu may be selected from the group consisting of adenine, guanine, thymine, uracil, cytosine, and hypoxanthine. More preferably Nu is thymine or uracil.

In preferred embodiments, the invention provides an oligonucleotide having a sequence of nucleotides having a formula:

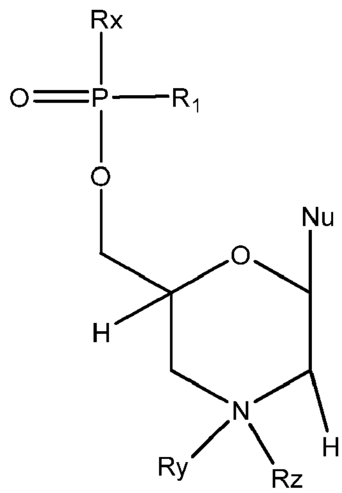

wherein Nu is a nucleobase;

$R_1$ is selected from the group consisting of $R_1$' and $R_1$" wherein $R_1$' is dimethyl- amino and $R_1$" has the formula

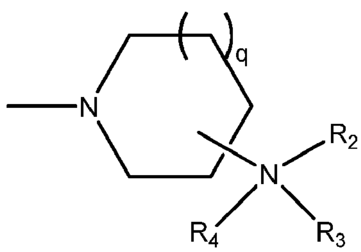

wherein at least one $R_1$ is $R_1$";

q is 0, 1, or 2; with the proviso that at least one of $R_1$ is a piperidinyl moiety;

$R_2$ is selected from the group consisting of hydrogen, $C_1$-$C_5$ alkyl, $C_1$-$C_5$ aralkyl, and a formamidinyl group, and

$R_3$ is selected from the group consisting of hydrogen, $C_1$-$C_{10}$ acyl, $C_1$-$C_{10}$ aminoacyl, acyl moiety of a natural or unnatural alpha or beta amino acid, $C_1$-$C_{10}$ aralkyl, and $C_1$-$C_{10}$ alkyl, or

$R_2$ and $R_3$ are joined to form a 5-7 membered ring where the ring may be optionally substituted with a substituent selected from the group consisting of $C_1$-$C_{10}$ alkyl, phenyl, halogen, and $C_1$-$C_{10}$ aralkyl;

$R_4$ is selected from the group consisting of an electron pair, hydrogen, a $C_1$-$C_6$ alkyl and aralkyl;

Rx is selected from the group consisting of sarcosinamide, hydroxyl, a nucleotide, a cell penetrating peptide moiety, and piperazinyl;

Ry is selected from the group consisting of hydrogen, a $C_1$-$C_6$ alkyl, a nucleotide a cell penetrating peptide moiety, an amino acid, a formamidinyl group, and $C_1$-$C_6$ acyl; and,

Rz is selected from the group consisting of an electron pair, hydrogen, a $C_1$-$C_6$ alkyl, and $C_1$-$C_6$ acyl pharmaceutically acceptable salts thereof.

Nu may be selected from the group consisting of adenine, guanine, thymine, uracil, cytosine, and hypoxanthine. More preferably Nu is thymine or uracil.

About 90-50% of the $R_1$ groups are dimethylamino (i.e. $R_1$'). More, preferably, 90-50% of the $R_1$ groups are dimethylamino. Most, preferably about 66% of the $R_1$ groups are dimethylamino.

$R_1$" may be selected from the group consisting of

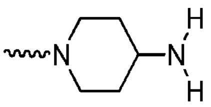
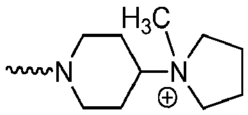

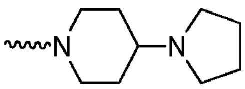
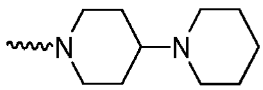

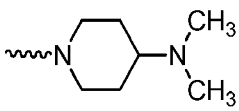
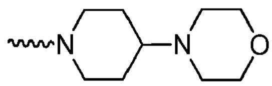

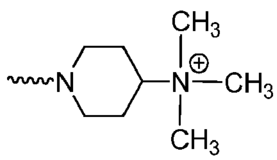
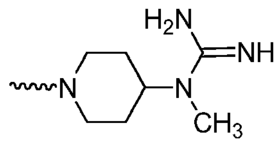

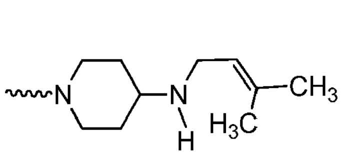
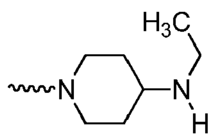

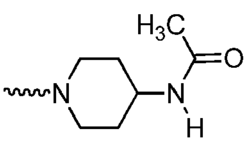
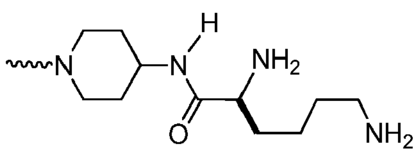

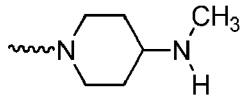
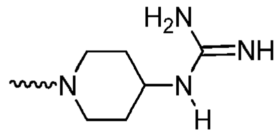
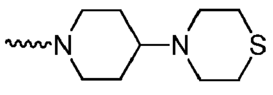
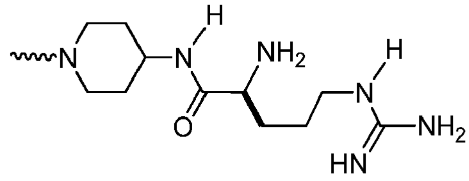

Preferably, at least one nucleotide of the oligonucleotide has the formula:

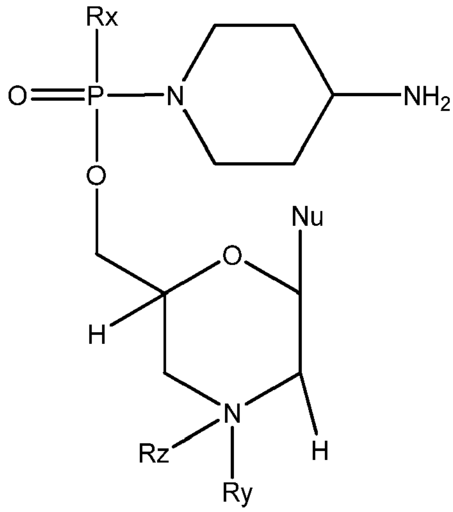

wherein Rx, Ry, Rz, and Nu are as stated above. Most preferably, Nu is thymine or uracil.

Although thymine (T) is the preferred base pairing moiety (Nu or Pi) containing the chemical modifications described above, any base subunit known to a person of skill in the art can be used as the base pairing moiety.

***B. Peptide Transporters***

The antisense oligonucleotides of the invention may include an oligonucleotide moiety conjugated to a CPP, preferably an arginine-rich peptide transport moiety effective to enhance transport of the compound into cells. The transport moiety is preferably attached to a terminus of the oligomer, as shown, for example, in **FIGs 1B** and **1C.** The peptides have the capability of inducing cell penetration within 30%, 40%, 50%, 60%, 70%, 80%, 90% or 100% of cells of a given cell culture population, including all integers in between, and allow macromolecular translocation within multiple tissues *in vivo* upon systemic administration. In one embodiment, the cell-penetrating peptide may be an arginine-rich peptide transporter. In another embodiment, the cell-penetrating peptide may be Penetratin or the Tat peptide. These peptides are well known in the art and are disclosed, for example, in US Publication No. 2010-0016215 A1, incorporated by reference in its entirety. A particularly preferred approach to conjugation of peptides to antisense oligonucleotides can be found in PCT publication WO2012/150960, which is incorporated by reference in its entirety. A preferred embodiment of a peptide conjugated oligonucleotide of the present invention utilizes glycine as the linker between the CPP and the antisense oligonucleotide. For example, a preferred peptide conjugated PMO consists of $R_6$-G-PMO.

The transport moieties as described above have been shown to greatly enhance cell entry of attached oligomers, relative to uptake of the oligomer in the absence of the attached transport moiety. Uptake is preferably enhanced at least ten fold, and more preferably twenty fold, relative to the unconjugated compound.

The use of arginine-rich peptide transporters (i.e., cell-penetrating peptides) are particularly useful in practicing the present invention. Certain peptide transporters have been shown to be highly effective at delivery of antisense compounds into primary cells including muscle cells (Marshall, Oda et al. 2007; Jearawiriyapaisarn, Moulton et al. 2008; Wu, Moulton et al. 2008). Furthermore, compared to other known peptide transporters such as Penetratin and the Tat

peptide, the peptide transporters described herein, when conjugated to an antisense PMO, demonstrate an enhanced ability to alter splicing of several gene transcripts (Marshall, Oda et al. 2007). Preferred embodiments of morpholino-peptide transporter conjugates are described in WO/2012/150960 which is incorporated herein in its entirety.

Exemplary peptide transporters, excluding linkers are given below in Table 1.

Table 1. Exemplary peptide transporters

| NAME (DESIGNATION) | SEQUENCE | SEQ ID NO[A] |
|---|---|---|
| rTAT | RRRQRRKKR | 16 |
| Tat | RKKRRQRRR | 17 |
| $R_9F_2$ | RRRRRRRRRFF | 18 |
| $R_5F_2R_4$ | RRRRRFFRRRR | 19 |
| $R_4$ | RRRR | 20 |
| $R_5$ | RRRRR | 21 |
| $R_6$ | RRRRRR | 22 |
| $R_7$ | RRRRRRR | 23 |
| $R_8$ | RRRRRRRR | 24 |
| $R_9$ | RRRRRRRRR | 25 |
| $(RX)_8$ | RXRXRXRXRXRXRXRX | 26 |
| $(RAhxR)_4$; (P007) | RAhxRRAhxRRAhxRRAhxR | 27 |
| $(RAhxR)_5$; (CP04057) | RAhxRRAhxRRAhxRRAhxRRAhxR | 28 |
| $(RAhxRRBR)_2$; (CP06062) | RAhxRRBRRAhxRRBR | 29 |
| $(RAR)_4F_2$ | RARRARRARRARFF | 30 |
| $(RGR)_4F_2$ | RGRRGRRGRRGRFF | 31 |
| [A]Sequences assigned to SEQ ID NOs do not include the linkage portion *(e.g.,* C, G, P, Ahx, B, AhxB where Ahx and B refer to 6-aminohexanoic acid and beta-alanine, respectively). | | |

### *C. Expression Vectors*

In one embodiment, the invention includes expression vectors for expression of the dystrophin-targeting sequences described herein in cells. Vector delivery systems are capable of expressing the oligomeric, dystrophin-targeting sequences of the present invention. In one embodiment, such vectors express a polynucleotide sequence comprising at least 10 consecutive nucleotides of one or more of SEQ ID NOs: 1-5. In another embodiment, such vectors express a polynucleotide sequence comprising one or more of SEQ ID NOs: 1-5. Expression vectors suitable for gene delivery are known in the art. Such expression vectors can be modified to express the dystrophin-targeting sequences described herein. Exemplary expression vectors include polynucleotide molecules, preferably DNA molecules, that are derived, for example, from a plasmid, bacteriophage, yeast or virus (e.g., adenovirus, adeno-associated virus, lentivirus, etc.), into which a polynucleotide can be inserted or cloned. A vector preferably contains one or more unique restriction sites and can be capable of autonomous replication in a defined host cell including a target cell or tissue or a progenitor cell or tissue thereof, or be integrable with the genome of the defined host such that the cloned sequence is reproducible. Accordingly, the vector can be an autonomously replicating vector, i.e., a vector that exists as an extra-chromosomal entity, the replication of which is independent of chromosomal replication, e.g., a linear or closed circular plasmid, an extra-chromosomal element, a mini-chromosome, or an artificial chromosome. The vector can contain any means for assuring self-replication. Alternatively, the vector can be one which, when introduced into the host cell, is integrated into the genome and replicated together with the chromosome(s) into which it has been integrated.

In one embodiment, the expression vectors include a tissue-specific promoter, *e.g.,* a muscle-specific promoter and/or enhancer, which promotes expression of the oligomeric dystrophin-targeting sequences described herein in particular cells or tissues of interest *(e.g.,* in muscle). Promoter sequences and expression vectors suitable for expression in muscle cells include, for example, those described in US 2011/0212529, the entire contents of which are incorporated herein by reference. Exemplary muscle-specific promoters include a desmin promoter, a muscle creatine kinase (MCK)

promoter, a Pitx3 promoter, a skeletal alpha-actin promoter, or a troponin I promoter. Use of muscle-specific promoters are further described in, for example, Talbot et al., Molecular Therapy (2010), 18(3): 601-608; Wang et al., Gene Therapy (2008), 15(22): 1489-99; and Coulon et al., Journal of Biological Chemistry (2007), 282(45): 33192-33200.

## III. Formulations and Modes of Administration

In certain embodiments, the present invention provides formulations or compositions suitable for the therapeutic delivery of antisense oligomers, as described herein. Hence, in certain embodiments, the present invention provides pharmaceutically acceptable compositions that comprise a therapeutically-effective amount of one or more of the oligomers described herein, formulated together with one or more pharmaceutically acceptable carriers (additives) and/or diluents. While it is possible for an oligomer of the present invention to be administered alone, it is preferable to administer the compound as a pharmaceutical formulation (composition).

Methods for the delivery of nucleic acid molecules are described, for example, in Akhtar et al., 1992, Trends Cell Bio., 2:139; and Delivery Strategies for Antisense Oligonucleotide Therapeutics, ed. Akhtar; Sullivan et al., PCT WO 94/02595. These and other protocols can be utilized for the delivery of virtually any nucleic acid molecule, including the isolated oligomers of the present invention.

As detailed below, the pharmaceutical compositions of the present invention may be specially formulated for administration in solid or liquid form, including those adapted for the following: (1) oral administration, for example, drenches (aqueous or non-aqueous solutions or suspensions), tablets, e.g., those targeted for buccal, sublingual, and systemic absorption, boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; or (8) nasally.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient.

Some examples of materials that can serve as pharmaceutically-acceptable carriers include, without limitation: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

Additional non-limiting examples of agents suitable for formulation with the antisense oligomers of the instant invention include: PEG conjugated nucleic acids, phospholipid conjugated nucleic acids, nucleic acids containing lipophilic moieties, phosphorothioates, P-glycoprotein inhibitors (such as Pluronic P85) which can enhance entry of drugs into various tissues; biodegradable polymers, such as poly (DL-lactide-coglycolide) microspheres for sustained release delivery after implantation (Emerich, D F et al., 1999, Cell Transplant, 8, 47-58) Alkermes, Inc. Cambridge, Mass.; and loaded nanoparticles, such as those made of polybutylcyanoacrylate, which can deliver drugs across the blood brain barrier and can alter neuronal uptake mechanisms (Prog Neuropsychopharmacol Biol Psychiatry, 23, 941-949, 1999).

The invention also features the use of the composition comprising surface-modified liposomes containing poly (ethylene glycol) lipids (PEG-modified, branched and unbranched or combinations thereof, or long-circulating liposomes or stealth liposomes). Oligomers of the invention can also comprise covalently attached PEG molecules of various molecular weights. These formulations offer a method for increasing the accumulation of drugs in target tissues. This class of drug carriers resists opsonization and elimination by the mononuclear phagocytic system (MPS or RES), thereby enabling longer blood circulation times and enhanced tissue exposure for the encapsulated drug (Lasic et al. Chem. Rev. 1995, 95, 2601-2627; Ishiwata et al., Chem. Pharm. Bull. 1995, 43, 1005-1011). Such liposomes have been shown

to accumulate selectively in tumors, presumably by extravasation and capture in the neovascularized target tissues (Lasic et al., Science 1995, 267, 1275-1276; Oku et al., 1995, Biochim. Biophys. Acta, 1238, 86-90). The long-circulating liposomes enhance the pharmacokinetics and pharmacodynamics of DNA and RNA, particularly compared to conventional cationic liposomes which are known to accumulate in tissues of the MPS (Liu et al., J. Biol. Chem. 1995, 42, 24864-24870; Choi et al., International PCT Publication No. WO 96/10391; Ansell et al., International PCT Publication No. WO 96/10390; Holland et al., International PCT Publication No. WO 96/10392). Long-circulating liposomes are also likely to protect drugs from nuclease degradation to a greater extent compared to cationic liposomes, based on their ability to avoid accumulation in metabolically aggressive MPS tissues such as the liver and spleen.

In a further embodiment, the present invention includes oligomer compositions prepared for delivery as described in U.S. Pat. Nos. 6,692,911, 7,163,695 and 7,070,807. In this regard, in one embodiment, the present invention provides an oligomer of the present invention in a composition comprising copolymers of lysine and histidine (HK) (as described in U.S. Pat. Nos. 7,163,695, 7,070,807, and 6,692,911) either alone or in combination with PEG (e.g., branched or unbranched PEG or a mixture of both), in combination with PEG and a targeting moiety or any of the foregoing in combination with a crosslinking agent. In certain embodiments, the present invention provides antisense oligomers in compositions comprising gluconic-acid-modified polyhistidine or gluconylated-polyhistidine/transferrin-polylysine. One skilled in the art will also recognize that amino acids with properties similar to His and Lys may be substituted within the composition.

Certain embodiments of the oligomers described herein may contain a basic functional group, such as amino or alkylamino, and are, thus, capable of forming pharmaceutically-acceptable salts with pharmaceutically-acceptable acids. The term "pharmaceutically-acceptable salts" in this respect, refers to the relatively non-toxic, inorganic and organic acid addition salts of compounds of the present invention. These salts can be prepared in situ in the administration vehicle or the dosage form manufacturing process, or by separately reacting a purified compound of the invention in its free base form with a suitable organic or inorganic acid, and isolating the salt thus formed during subsequent purification. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, e.g., Berge et al. (1977) "Pharmaceutical Salts", J. Pharm. Sci. 66:1-19).

The pharmaceutically acceptable salts of the subject oligomers include the conventional nontoxic salts or quaternary ammonium salts of the compounds, e.g., from non-toxic organic or inorganic acids. For example, such conventional nontoxic salts include those derived from inorganic acids such as hydrochloride, hydrobromic, sulfuric, sulfamic, phosphoric, nitric, and the like; and the salts prepared from organic acids such as acetic, propionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like.

In certain embodiments, the oligomers of the present invention may contain one or more acidic functional groups and, thus, are capable of forming pharmaceutically-acceptable salts with pharmaceutically-acceptable bases. The term "pharmaceutically-acceptable salts" in these instances refers to the relatively non-toxic, inorganic and organic base addition salts of compounds of the present invention. These salts can likewise be prepared in situ in the administration vehicle or the dosage form manufacturing process, or by separately reacting the purified compound in its free acid form with a suitable base, such as the hydroxide, carbonate or bicarbonate of a pharmaceutically-acceptable metal cation, with ammonia, or with a pharmaceutically-acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like. (See, e.g., Berge et al., *supra*).

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient that can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect.

Generally, out of one hundred percent, this amount will range from about 0.1 percent to about ninety-nine percent of active ingredient, preferably from about 5 percent to about 70 percent, most preferably from about 10 percent to about 30 percent.

In certain embodiments, a formulation of the present invention comprises an excipient selected from cyclodextrins, celluloses, liposomes, micelle forming agents, e.g., bile acids, and polymeric carriers, e.g., polyesters and polyanhydrides; and an oligomer of the present invention. In certain embodiments, an aforementioned formulation renders orally bioavailable an oligomer of the present invention.

Methods of preparing these formulations or compositions include the step of bringing into association an oligomer of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. An oligomer of the present invention may also be administered as a bolus, electuary or paste.

In solid dosage forms of the invention for oral administration (capsules, tablets, pills, dragees, powders, granules, trouches and the like), the active ingredient may be mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds and surfactants, such as poloxamer and sodium lauryl sulfate; (7) wetting agents, such as, for example, cetyl alcohol, glycerol monostearate, and non-ionic surfactants; (8) absorbents, such as kaolin and bentonite clay; (9) lubricants, such as talc, calcium stearate, magnesium stearate, solid polyethylene glycols, sodium lauryl sulfate, zinc stearate, sodium stearate, stearic acid, and mixtures thereof; (10) coloring agents; and (11) controlled release agents such as crospovidone or ethyl cellulose. In the case of capsules, tablets and pills, the pharmaceutical compositions may also comprise buffering agents. Solid compositions of a similar type may also be employed as fillers in soft and hard-shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (e.g., gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, e.g., freeze-dried. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated

isostearyl alcohols, polyoxyethylene sorbitol and sorbitan esters, microcrystalline cellulose, aluminum metahydroxide, bentonite, agar-agar and tragacanth, and mixtures thereof.

Formulations for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations or dosage forms for the topical or transdermal administration of an oligomer as provided herein include powders, sprays, ointments, pastes, creams, lotions, gels, solutions, patches and inhalants. The active oligomers may be mixed under sterile conditions with a pharmaceutically-acceptable carrier, and with any preservatives, buffers, or propellants which may be required. The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to an oligomer of the present invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of an oligomer of the present invention to the body. Such dosage forms can be made by dissolving or dispersing the oligomer in the proper medium. Absorption enhancers can also be used to increase the flux of the agent across the skin. The rate of such flux can be controlled by either providing a rate controlling membrane or dispersing the agent in a polymer matrix or gel, among other methods known in the art.

Pharmaceutical compositions suitable for parenteral administration may comprise one or more oligomers of the invention in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents. Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the subject oligomers may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility, among other methods known in the art. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms may be made by forming microencapsule matrices of the subject oligomers in biodegradable polymers such as polylactide-polyglycolide. Depending on the ratio of oligomer to polymer, and the nature of the particular polymer employed, the rate of oligomer release can be controlled. Examples of other biodegradable polymers include poly(orthoesters) and poly(anhydrides). Depot injectable formulations may also prepared by entrapping the drug in liposomes or microemulsions that are compatible with body tissues.

When the oligomers of the present invention are administered as pharmaceuticals, to humans and animals, they can be given per se or as a pharmaceutical composition containing, for example, 0.1 to 99% (more preferably, 10 to 30%) of active ingredient in combination with a pharmaceutically acceptable carrier.

As noted above, the formulations or preparations of the present invention may be given orally, parenterally, topically, or rectally. They are typically given in forms suitable for each administration route. For example, they are administered in tablets or capsule form, by injection, inhalation, eye lotion, ointment, suppository, etc. administration by injection, infusion or inhalation; topical by lotion or ointment; and rectal by suppositories.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal,

subcutaneous, subcuticular, intraarticular, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

Regardless of the route of administration selected, the oligomers of the present invention, which may be used in a suitable hydrated form, and/or the pharmaceutical compositions of the present invention, may be formulated into pharmaceutically-acceptable dosage forms by conventional methods known to those of skill in the art. Actual dosage levels of the active ingredients in the pharmaceutical compositions of this invention may be varied so as to obtain an amount of the active ingredient which is effective to achieve the desired therapeutic response for a particular patient, composition, and mode of administration, without being unacceptably toxic to the patient.

The selected dosage level will depend upon a variety of factors including the activity of the particular oligomer of the present invention employed, or the ester, salt or amide thereof, the route of administration, the time of administration, the rate of excretion or metabolism of the particular oligomer being employed, the rate and extent of absorption, the duration of the treatment, other drugs, compounds and/or materials used in combination with the particular oligomer employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved. In general, a suitable daily dose of a compound of the invention will be that amount of the compound which is the lowest dose effective to produce a therapeutic effect. Such an effective dose will generally depend upon the factors described above. Generally, oral, intravenous, intracerebroventricular and subcutaneous doses of the compounds of this invention for a patient, when used for the indicated effects, will range from about 0.0001 to about 100 mg per kilogram of body weight per day.

If desired, the effective daily dose of the active compound may be administered as two, three, four, five, six or more sub-doses administered separately at appropriate intervals throughout the day, optionally, in unit dosage forms. In certain situations, dosing is one administration per day. In certain embodiments, dosing is one or more administration per every 2, 3, 4, 5, 6, 7, 8, 9, 10, 11, 12, 13, 14 days, or every 1, 2, 3, 4, 5, 6, 7, 8, 9, 10, 11, 12 weeks, or every 1, 2, 3, 4, 5, 6, 7, 8, 9, 10, 11, 12 months, as needed, to maintain the desired expression of a functional dystrophin protein.

Nucleic acid molecules can be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres, as described herein and known in the art. In certain embodiments, microemulsification technology may be utilized to improve bioavailability of lipophilic (water insoluble) pharmaceutical agents. Examples include Trimetrine (Dordunoo, S. K., et al., Drug Development and Industrial Pharmacy, 17(12), 1685-1713, 1991 and REV 5901 (Sheen, P. C., et al., J Pharm Sci 80(7), 712-714, 1991). Among other benefits, microemulsification provides enhanced bioavailability by preferentially directing absorption to the lymphatic system instead of the circulatory system, which thereby bypasses the liver, and prevents destruction of the compounds in the hepatobiliary circulation.

In one aspect of invention, the formulations contain micelles formed from an oligomer as provided herein and at least one amphiphilic carrier, in which the micelles have an average diameter of less than about 100 nm. More preferred embodiments provide micelles having an average diameter less than about 50 nm, and even more preferred embodiments provide micelles having an average diameter less than about 30 nm, or even less than about 20 nm.

While all suitable amphiphilic carriers are contemplated, the presently preferred carriers are generally those that have Generally-Recognized-as-Safe (GRAS) status, and that can both solubilize the compound of the present invention and microemulsify it at a later stage when the solution comes into a contact with a complex water phase (such as one found in human gastrointestinal tract). Usually, amphiphilic ingredients that satisfy these requirements have HLB (hydrophilic to lipophilic balance) values of 2-20, and their structures contain straight chain aliphatic radicals in the range of C-6 to C-20. Examples are polyethylene-glycolized fatty glycerides and polyethylene glycols.

Examples of amphiphilic carriers include saturated and monounsaturated polyethyleneglycolyzed fatty acid glycerides, such as those obtained from fully or partially hydrogenated various vegetable oils. Such oils may advantageously consist of tri-, di-, and mono-fatty acid glycerides and di- and mono-polyethyleneglycol esters of the corresponding fatty acids, with a particularly preferred fatty acid composition including capric acid 4-10, capric acid 3-9, lauric acid 40-50, myristic acid 14-24, palmitic acid 4-14 and stearic acid 5-15%. Another useful class of amphiphilic carriers includes partially esterified sorbitan and/or sorbitol, with saturated or mono-unsaturated fatty acids (SPAN-series) or corresponding ethoxylated analogs (TWEEN-series).

Commercially available amphiphilic carriers may be particularly useful, including Gelucire-series, Labrafil, Labrasol, or Lauroglycol (all manufactured and distributed by Gattefosse Corporation, Saint Priest, France), PEG-monooleate, PEG-di-oleate, PEG-mono-laurate and dilaurate, Lecithin, Polysorbate 80, etc (produced and distributed by a number of companies in USA and worldwide).

In certain embodiments, the delivery may occur by use of liposomes, nanocapsules, microparticles, microspheres, lipid particles, vesicles, and the like, for the introduction of the compositions of the present invention into suitable host cells. In particular, the compositions of the present invention may be formulated for delivery either encapsulated in a lipid particle, a liposome, a vesicle, a nanosphere, a nanoparticle or the like. The formulation and use of such delivery vehicles can be carried out using known and conventional techniques.

Hydrophilic polymers suitable for use in the present invention are those which are readily water-soluble, can be covalently attached to a vesicle-forming lipid, and which are tolerated in vivo without toxic effects (i.e., are biocompatible). Suitable polymers include polyethylene glycol (PEG), polylactic (also termed polylactide), polyglycolic acid (also termed polyglycolide), a polylactic-polyglycolic acid copolymer, and polyvinyl alcohol. In certain embodiments, polymers have a molecular weight of from about 100 or 120 daltons up to about 5,000 or 10,000 daltons, or from about 300 daltons to about 5,000 daltons. In other embodiments, the polymer is polyethyleneglycol having a molecular weight of from about 100 to about 5,000 daltons, or having a molecular weight of from about 300 to about 5,000 daltons. In certain embodiments, the polymer is polyethyleneglycol of 750 daltons (PEG(750)). Polymers may also be defined by the number of monomers therein; a preferred embodiment of the present invention utilizes polymers of at least about three monomers, such PEG polymers consisting of three monomers (approximately 150 daltons).

Other hydrophilic polymers which may be suitable for use in the present invention include polyvinylpyrrolidone, polymethoxazoline, polyethyloxazoline, polyhydroxypropyl methacrylamide, polymethacrylamide, polydimethylacrylamide, and derivatized celluloses such as hydroxymethylcellulose or hydroxyethylcellulose.

In certain embodiments, a formulation of the present invention comprises a biocompatible polymer selected from the group consisting of polyamides, polycarbonates, polyalkylenes, polymers of acrylic and methacrylic esters, polyvinyl polymers, polyglycolides, polysiloxanes, polyurethanes and co-polymers thereof, celluloses, polypropylene, polyethylenes, polystyrene, polymers of lactic acid and glycolic acid, polyanhydrides, poly(ortho)esters, poly(butic acid), poly(valeric acid), poly(lactide-co-caprolactone), polysaccharides, proteins, polyhyaluronic acids, polycyanoacrylates, and blends, mixtures, or copolymers thereof.

Cyclodextrins are cyclic oligosaccharides, consisting of 6, 7 or 8 glucose units, designated by the Greek letter $\alpha$, $\beta$, or $\gamma$, respectively. The glucose units are linked by $\alpha$-1,4-glucosidic bonds. As a consequence of the chair conformation of the sugar units, all secondary hydroxyl groups (at C-2, C-3) are located on one side of the ring, while all the primary hydroxyl groups at C-6 are situated on the other side. As a result, the external faces are hydrophilic, making the cyclodextrins water-soluble. In contrast, the cavities of the cyclodextrins are hydrophobic, since they are lined by the hydrogen of atoms C-3 and C-5, and by ether-like oxygens. These matrices allow complexation with a variety of relatively hydrophobic compounds, including, for instance, steroid compounds such as 17$\alpha$-estradiol (see, e.g., van Uden et al. Plant Cell Tiss. Org. Cult. 38:1-3-113 (1994)). The complexation takes place by Van der Waals interactions and by hydrogen bond formation. For a general review of the chemistry of cyclodextrins, see, Wenz, Agnew. Chem. Int. Ed. Engl., 33:803-822 (1994).

The physico-chemical properties of the cyclodextrin derivatives depend strongly on the kind and the degree of substitution. For example, their solubility in water ranges from insoluble (e.g., triacetyl-beta-cyclodextrin) to 147% soluble (w/v) (G-2-beta-cyclodextrin). In addition, they are soluble in many organic solvents. The properties of the cyclodextrins enable the control over solubility of various formulation components by increasing or decreasing their solubility.

Numerous cyclodextrins and methods for their preparation have been described. For example, Parmeter (I), et al. (U.S. Pat. No. 3,453,259) and Gramera, et al. (U.S. Pat. No. 3,459,731) described electroneutral cyclodextrins. Other derivatives include cyclodextrins with cationic properties [Parmeter (II), U.S. Pat. No. 3,453,257], insoluble crosslinked cyclodextrins (Solms, U.S. Pat. No. 3,420,788), and cyclodextrins with anionic properties [Parmeter (III), U.S. Pat. No. 3,426,011]. Among the cyclodextrin derivatives with anionic properties, carboxylic acids, phosphorous acids, phosphinous acids, phosphonic acids, phosphoric acids, thiophosphonic acids, thiosulphinic acids, and sulfonic acids have been appended to the parent cyclodextrin [see, Parmeter (III), supra]. Furthermore, sulfoalkyl ether cyclodextrin derivatives have been described by Stella, et al. (U.S. Pat. No. 5,134,127).

Liposomes consist of at least one lipid bilayer membrane enclosing an aqueous internal compartment. Liposomes may be characterized by membrane type and by size. Small unilamellar vesicles (SUVs) have a single membrane and typically range between 0.02 and 0.05 $\mu$m in diameter; large unilamellar vesicles (LUVS) are typically larger than 0.05 $\mu$m. Oligolamellar large vesicles and multilamellar vesicles have multiple, usually concentric, membrane layers and are typically larger than 0.1 $\mu$m. Liposomes with several nonconcentric membranes, i.e., several smaller vesicles contained within a larger vesicle, are termed multivesicular vesicles.

One aspect of the present invention relates to formulations comprising liposomes containing an oligomer of the present invention, where the liposome membrane is formulated to provide a liposome with increased carrying capacity. Alternatively or in addition, the compound of the present invention may be contained within, or adsorbed onto, the

liposome bilayer of the liposome. An oligomer of the present invention may be aggregated with a lipid surfactant and carried within the liposome's internal space; in these cases, the liposome membrane is formulated to resist the disruptive effects of the active agent-surfactant aggregate.

According to one embodiment of the present invention, the lipid bilayer of a liposome contains lipids derivatized with polyethylene glycol (PEG), such that the PEG chains extend from the inner surface of the lipid bilayer into the interior space encapsulated by the liposome, and extend from the exterior of the lipid bilayer into the surrounding environment.

Active agents contained within liposomes of the present invention are in solubilized form. Aggregates of surfactant and active agent (such as emulsions or micelles containing the active agent of interest) may be entrapped within the interior space of liposomes according to the present invention. A surfactant acts to disperse and solubilize the active agent, and may be selected from any suitable aliphatic, cycloaliphatic or aromatic surfactant, including but not limited to biocompatible lysophosphatidylcholines (LPGs) of varying chain lengths (for example, from about C14 to about C20). Polymer-derivatized lipids such as PEG-lipids may also be utilized for micelle formation as they will act to inhibit micelle/membrane fusion, and as the addition of a polymer to surfactant molecules decreases the CMC of the surfactant and aids in micelle formation. Preferred are surfactants with CMOs in the micromolar range; higher CMC surfactants may be utilized to prepare micelles entrapped within liposomes of the present invention.

Liposomes according to the present invention may be prepared by any of a variety of techniques that are known in the art. See, e.g., U.S. Pat. No. 4,235,871; Published PCT applications WO 96/14057; New RRC, Liposomes: A practical approach, IRL Press, Oxford (1990), pages 33-104; Lasic DD, Liposomes from physics to applications, Elsevier Science Publishers BV, Amsterdam, 1993. For example, liposomes of the present invention may be prepared by diffusing a lipid derivatized with a hydrophilic polymer into preformed liposomes, such as by exposing preformed liposomes to micelles composed of lipid-grafted polymers, at lipid concentrations corresponding to the final mole percent of derivatized lipid which is desired in the liposome. Liposomes containing a hydrophilic polymer can also be formed by homogenization, lipid-field hydration, or extrusion techniques, as are known in the art.

In another exemplary formulation procedure, the active agent is first dispersed by sonication in a lysophosphatidylcholine or other low CMC surfactant (including polymer grafted lipids) that readily solubilizes hydrophobic molecules. The resulting micellar suspension of active agent is then used to rehydrate a dried lipid sample that contains a suitable mole percent of polymer-grafted lipid, or cholesterol. The lipid and active agent suspension is then formed into liposomes using extrusion techniques as are known in the art, and the resulting liposomes separated from the unencapsulated solution by standard column separation.

In one aspect of the present invention, the liposomes are prepared to have substantially homogeneous sizes in a selected size range. One effective sizing method involves extruding an aqueous suspension of the liposomes through a series of polycarbonate membranes having a selected uniform pore size; the pore size of the membrane will correspond roughly with the largest sizes of liposomes produced by extrusion through that membrane. See e.g., U.S. Pat. No. 4,737,323 (Apr. 12, 1988). In certain embodiments, reagents such as DharmaFECT® and Lipofectamine® may be utilized to introduce polynucleotides or proteins into cells.

The release characteristics of a formulation of the present invention depend on the encapsulating material, the concentration of encapsulated drug, and the presence of release modifiers. For example, release can be manipulated to be pH dependent, for example, using a pH sensitive coating that releases only at a low pH, as in the stomach, or a higher pH, as in the intestine. An enteric coating can be used to prevent release from occurring until after passage through the stomach. Multiple coatings or mixtures of cyanamide encapsulated in different materials can be used to obtain an initial release in the stomach, followed by later release in the intestine. Release can also be manipulated by inclusion of salts or pore forming agents, which can increase water uptake or release of drug by diffusion from the capsule. Excipients which modify the solubility of the drug can also be used to control the release rate. Agents which enhance degradation of the matrix or release from the matrix can also be incorporated. They can be added to the drug, added as a separate phase (i.e., as particulates), or can be co-dissolved in the polymer phase depending on the compound. In most cases the amount should be between 0.1 and thirty percent (w/w polymer). Types of degradation enhancers include inorganic salts such as ammonium sulfate and ammonium chloride, organic acids such as citric acid, benzoic acid, and ascorbic acid, inorganic bases such as sodium carbonate, potassium carbonate, calcium carbonate, zinc carbonate, and zinc hydroxide, and organic bases such as protamine sulfate, spermine, choline, ethanolamine, diethanolamine, and triethanolamine and surfactants such as Tween® and Pluronic®. Pore forming agents which add microstructure to the matrices (i.e., water soluble compounds such as inorganic salts and sugars) are added as particulates. The range is typically between one and thirty percent (w/w polymer).

Uptake can also be manipulated by altering residence time of the particles in the gut. This can be achieved, for example, by coating the particle with, or selecting as the encapsulating material, a mucosal adhesive polymer. Examples include most polymers with free carboxyl groups, such as chitosan, celluloses, and especially polyacrylates (as used herein, polyacrylates refers to polymers including acrylate groups and modified acrylate groups such as cyanoacrylates and methacrylates).

An oligomer may be formulated to be contained within, or, adapted to release by a surgical or medical device

or implant. In certain aspects, an implant may be coated or otherwise treated with an oligomer. For example, hydrogels, or other polymers, such as biocompatible and/or biodegradable polymers, may be used to coat an implant with the compositions of the present invention (i.e., the composition may be adapted for use with a medical device by using a hydrogel or other polymer). Polymers and copolymers for coating medical devices with an agent are well-known in the art. Examples of implants include, but are not limited to, stents, drug-eluting stents, sutures, prosthesis, vascular catheters, dialysis catheters, vascular grafts, prosthetic heart valves, cardiac pacemakers, implantable cardioverter defibrillators, IV needles, devices for bone setting and formation, such as pins, screws, plates, and other devices, and artificial tissue matrices for wound healing.

In addition to the methods provided herein, the oligomers for use according to the invention may be formulated for administration in any convenient way for use in human or veterinary medicine, by analogy with other pharmaceuticals. The antisense oligomers and their corresponding formulations may be administered alone or in combination with other therapeutic strategies in the treatment of muscular dystrophy, such as myoblast transplantation, stem cell therapies, administration of aminoglycoside antibiotics, proteasome inhibitors, and up-regulation therapies (e.g., upregulation of utrophin, an autosomal paralogue of dystrophin).

The routes of administration described are intended only as a guide since a skilled practitioner will be able to determine readily the optimum route of administration and any dosage for any particular animal and condition. Multiple approaches for introducing functional new genetic material into cells, both in vitro and in vivo have been attempted (Friedmann (1989) Science, 244:1275-1280). These approaches include integration of the gene to be expressed into modified retroviruses (Friedmann (1989) supra; Rosenberg (1991) Cancer Research 51(18), suppl.: 5074S-5079S); integration into non-retrovirus vectors (e.g., adeno-associated viral vectors) (Rosenfeld, et al. (1992) Cell, 68:143-155; Rosenfeld, et al. (1991) Science, 252:431-434); or delivery of a transgene linked to a heterologous promoter-enhancer element via liposomes (Friedmann (1989), supra; Brigham, et al. (1989) Am. J. Med. Sci., 298:278-281; Nabel, et al. (1990) Science, 249:1285-1288; Hazinski, et al. (1991) Am. J. Resp. Cell Molec. Biol., 4:206-209; and Wang and Huang (1987) Proc. Natl. Acad. Sci. (USA), 84:7851-7855); coupled to ligand-specific, cation-based transport systems (Wu and Wu (1988) J. Biol. Chem., 263:14621-14624) or the use of naked DNA, expression vectors (Nabel et al. (1990), supra); Wolff et al. (1990) Science, 247:1465-1468). Direct injection of transgenes into tissue produces only localized expression (Rosenfeld (1992) supra); Rosenfeld et al. (1991) supra; Brigham et al. (1989) supra; Nabel (1990) supra; and Hazinski et al. (1991) supra). The Brigham et al. group (Am. J. Med. Sci. (1989) 298:278-281 and Clinical Research (1991) 39 (abstract)) have reported in vivo transfection only of lungs of mice following either intravenous or intratracheal administration of a DNA liposome complex. An example of a review article of human gene therapy procedures is: Anderson, Science (1992) 256:808-813.

## IV. Kits

The invention also provides kits for treatment of a patient with a genetic disease which kit comprises at least an antisense molecule (e.g., an antisense oligomer set forth in SEQ ID NOs: 1-5), packaged in a suitable container, together with instructions for its use. The kits may also contain peripheral reagents such as buffers, stabilizers, etc. Those of ordinary skill in the field should appreciate that applications of the above method has wide application for identifying antisense molecules suitable for use in the treatment of many other diseases.

## V. Examples

Although the foregoing invention has been described in some detail by way of illustration and example for purposes of clarity of understanding, it will be readily apparent to one of ordinary skill in the art in light of the teachings of this invention that certain changes and modifications may be made thereto without departing from the spirit or scope of the appended claims. The following examples are provided by way of illustration only and not by way of limitation. Those of skill in the art will readily recognize a variety of noncritical parameters that could be changed or modified to yield essentially similar results.

### Materials and Methods

*Cells and Tissue Culture Treatment Conditions*

Human Rhabdomyosarcoma cells (ATCC, CCL-136; RD cells) were seeded into tissue culture-treated T75 flasks (Nunc) at 1.5 x $10^6$ cells/flask in 24 mL of warmed DMEM with L-Glutamine (HyClone), 10% fetal bovine serum, and 1% Penicillin-Streptomycin antibiotic solution (CelGro); after 24 hours, media was aspirated, cells were washed once in warmed PBS, and fresh media was added. Cells were grown to 80% confluence in a 37°C incubator at 5.0% CO2 and harvested using trypsin. Lyophilized phosphorodiamidate morpholino oligomers (PMOs) were resuspended

at approximately 0.5 to 2.0 mM in nuclease-free water; to verify molarity, PMO solutions were measured using a NanoDrop 2000 spectrophotometer (Thermo Scientific). PMOs were delivered to RD cells using nucleoporation according to the manufacturer's instructions and the SG kit (Lonza). PMOs were tested at various concentrations as indicated (e.g., 2.5, 5, 10, 12.5, 20 and 25 micromolar). Cells were incubated for 24 hours post nucleoporation at approximately 2 - 3 X $10^5$ cells per well of a 12 or 24-well plate (n=2 or 3) and then subjected to RNA extraction as described below.

Primary human myoblasts were cultured in Skeletal Muscle Cell Growth Media (PromoCell) using standard techniques. Nucleoporation of the PMOs at various concentrations was done as described for RD cells above. Cells were then plated in triplicate wells of a 12-well plate in PromoCell growth media and allowed to incubate for 24 hours before RNA extraction as described below.

*RNA Extraction and PCR Amplification*

RNA was extracted from PMO-treated cells (RD cells or primary human myoblasts) using the RNAspin 96 well RNA isolation kit from GE Healthcare and subjected to nested RT-PCR using standard techniques and the following primer pairs. Outer primers: forward 5'-CTTGGACAGAACTTACCGACTGG-3' (SEQ ID NO: 22), reverse 5'-GTTTCTTCCAAAGCAGCCTCTCG -3'(SEQ ID NO: 23); inner primers: forward 5'-GCAGGATTTGGAACAGAGGCG-3'(SEQ ID NO: 25), reverse 5'-CATCTACATTTGTCTGCCACTGG-3'(SEQ ID NO: 25). Exon skipping was measured by gel electrophoresis or by using the Caliper LabChip bioanalyzer and the % exon skipping (i.e., band intensity of the exon-skipped product relative to the full length PCR product) was graphed as shown in **FIGs 3-5.**

*Preparation of Morpholino Subunits, PMO and PMO with Modified Intersubunit Linkages*

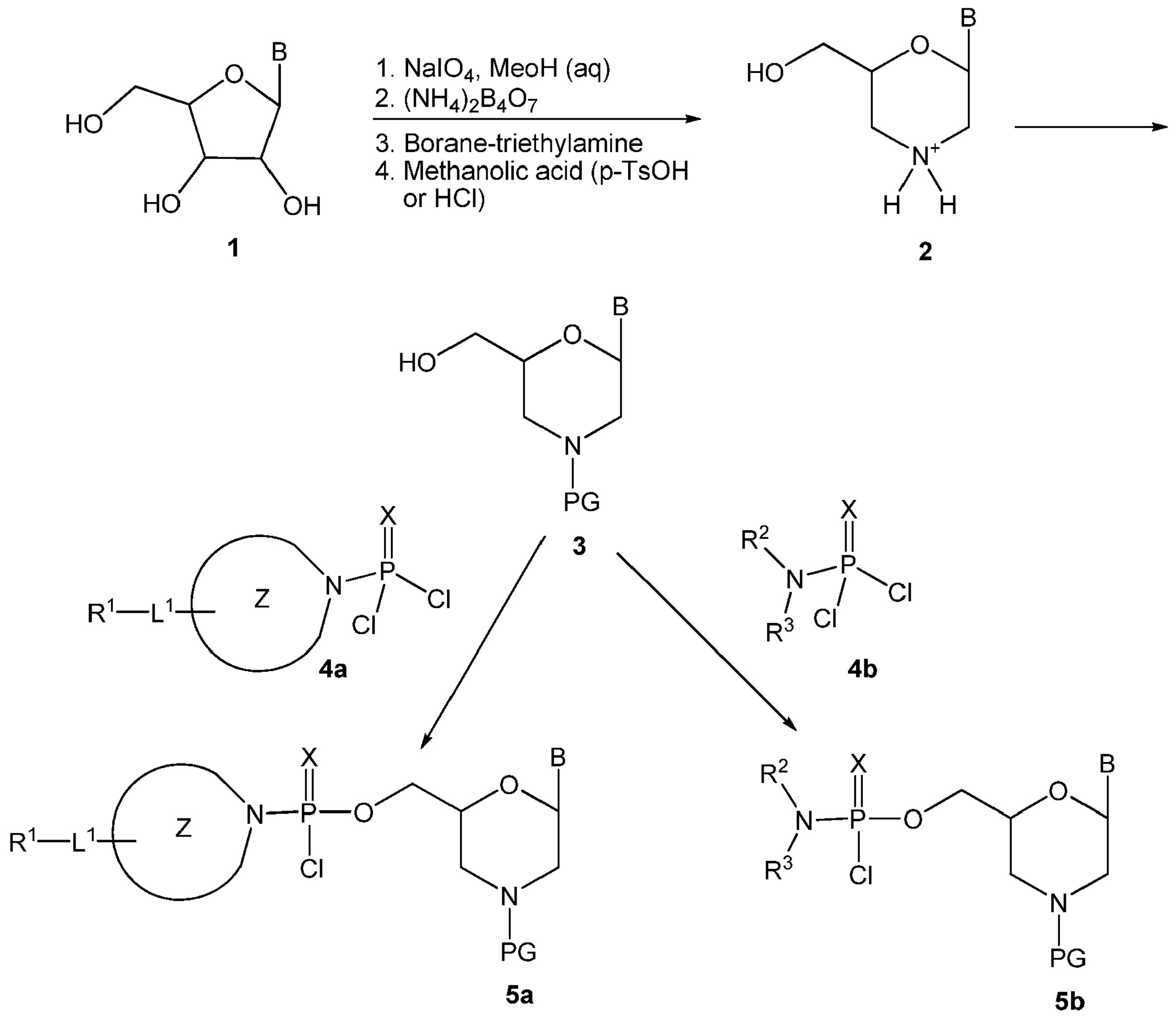


1 2 3 4a 4b 5a 5b

Scheme 1: General synthetic route to PMO and modified-PMO Subunits

Referring to Reaction Scheme 1, wherein B represents a base pairing moiety and PG represents a protecting group, the morpholino subunits may be prepared from the corresponding ribinucleoside (**1**) as shown. The morpholino subunit (2) may be optionally protected by reaction with a suitable protecting group precursor, for example trityl chloride. The 3' protecting group is generally removed during solid-state oligomer synthesis as described in more detail below. The base pairing moiety may be suitably protected for solid-phase oligomer synthesis. Suitable protecting groups include benzoyl for adenine and cytosine, phenylacetyl for guanine, and pivaloyloxymethyl for hypoxanthine (I). The pivaloyloxymethyl group can be introduced onto the N1 position of the hypoxanthine heterocyclic base. Although an unprotected hypoxanthine subunit, may be employed, yields in activation reactions are far superior when the base is protected. Other suitable protecting groups include those disclosed in U.S. Patent No. 8,076,476, which is hereby incorporated by reference in its entirety.

Reaction of **3** with the activated phosphorous compound **4a** or **4b** results in morpholino subunits having the desired linkage moiety (**5a** or **5b**). It should be noted that the $R^1$ and/or $L^1$ moieties may also be installed on the heterocyclic ring Z after formation of the P-O bond or even after the subunit has been incorporated into an oligomer.

Compounds of structure **4a** or **4b** can be prepared using any number of methods known to those of skill in the art, including those described in the examples. Coupling with the morpholino moiety then proceeds as outlined above.

Compounds of structure **5a** or **5b** can be used in solid-phase oligomer synthesis for preparation of oligomers comprising the intersubunit linkages. Such methods are well known in the art. Briefly, a compound of structure **5a** or **5b** may be modified at the 5' end to contain a linker to a solid support. Once supported, the protecting group of **5a** or **5b** (e.g., trityl at 3'-end)) is removed and the free amine is reacted with an activated phosphorous moiety of a second

compound of structure **5** (or analogue thereof). This sequence is repeated until the desired length oligo is obtained. The protecting group in the terminal 3' end may either be removed or left on if a 3' modification is desired. The oligo can be removed from the solid support using any number of methods, or example treatment with a base to cleave the linkage to the solid support.

The preparation of morpholino oligomers in general and specific morpholino oligomers of the invention are described in more detail in the Examples.

**Example 1**

**Preparation of Morpholino Oligomers**

The preparation of the compounds of the invention are performed using the following protocol:
Preparation of trityl piperazine phenyl carbamate 35 (**FIG. 2**): To a cooled suspension of compound 11 in dichloromethane (6 mL/g 11) was added a solution of potassium carbonate (3.2 eq) in water (4 mL/g potassium carbonate). To this two-phase mixture was slowly added a solution of phenyl chloroformate (1.03 eq) in dichloromethane (2 g/g phenyl chloroformate). The reaction mixture was warmed to 20 °C. Upon reaction completion (1-2 hr), the layers were separated. The organic layer was washed with water, and dried over anhydrous potassium carbonate. The product 35 was isolated by crystallization from acetonitrile.

Preparation of carbamate alcohol 36: Sodium hydride (1.2 eq) was suspended in 1-methyl-2-pyrrolidinone (32 mL/g sodium hydride). To this suspension were added triethylene glycol (10.0 eq) and compound 35 (1.0 eq). The resulting slurry was heated to 95 °C. Upon reaction completion (1-2 hr), the mixture was cooled to 20 °C. To this mixture was added 30% dichloromethane/methyl tert-butyl ether (v:v) and water. The product-containing organic layer was washed successively with aqueous NaOH, aqueous succinic acid, and saturated aqueous sodium chloride. The product 36 was isolated by crystallization from dichloromethane/methyl tert-butyl ether/heptane.

Preparation of Tail acid 37: To a solution of compound 36 in tetrahydrofuran (7 mL/g 36) was added succinic anhydride (2.0 eq) and DMAP (0.5 eq). The mixture was heated to 50 °C. Upon reaction completion (5 hr), the mixture was cooled to 20 °C and adjusted to pH 8.5 with aqueous $NaHCO_3$. Methyl tert-butyl ether was added, and the product was extracted into the aqueous layer. Dichloromethane was added, and the mixture was adjusted to pH 3 with aqueous citric acid. The product-containing organic layer was washed with a mixture of pH=3 citrate buffer and saturated aqueous sodium chloride. This dichloromethane solution of 37 was used without isolation in the preparation of compound 38.

Preparation of 38: To the solution of compound 37 was added N-hydroxy-5-norbornene-2,3-dicarboxylic acid imide (HONB) (1.02 eq), 4-dimethylaminopyridine (DMAP) (0.34 eq), and then 1-(3-dimethylaminopropyl)-N'-ethylcarbodiimide hydrochloride (EDC) (1.1 eq). The mixture was heated to 55 °C. Upon reaction completion (4-5 hr), the mixture was cooled to 20 °C and washed successively with 1:1 0.2 M citric acid/brine and brine. The dichloromethane solution underwent solvent exchange to acetone and then to N,N-dimethylformamide, and the product was isolated by precipitation from acetone/ N,N-dimethylformamide into saturated aqueous sodium chloride. The crude product was reslurried several times in water to remove residual N,N-dimethylformamide and salts.

Introduction of the activated "Tail" onto the anchor-loaded resin was performed in dimethyl imidazolidinone (DMI) by the procedure used for incorporation of the subunits during solid phase synthesis.

Preparation of the Solid Support for Synthesis of Morpholino Oligomers: This procedure was performed in a silanized, jacketed peptide vessel (ChemGlass, NJ, USA) with a coarse porosity (40-60 μm) glass frit, overhead stirrer, and 3-way Teflon stopcock to allow $N_2$ to bubble up through the frit or a vacuum extraction.

The resin treatment/wash steps in the following procedure consist of two basic operations: resin fluidization or stirrer bed reactor and solvent/solution extraction. For resin fluidization, the stopcock was positioned to allow $N_2$ flow up through the frit and the specified resin treatment/wash was added to the reactor and allowed to permeate and completely wet the resin. Mixing was then started and the resin slurry mixed for the specified time. For solvent/solution extraction, mixing and $N_2$ flow were stopped and the vacuum pump was started and then the stopcock was positioned to allow evacuation of resin treatment/wash to waste. All resin treatment/wash volumes were 15 mL/g of resin unless noted otherwise.

To aminomethylpolystyrene resin (100-200 mesh; ~1.0 mmol/g load based on nitrogen substitution; 75 g, 1 eq, Polymer Labs, UK, part #1464-X799) in a silanized, jacketed peptide vessel was added 1-methyl-2-pyrrolidinone (NMP; 20 ml/g resin) and the resin was allowed to swell with mixing for 1-2 hr. Following evacuation of the swell solvent, the resin was washed with dichloromethane (2 x 1-2 min), 5% diisopropylethylamine in 25% isopropanol/dichloromethane (2 x 3-4 min) and dichloromethane (2 x 1-2 min). After evacuation of the final wash, the resin was treated with a solution of disulfide anchor 34 in 1-methyl-2-pyrrolidinone (0.17 M; 15 mL/g resin, ~2.5 eq) and the resin/reagent mixture was heated at 45 °C for 60 hr. On reaction completion, heating was discontinued and the anchor solution was evacuated and the resin washed with 1-methyl-2-pyrrolidinone (4 x 3-4 min) and dichloromethane (6 x 1-2 min). The resin was treated with a solution of 10% (v/v) diethyl dicarbonate in dichloromethane (16 mL/g; 2 x 5-6 min) and then washed with

dichloromethane (6 x 1-2 min). The resin 39 (see FIG. 4) was dried under a N2 stream for 1-3 hr and then under vacuum to constant weight (± 2%). Yield: 110-150% of the original resin weight.

Determination of the Loading of Aminomethylpolystyrene-disulfide resin: The loading of the resin (number of potentially available reactive sites) is determined by a spectrometric assay for the number of triphenylmethyl (trityl) groups per gram of resin.

A known weight of dried resin (25 ± 3 mg) is transferred to a silanized 25 ml volumetric flask and ~5 mL of 2% (v/v) trifluoroacetic acid in dichloromethane is added. The contents are mixed by gentle swirling and then allowed to stand for 30 min. The volume is brought up to 25 mL with additional 2% (v/v) trifluoroacetic acid in dichloromethane and the contents thoroughly mixed. Using a positive displacement pipette, an aliquot of the trityl-containing solution (500 μL) is transferred to a 10 mL volumetric flask and the volume brought up to 10 mL with methanesulfonic acid.

The trityl cation content in the final solution is measured by UV absorbance at 431.7 nm and the resin loading calculated in trityl groups per gram resin (μmol/g) using the appropriate volumes, dilutions, extinction coefficient (ε: 41 μmol-1cm-1) and resin weight. The assay is performed in triplicate and an average loading calculated.

The resin loading procedure in this example will provide resin with a loading of approximately 500 μmol/g. A loading of 300-400 in μmol/g was obtained if the disulfide anchor incorporation step is performed for 24 hr at room temperature.

Tail loading: Using the same setup and volumes as for the preparation of aminomethylpolystyrene-disulfide resin, the Tail can be introduced into solid support. The anchor loaded resin was first deprotected under acidic condition and the resulting material neutralized before coupling. For the coupling step, a solution of 38 (0.2 M) in DMI containing 4-ethylmorpholine (NEM, 0.4 M) was used instead of the disulfide anchor solution. After 2 hr at 45 °C, the resin 39 was washed twice with 5% diisopropylethylamine in 25% isopropanol/dichloromethane and once with DCM. To the resin was added a solution of benzoic anhydride (0.4 M) and NEM (0.4 M). After 25 min, the reactor jacket was cooled to room temperature, and the resin washed twice with 5% diisopropylethylamine in 25% isopropanol/dichloromethane and eight times with DCM. The resin 40 was filtered and dried under high vacuum. The loading for resin 40 is defined to be the loading of the original aminomethylpolystyrene-disulfide resin 39 used in the Tail loading.

Solid Phase Synthesis: Morpholino Oligomers were prepared on a Gilson AMS-422 Automated Peptide Synthesizer in 2 mL Gilson polypropylene reaction columns (Part # 3980270). An aluminum block with channels for water flow was placed around the columns as they sat on the synthesizer. The AMS-422 will alternatively add reagent/wash solutions, hold for a specified time, and evacuate the columns using vacuum.

For oligomers in the range up to about 25 subunits in length, aminomethylpolystyrene-disulfide resin with loading near 500 μmol/g of resin is preferred. For larger oligomers, aminomethylpolystyrene-disulfide resin with loading of 300-400 μmol/g of resin is preferred. If a molecule with 5'-Tail is desired, resin that has been loaded with Tail is chosen with the same loading guidelines.

The following reagent solutions were prepared:

Detritylation Solution: 10% Cyanoacetic Acid (w/v) in 4:1 dichloromethane/acetonitrile; Neutralization Solution: 5% Diisopropylethylamine in 3:1 dichloromethane/isopropanol; Coupling Solution: 0.18 M (or 0.24 M for oligomers having grown longer than 20 subunits) activated Morpholino Subunit of the desired base and linkage type and 0.4 M N ethylmorpholine, in 1,3-dimethylimidazolidinone. Dichloromethane (DCM) was used as a transitional wash separating the different reagent solution washes.

On the synthesizer, with the block set to 42 °C, to each column containing 30 mg of aminomethylpolystyrene-disulfide resin (or Tail resin) was added 2 mL of 1-methyl-2-pyrrolidinone and allowed to sit at room temperature for 30 min. After washing with 2 times 2 mL of dichloromethane, the following synthesis cycle was employed:

| Step | Volume | Delivery | Hold time |
|---|---|---|---|
| Detritylation | 1.5 mL | Manifold | 15 seconds |
| Detritylation | 1.5 mL | Manifold | 15 seconds |
| Detritylation | 1.5 mL | Manifold | 15 seconds |
| Detritylation | 1.5 mL | Manifold | 15 seconds |
| Detritylation | 1.5 mL | Manifold | 15 seconds |
| Detritylation | 1.5 mL | Manifold | 15 seconds |
| Detritylation | 1.5 mL | Manifold | 15 seconds |
| DCM | 1.5 mL | Manifold | 30 seconds |
| Neutralization | 1.5 mL | Manifold | 30 seconds |
| Neutralization | 1.5 mL | Manifold | 30 seconds |
| Neutralization | 1.5 mL | Manifold | 30 seconds |
| Neutralization | 1.5 mL | Manifold | 30 seconds |

(continued)

| Step | Volume | Delivery | Hold time |
|---|---|---|---|
| Neutralization | 1.5 mL | Manifold | 30 seconds |
| Neutralization | 1.5 mL | Manifold | 30 seconds |
| DCM | 1.5 mL | Manifold | 30 seconds |
| Coupling | 350-500uL | Syringe | 40 minutes |
| DCM | 1.5 mL | Manifold | 30 seconds |
| Neutralization | 1.5 mL | Manifold | 30 seconds |
| Neutralization | 1.5 mL | Manifold | 30 seconds |
| DCM | 1.5 mL | Manifold | 30 seconds |
| DCM | 1.5 mL | Manifold | 30 seconds |
| DCM | 1.5 mL | Manifold | 30 seconds |

The sequences of the individual oligomers were programmed into the synthesizer so that each column receives the proper coupling solution (A,C,G,T,I) in the proper sequence. When the oligomer in a column had completed incorporation of its final subunit, the column was removed from the block and a final cycle performed manually with a coupling solution comprised of 4-methoxytriphenylmethyl chloride (0.32 M in DMI) containing 0.89 M 4-ethylmorpholine.

Cleavage from the resin and removal of bases and backbone protecting groups: After methoxytritylation, the resin was washed 8 times with 2 mL 1-methyl-2-pyrrolidinone. One mL of a cleavage solution consisting of 0.1 M 1,4-dithiothreitol (DTT) and 0.73 M triethylamine in 1-methyl-2-pyrrolidinone was added, the column capped, and allowed to sit at room temperature for 30 min. After that time, the solution was drained into a 12 mL Wheaton vial. The greatly shrunken resin was washed twice with 300 μL of cleavage solution. To the solution was added 4.0 mL conc. aqueous ammonia (stored at -20 °C), the vial capped tightly (with Teflon lined screw cap), and the mixture swirled to mix the solution. The vial was placed in a 45 °C oven for 16-24 hr to effect cleavage of base and backbone protecting groups.

Crude product purification: The vialed ammonolysis solution was removed from the oven and allowed to cool to room temperature. The solution was diluted with 20 mL of 0.28% aqueous ammonia and passed through a 2.5x10 cm column containing Macroprep HQ resin (BioRad). A salt gradient (A: 0.28% ammonia with B: 1 M sodium chloride in 0.28% ammonia; 0-100% B in 60 min) was used to elute the methoxytrityl containing peak. The combined fractions were pooled and further processed depending on the desired product.

Demethoxytritylation of Morpholino Oligomers: The pooled fractions from the Macroprep purification were treated with 1 M H3PO4 to lower the pH to 2.5. After initial mixing, the samples sat at room temperature for 4 min, at which time they are neutralized to pH 10-11 with 2.8% ammonia/water. The products were purified by solid phase extraction (SPE).

SPE column packing and conditioning: Amberchrome CG-300M (Rohm and Haas; Philadelphia, PA) (3 mL) is packed into 20 mL fritted columns (BioRad Econo-Pac Chromatography Columns (732-1011)) and the resin rinsed with 3 mL of the following: 0.28% NH4OH/80% acetonitrile; 0.5M NaOH/20%ethanol; water; 50 mM H3PO4/80% acetonitrile; water; 0.5 NaOH/20% ethanol; water; 0.28% NH4OH.

SPE purification: The solution from the demethoxytritylation was loaded onto the column and the resin rinsed three times with 3-6 mL 0.28% aqueous ammonia. A Wheaton vial (12 mL) was placed under the column and the product eluted by two washes with 2 mL of 45% acetonitrile in 0.28% aqueous ammonia.

Product isolation: The solutions were frozen in dry ice and the vials placed in a freeze dryer to produce a fluffy white powder. The samples were dissolved in water, filtered through a 0.22 micron filter (Pall Life Sciences, Acrodisc 25 mm syringe filter, with a 0.2 micron HT Tuffryn membrane) using a syringe and the Optical Density (OD) was measured on a UV spectrophotometer to determine the OD units of oligomer present, as well as dispense sample for analysis. The solutions were then placed back in Wheaton vials for lyophilization.

Analysis of Morpholino Oligomers by MALDI: MALDI-TOF mass spectrometry was used to determine the composition of fractions in purifications as well as provide evidence for identity (molecular weight) of the oligomers. Samples were run following dilution with solution of 3,5-dimethoxy-4-hydroxycinnamic acid (sinapinic acid), 3,4,5-trihydoxyacetophenone (THAP) or alpha-cyano-4-hydoxycinnamic acid (HCCA) as matrices.

**Example 2**

Using the protocol described in Example 1, the following PMO was synthesized, H53A(+36+60), SEQ ID NO: 1 (5'- GTTGCCTCCGGTTCTGAAGGTGTTC -3') and used in the Examples.

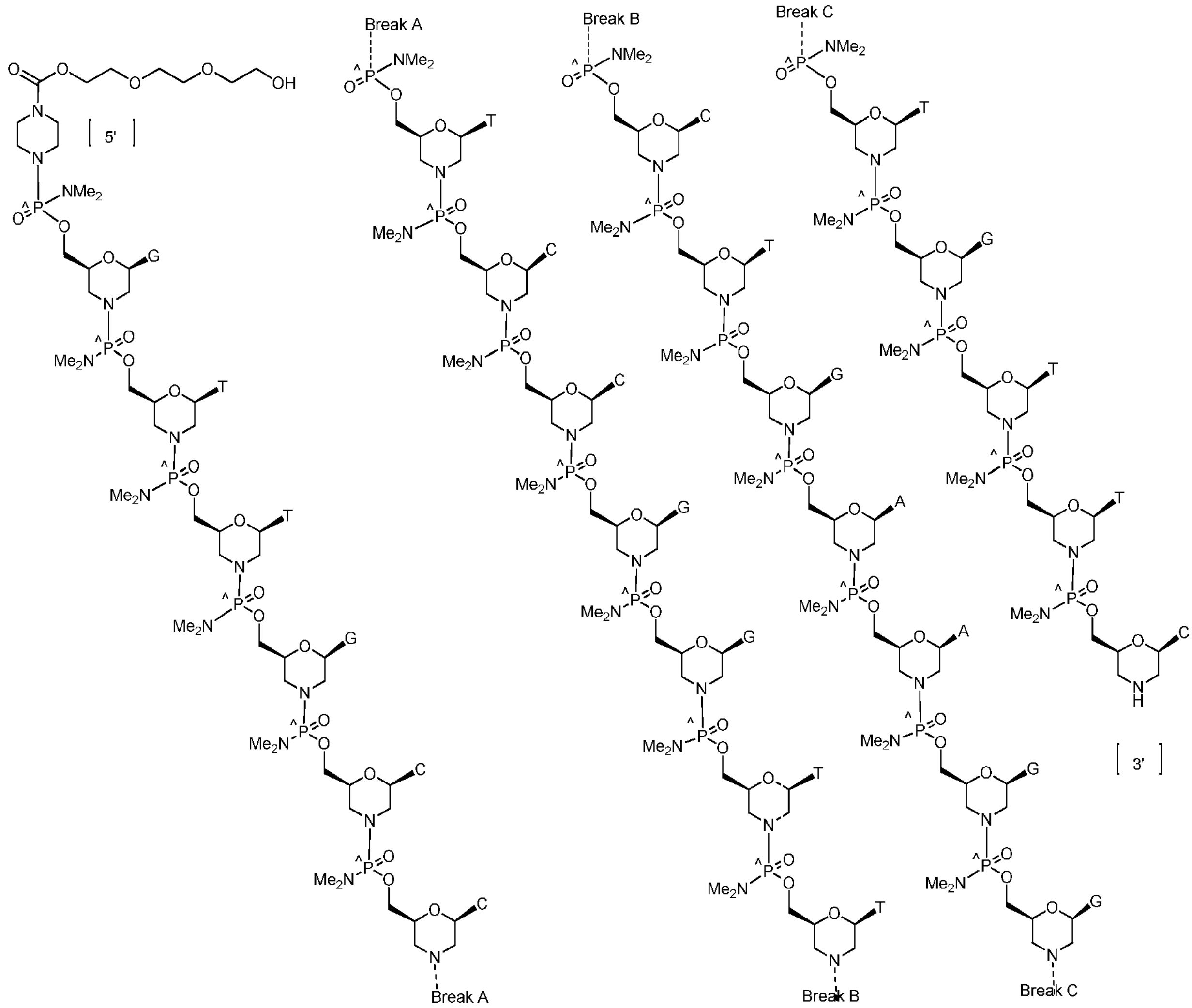

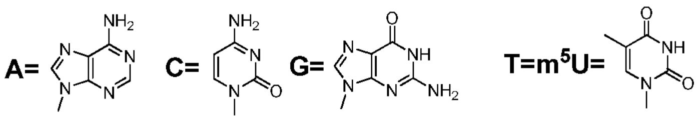

### Example 3

Using the protocol described in Example 1, the following PMO was synthesized, H53A(+30+57), SEQ ID NO: 2 (5'- GCC TCC GGT TCT GAA GGT GTT CTT GTA C-3') and used in the Examples.

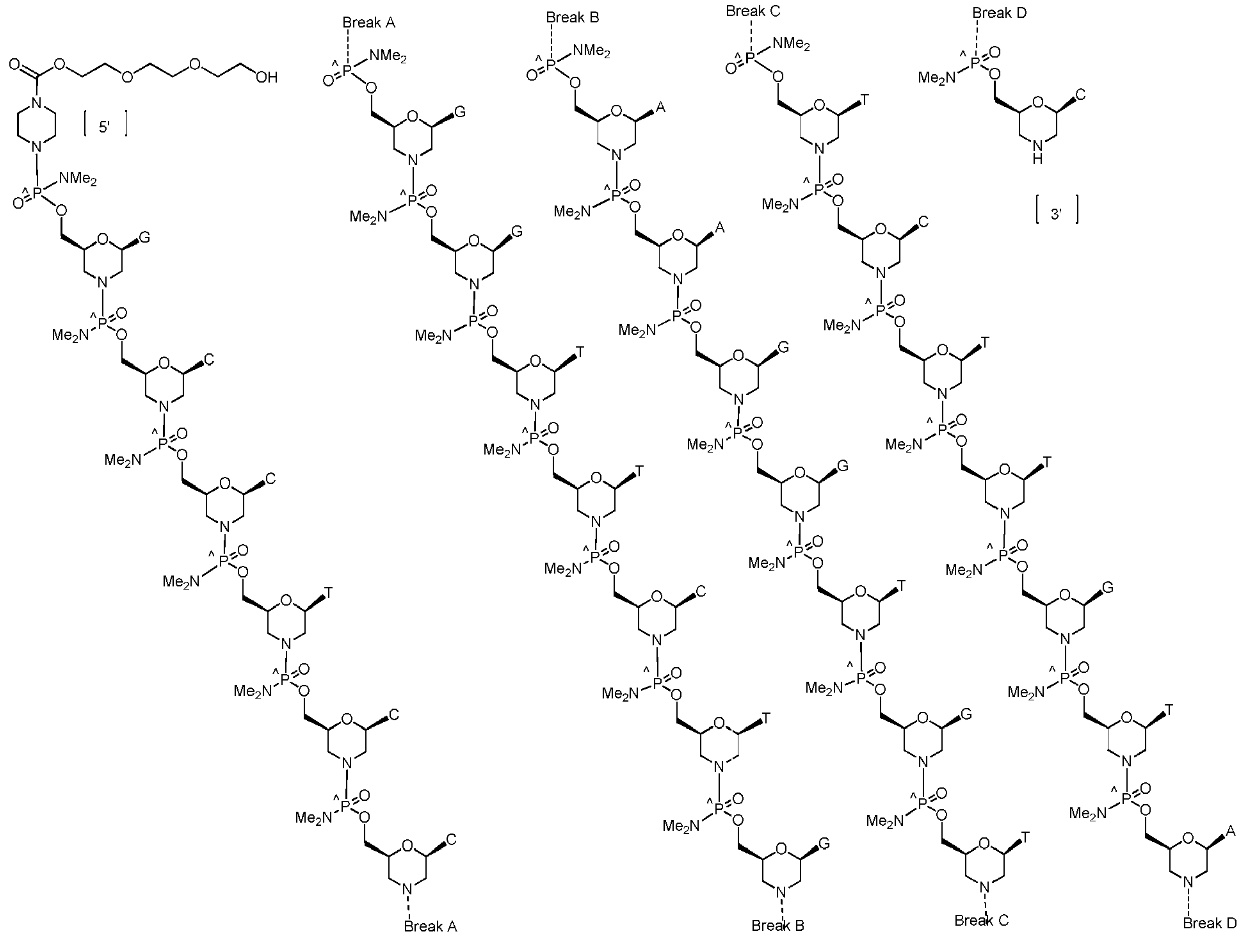

A= 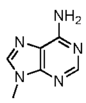 C= 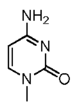 G= 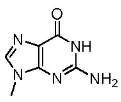 T=$m^5U$= 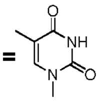

## Example 4

Using the protocol described in Example 1, the following PMO was synthesized, H53A(+30+56), SEQ ID NO: 3 (5'- CCT CCG GTT CTG AAG GTG TTC TTG TAC-3') and used in the Examples.

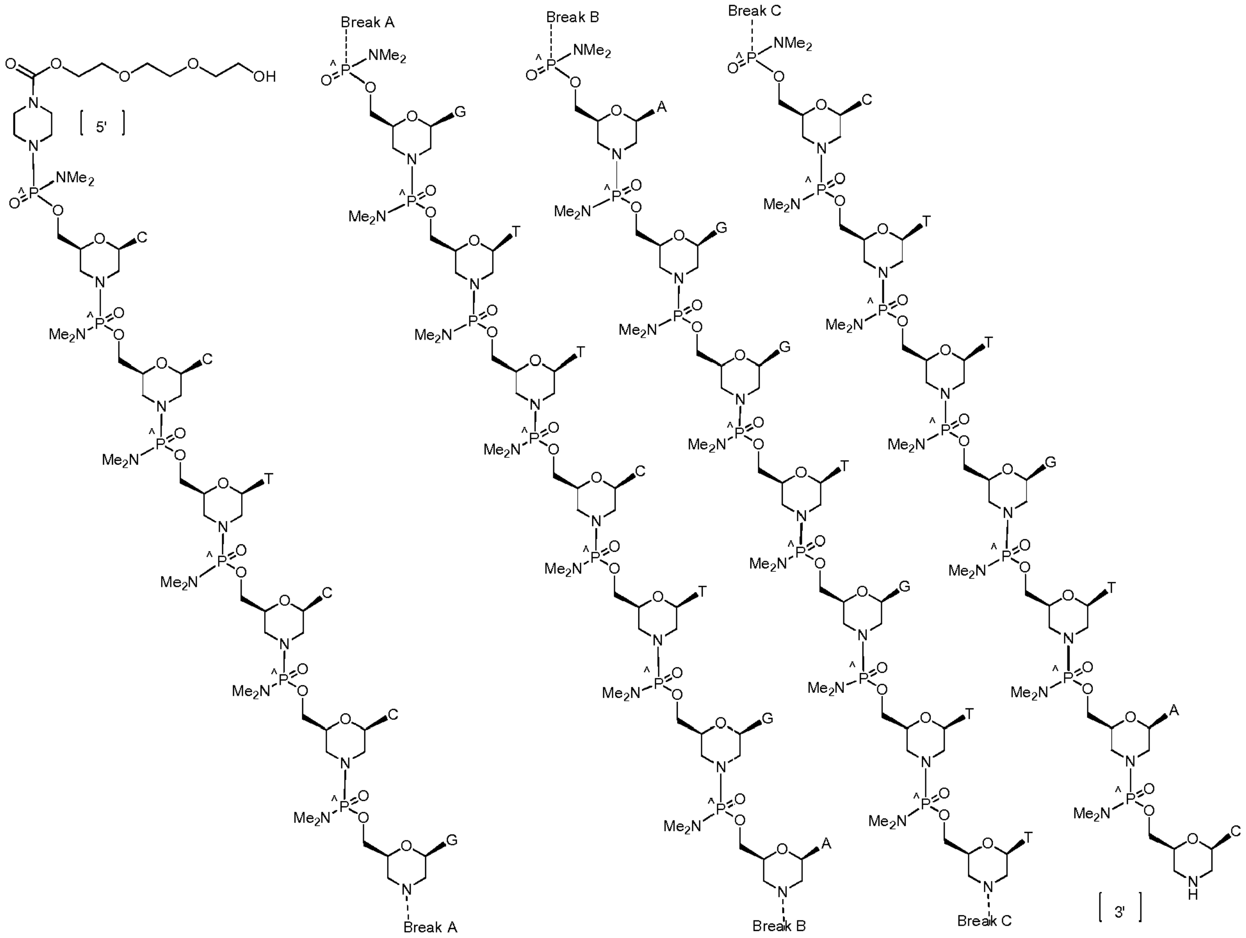

A= 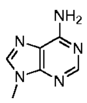 C= 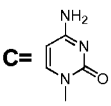 G= 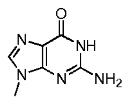 T=m$^5$U= 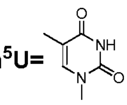

### Example 5

Using the protocol described in Example 1, the following PMO was synthesized, H53A(+30+55), SEQ ID NO: 4 (5'- CTC CGG TTC TGA AGG TGT TCT TGT AC-3') and used in the Examples.

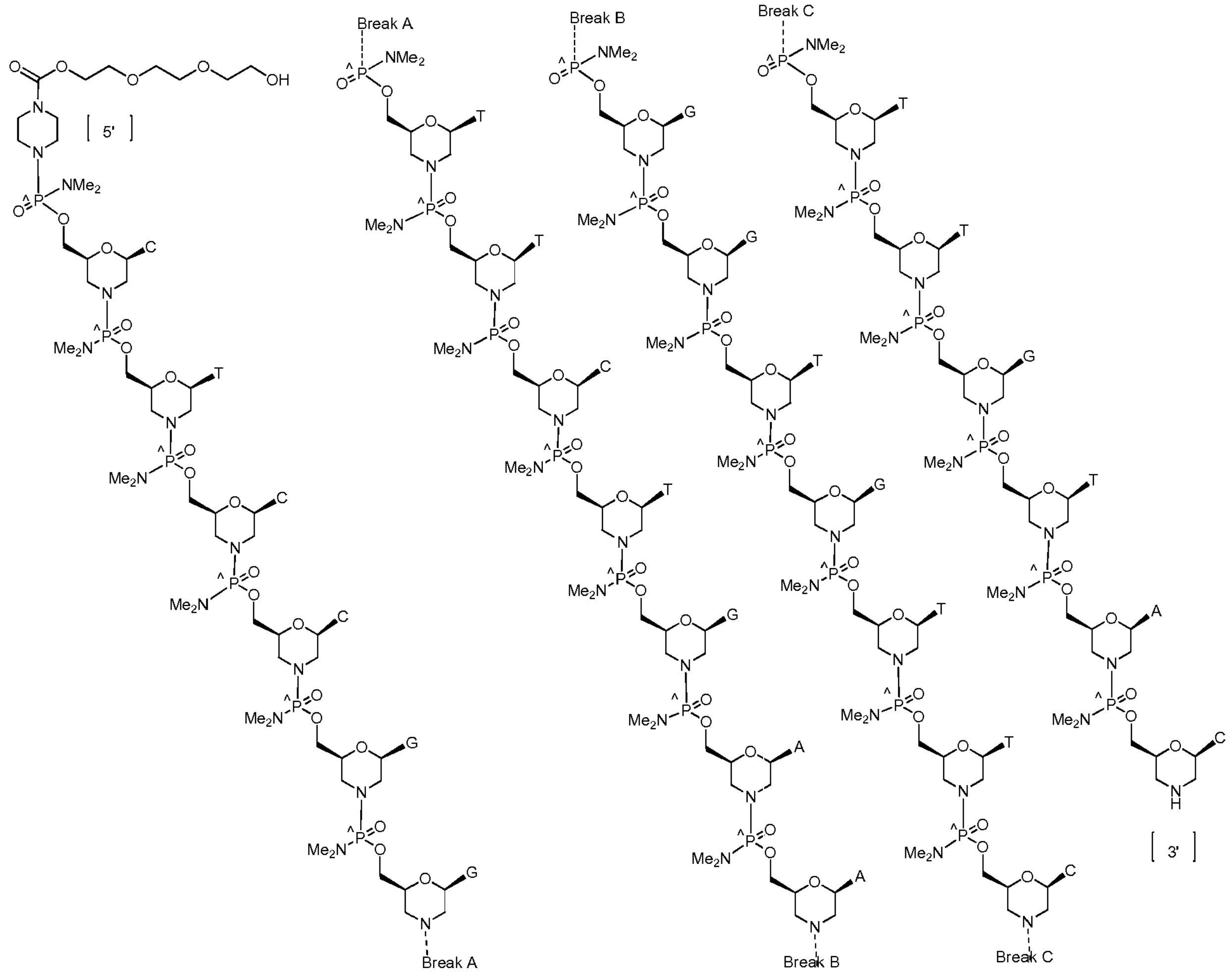

A= 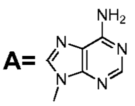 C= 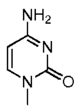 G= 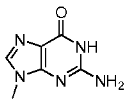 T=m⁵U= 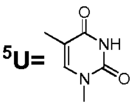

## Example 6

Using the protocol described in Example 1, the following PMO was synthesized, H53A(+33+57), SEQ ID NO: 5 (5'- GCC TCC GGT TCT GAA GGT GTT CTT G-3') and used in the Examples.

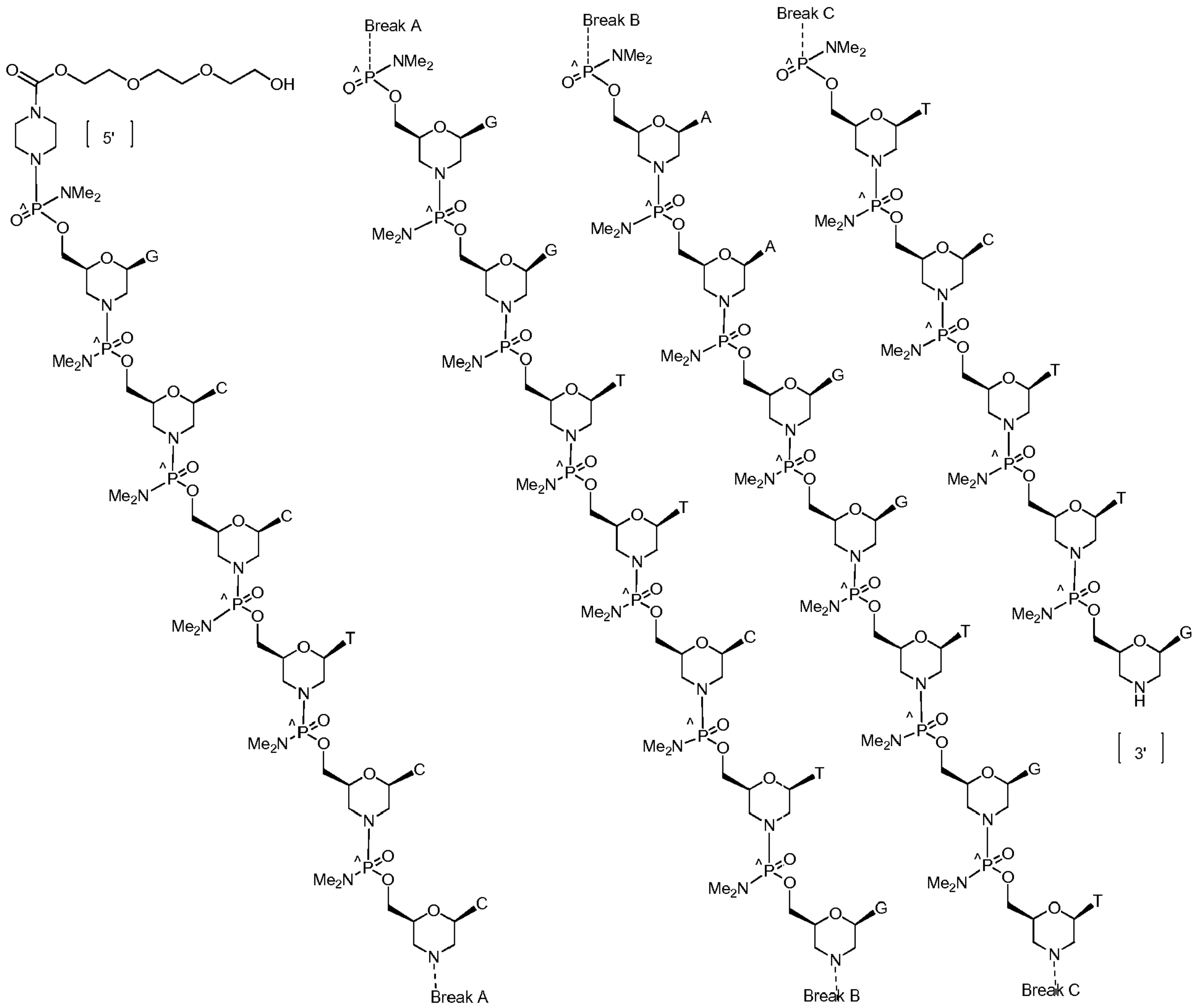

A= 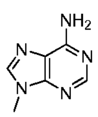 C= 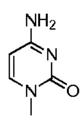 G= 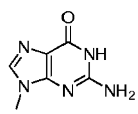 T=$m^5U$= 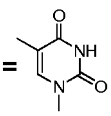

## Example 7

### Exon 53 skipping

A series of antisense oligomers that target human dystrophin exon 53 were designed and synthesized as follows:

| Description | Sequence | SEQ ID NO |
|---|---|---|
| H53A(+36+60) | GTTGCCTCCGGTTCTGAAGGTGTTC | 1 |
| H53A(+30+57) | GCCTCCGGTTCTGAAGGTGTTCTTGTAC | 2 |
| H53A(+30+56) | CCTCCGGTTCTGAAGGTGTTCTTGTAC | 3 |
| H53A(+30+55) | CTCCGGTTCTGAAGGTGTTCTTGTAC | 4 |
| H53A(+33+57) | GCCTCCGGTTCTGAAGGTGTTCTTG | 5 |
| H53A(+33+60) | GTTGCCTCCGGTTCTGAAGGTGTTCTTG | 6 |
| H53A(+31+55) | CTCCGGTTCTGAAGGTGTTCTTGTA | 7 |

(continued)

| Description | Sequence | SEQ ID NO |
|---|---|---|
| H53A(+22+46) | TGAAGGTGTTCTTGTACTTCATCCC | 8 |
| H53A(+23+47) | CTGAAGGTGTTCTTGTACTTCATCC | 9 |

The antisense oligomers above were evaluated for exon skipping efficacy by treating primary human myoblasts at the various indicated concentrations. In these experiments, antisense oligomers corresponding to H53A(+33+60), and H53A(+31+55) were used as comparative oligomers. As shown in **FIGs. 3 and 4,** oligomer H53A(+36+60) (SEQ ID NO: 1) was highly effective at inducing exon 53 skipping in primary human myoblast cells. H53A(+31+55) (SEQ ID NO: 7) and H53A(+22+46) (SEQ ID NO: 8) also induced exon 53 skipping, but to a lesser degree than H53A(+33+60) (SEQ ID NO: 6) and H53A(+36+60) (SEQ ID NO: 1). As shown in **FIG. 3,** H53A(+33+60) and H53A(+36+60) (SEQ ID NO: 6 and SEQ ID NO: 1, respectively) were highly effective in inducing exon 53 skipping in cultured myoblasts compared to other highly active antisense oligonucleotides.

Additional antisense oligomers above were evaluated for exon skipping efficacy by treating RD cells at the various indicated concentrations. In these experiments, antisense oligomer corresponding to H53A(+33+60) (SEQ ID NO: 6) was used as a comparative oligomer. As shown in **FIG. 4,** the oligomers H53A(+30+57), H53A(+30+56), H53A(+30+55) and H53A(+33+57) (SEQ ID NO: 2-5, respectively) were comparable in activity to oligomer H53A(+33+60) (SEQ ID NO: 6).

In another experiment, an antisense oligomer of the invention was tested for exon 53 skipping in primary human myoblasts in comparison to published antisense oligonucleotides. In addition to H53A(+23+47) (SEQ ID NO: 9) described above, H53A(+39+69), H53A(+39+62), and H53A(+45+69) (SEQ ID NOs: 10, 11, and 12, respectively) from US 8,232,384; h53AON1 (+45+62) (SEQ ID NO: 13) from WO2004/083446; and PMO3(+32+56) and PMO8(+36+56) (SEQ ID NOs: 14 and 15, respectively) from WO2012/029986 were compared to an exemplary oligomer of the invention H53A(+36+60) (SEQ ID NO:1). As shown in FIG. 5A and 5B, the oligomer that demonstrated the lowest EC50 was determined to be H53A(+36+60) SEQ ID NO: 1. This was confirmed by subjecting the results to a three parameter regression analysis within the GraphPad Prism program to generate EC50 values as shown in the following table.

| | EC50 |
|---|---|
| SEQIDNO:10 H53A(+39+69) | 6.482 |
| SEQIDNO:9 H53A(+23+47) | 9.135 |
| SEQIDNO:1 H53A(+36+60) | 3.910 |
| SEQIDNO:13 H53A(+45+62) | 17.13 |
| SEQIDNO:11 H53A(+39+62) | 6.061 |
| SEQIDNO:12 H53A(+45+69) | 13.11 |
| SEQIDNO:14 PMO3(+32+56) | 11.80 |
| SEQIDNO:15 PMO8(+36+56) | 15.81 |

All publications and patent applications cited in this specification are herein incorporated by reference as if each individual publication or patent application were specifically and individually indicated to be incorporated by reference.

**REFERENCES**

Aartsma-Rus, A., A. A. Janson, et al. (2004). "Antisense-induced multiexon skipping for Duchenne muscular dystrophy makes more sense." Am J Hum Genet 74(1): 83-92.

Cirak, S., V. Arechavala-Gomeza, et al. (2011). "Exon skipping and dystrophin restoration in patients with Duchenne muscular dystrophy after systemic phosphorodiamidate morpholino oligomer treatment: an open-label, phase 2, dose-escalation study." Lancet 378(9791): 595-605.

Dunckley, M. G., I. C. Eperon, et al. (1997). "Modulation of splicing in the DMD gene by antisense oligoribonucleotides." Nucleosides & Nucleotides 16(7-9): 1665-1668.
Dunckley, M. G., M. Manoharan, et al. (1998). "Modification of splicing in the dystrophin gene in cultured Mdx muscle cells by antisense oligoribonucleotides." Hum Mol Genet 7(7): 1083-90.
Errington, S. J., C. J. Mann, et al. (2003). "Target selection for antisense oligonucleotide induced exon skipping in the dystrophin gene." J Gene Med 5(6): 518-27.
Goemans, N. M., M. Tulinius, et al. (2011). "Systemic Administration of PRO051 in Duchenne's Muscular Dystrophy." N Engl J Med.
Jearawiriyapaisarn, N., H. M. Moulton, et al. (2008). "Sustained Dystrophin Expression Induced by Peptide-conjugated Morpholino Oligomers in the Muscles of mdx Mice." Mol Ther.
Kinali, M., V. Arechavala-Gomeza, et al. (2009). "Local restoration of dystrophin expression with the morpholino oligomer AVI-4658 in Duchenne muscular dystrophy: a single-blind, placebo-controlled, dose-escalation, proof-of-concept study." Lancet Neurol 8(10): 918-28.
Lu, Q. L., C. J. Mann, et al. (2003). "Functional amounts of dystrophin produced by skipping the mutated exon in the mdx dystrophic mouse." Nat Med 9(8): 1009-14.
Mann, C. J., K. Honeyman, et al. (2002). "Improved antisense oligonucleotide induced exon skipping in the mdx mouse model of muscular dystrophy." J Gene Med 4(6): 644-54.
Marshall, N. B., S. K. Oda, et al. (2007). "Arginine-rich cell-penetrating peptides facilitate delivery of antisense oligomers into murine leukocytes and alter pre-mRNA splicing." Journal of Immunological Methods 325(1-2): 114-126.
Matsuo, M., T. Masumura, et al. (1991). "Exon skipping during splicing of dystrophin mRNA precursor due to an intraexon deletion in the dystrophin gene of Duchenne muscular dystrophy kobe." J Clin Invest 87(6): 2127-31.
Monaco, A. P., C. J. Bertelson, et al. (1988). "An explanation for the phenotypic differences between patients bearing partial deletions of the DMD locus." Genomics 2(1): 90-5.
Pramono, Z. A., Y. Takeshima, et al. (1996). "Induction of exon skipping of the dystrophin transcript in lymphoblastoid cells by transfecting an antisense oligodeoxynucleotide complementary to an exon recognition sequence." Biochem Biophys Res Commun 226(2): 445-9.
Sazani, P., R. Kole, et al. (2007). Splice switching oligomers for the TNF superfamily receptors and their use in treatment of disease. PCT WO2007058894, University of North Carolina
Sierakowska, H., M. J. Sambade, et al. (1996). "Repair of thalassemic human beta-globin mRNA in mammalian cells by antisense oligonucleotides." Proc Natl Acad Sci U S A 93(23): 12840-4.
Summerton, J. and D. Weller (1997). "Morpholino antisense oligomers: design, preparation, and properties." Antisense Nucleic Acid Drug Dev 7(3): 187-95.
Takeshima, Y., H. Nishio, et al. (1995). "Modulation of in vitro splicing of the upstream intron by modifying an intra-exon sequence which is deleted from the dystrophin gene in dystrophin Kobe." J Clin Invest 95(2): 515-20.
van Deutekom, J. C., M. Bremmer-Bout, et al. (2001). "Antisense-induced exon skipping restores dystrophin expression in DMD patient derived muscle cells." Hum Mol Genet 10(15): 1547-54.
van Deutekom, J. C., A. A. Janson, et al. (2007). "Local dystrophin restoration with antisense oligonucleotide PRO051." N Engl J Med 357(26): 2677-86.
Wilton, S. D., A. M. Fall, et al. (2007). "Antisense oligonucleotide-induced exon skipping across the human dystrophin gene transcript." Mol Ther 15(7): 1288-96.
Wilton, S. D., F. Lloyd, et al. (1999). "Specific removal of the nonsense mutation from the mdx dystrophin mRNA using antisense oligonucleotides." Neuromuscul Disord 9(5): 330-8.
Wu, B., H. M. Moulton, et al. (2008). "Effective rescue of dystrophin improves cardiac function in dystrophin-deficient mice by a modified morpholino oligomer." Proc Natl Acad Sci U S A 105(39): 14814-9.
Yin, H., H. M. Moulton, et al. (2008). "Cell-penetrating peptide-conjugated antisense oligonucleotides restore systemic muscle and cardiac dystrophin expression and function." Hum Mol Genet 17(24): 3909-18.

## SEQUENCE LISTING

| Description | Sequence | SEQ ID NO |
|---|---|---|
| H53A(+36+60) | GTTGCCTCCGGTTCTGAAGGTGTTC | 1 |
| H53A(+30+57) | GCCTCCGGTTCTGAAGGTGTTCTTGTAC | 2 |
| H53A(+30+56) | CCTCCGGTTCTGAAGGTGTTCTTGTAC | 3 |
| H53A(+30+55) | CTCCGGTTCTGAAGGTGTTCTTGTAC | 4 |
| H53A(+33+57) | GCCTCCGGTTCTGAAGGTGTTCTTG | 5 |

(continued)

| Description | Sequence | SEQ ID NO |
|---|---|---|
| H53A(+33+60) | GTTGCCTCCGGTTCTGAAGGTGTTCTTG | 6 |
| h53A(+31+55) | CTCCGGTTCTGAAGGTGTTCTTGTA | 7 |
| H53A(+22+46) | TGAAGGTGTTCTTGTACTTCATCCC | 8 |
| H53A(+23+47) | CTGAAGGTGTTCTTGTACTTCATCC | 9 |
| H53 (+39+69) | CATTCAACTGTTGCCTCCGGTTCTGAAGGTG | 10 |
| H53(+39+62) | CTGTTGCCTCCGGTTCTGAAGGTG | 11 |
| H53 (+45+69) | CATTCAACTGTTGCCTCCGGTTCTG | 12 |
| H53 (+45+62) | CTGTTGCCTCCGGTTCTG | 13 |
| PMO3(+32+56) | CCTCCGGTTCTGAAGGTGTTCTTGT | 14 |
| PMO8(+36+56) | CCTCCGGTTCTGAAGGTGTTC | 15 |
| rTAT | RRRQRRKKR | 16 |
| Tat | RKKRRQRRR | 17 |
| $R_9F_2$ | RRRRRRRRRFF | 18 |
| $R_5F_2R_4$ | RRRRRFFRRRR | 19 |
| $R_4$ | RRRR | 20 |
| $R_5$ | RRRRR | 21 |
| $R_6$ | RRRRRR | 22 |
| $R_7$ | RRRRRRR | 23 |
| $R_8$ | RRRRRRRR | 24 |
| $R_9$ | RRRRRRRRR | 25 |
| (RX)s | RXRXRXRXRXRXRXRX | 26 |
| $(RAhxR)_4$; (P007) | RAhxRRAhxRRAhxRRAhxR | 27 |
| $(RAhxR)_5$; (CP04057) | RAhxRRAhxRRAhxRRAhxRRAhxR | 28 |
| $(RAhxRRBR)_2$; (CP06062) | RAhxRRBRRAhxRRBR | 29 |
| $(RAR)_4F_2$ | RARRARRARRARFF | 30 |
| $(RGR)_4F_2$ | RGRRGRRGRRGRFF | 31 |
| Primer | CTTGGACAGAACTTACCGACTGG | 32 |
| Primer | GTTTCTTCCAAAGCAGCCTCTCG | 33 |
| Primer | GCAGGATTTGGAACAGAGGCG | 34 |
| Primer | CATCTACATTTGTCTGCCACTGG | 35 |

The present disclosure will now be further defined in the following paragraphs:

1. An antisense oligomer of 20-50 nucleotides in length capable of binding a selected target to induce exon skipping in the human dystrophin gene, wherein said antisense oligomer comprises a sequence of bases that specifically hybridizes to an exon 53 target region selected from the group consisting of H53A(+36+60), H53A(+30+57), H53A (+30+56), H53A(+30+55) and H53A(+33+57), wherein the bases of said oligomer are linked to morpholino ring structures, and wherein said morpholino ring structures are joined by phosphorous-containing intersubunit linkages joining a morpholino nitrogen of one ring structure to a 5' exocyclic carbon of an adjacent ring structure.

2. The antisense oligomer of paragraph 1, wherein the sequence is SEQ ID NO: 1-5.

3. The antisense oligomer of paragraph 1 or 2, which is about 20 to 30 nucleotides in length.

4. The antisense oligomer of paragraph 1 or 2, which is about 25 to 28 nucleotides in length.

5. The antisense oligomer of paragraph 2, wherein the sequence consists of a sequence selected from SEQ ID NO: 1-5.

6. The antisense oligomer of any one of paragraphs 1-5, wherein the oligomer does not activate RNase H.

7. The antisense oligomer of any one of paragraphs 1-5, wherein the oligomer is chemically linked to one or more moieties or conjugates that enhance the activity, cellular distribution, or cellular uptake of the antisense oligomer.

8. The antisense oligomer of paragraph 7, wherein the oligomer is chemically linked to a polyethylene glycol molecule.

9. The antisense oligomer of any one of paragraphs 1-5, wherein the antisense oligomer is conjugated to an arginine-rich peptide.

10. The antisense oligomer of paragraph 9, wherein the arginine-rich peptide comprises a sequence selected from SEQ ID NOS: 16-31.

11. The antisense oligomer of any of the preceding paragraphs, comprising morpholino ring structures joined by substantially uncharged phosphorus-containing intersubunit linkages joining a morpholino nitrogen of one ring structure to a 5' exocyclic carbon of an adjacent ring structure.

12. The antisense oligomer of paragraph 11, wherein 5%-35% of the linkages is positively charged.

13. The antisense oligomer of any of the preceding paragraphs, wherein the intersubunit linkages are uncharged and interspersed with linkages that are positively charged at physiological pH, wherein the total number of positively charged linkages is between 2 and no more than half of the total number of linkages.

14. The antisense oligomer of any one of paragraphs 1-13, comprising morpholino ring structures and phosphorodiamidate intersubunit linkages.

15. The antisense oligomer are modified with a pendant cationic group.

16. An antisense oligomer, wherein the oligomer has a structure selected from the group consisting of:

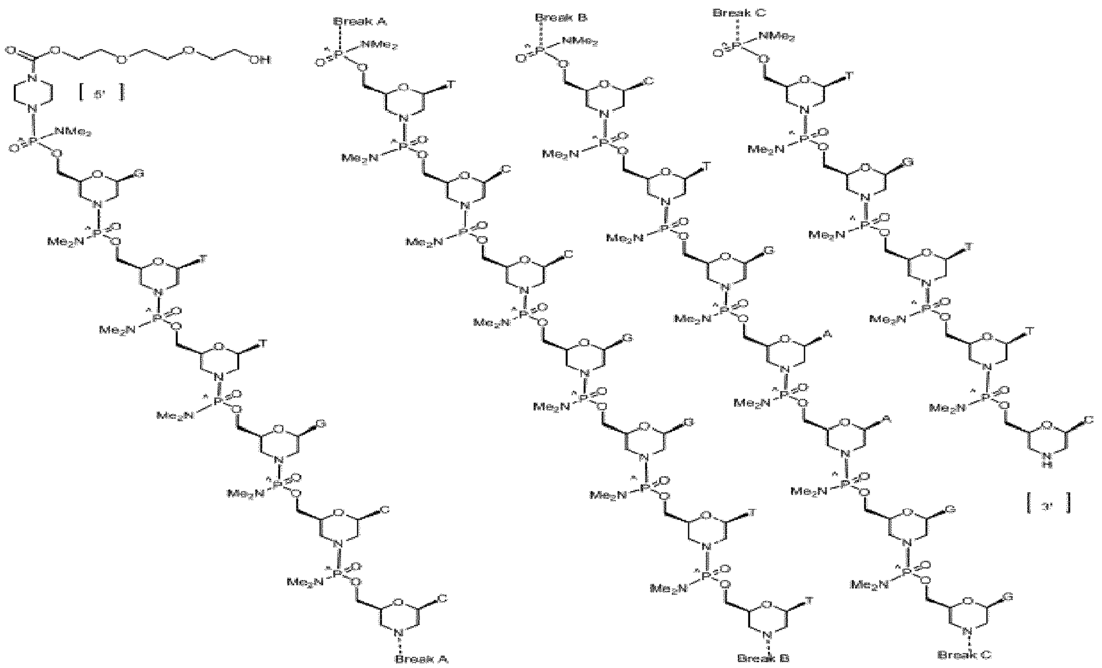

H53A(+36+60),

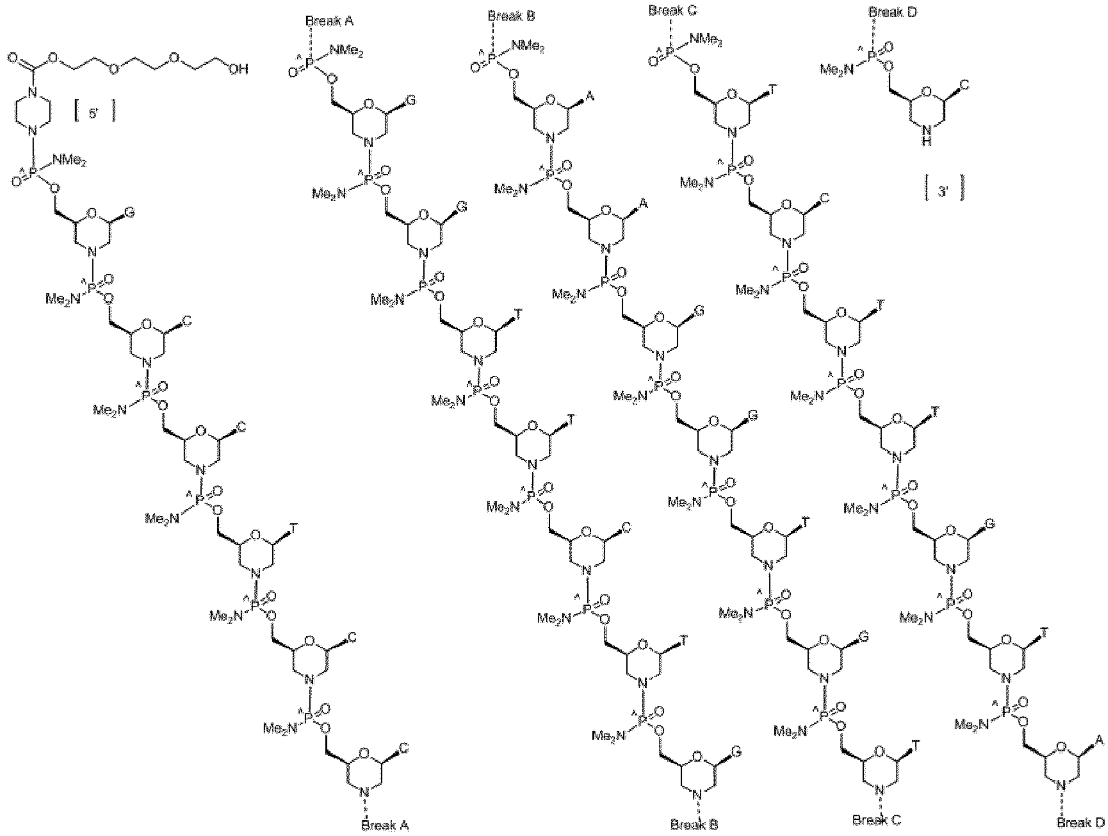

H53A(+30+57),

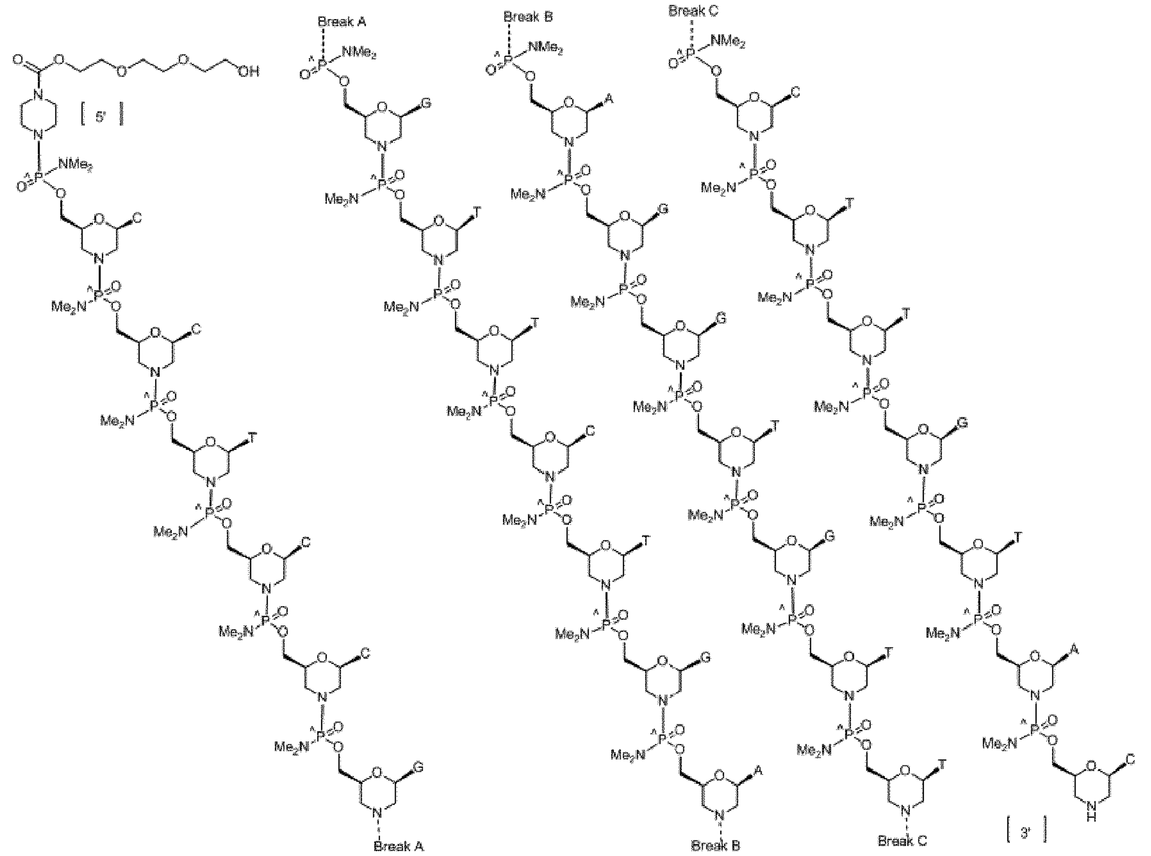

H53A(+30+56),

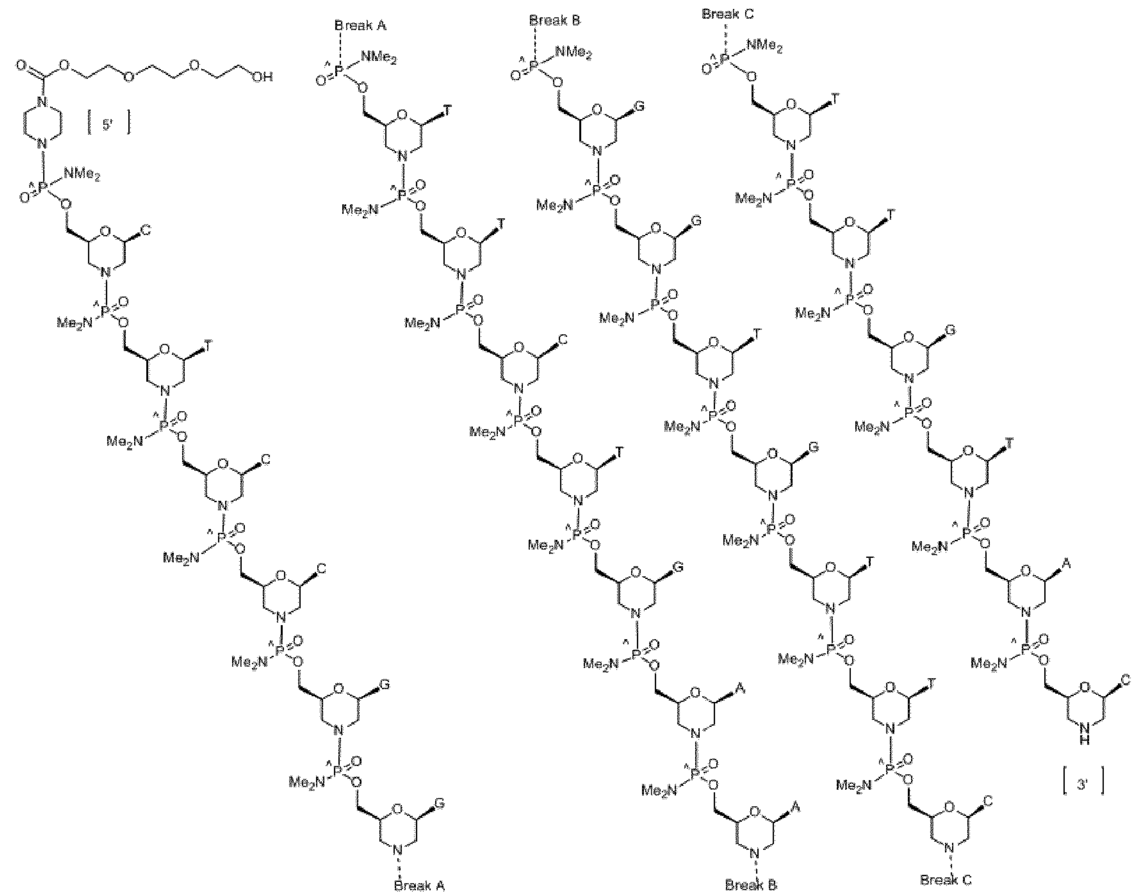

H53A(+30+55),

and

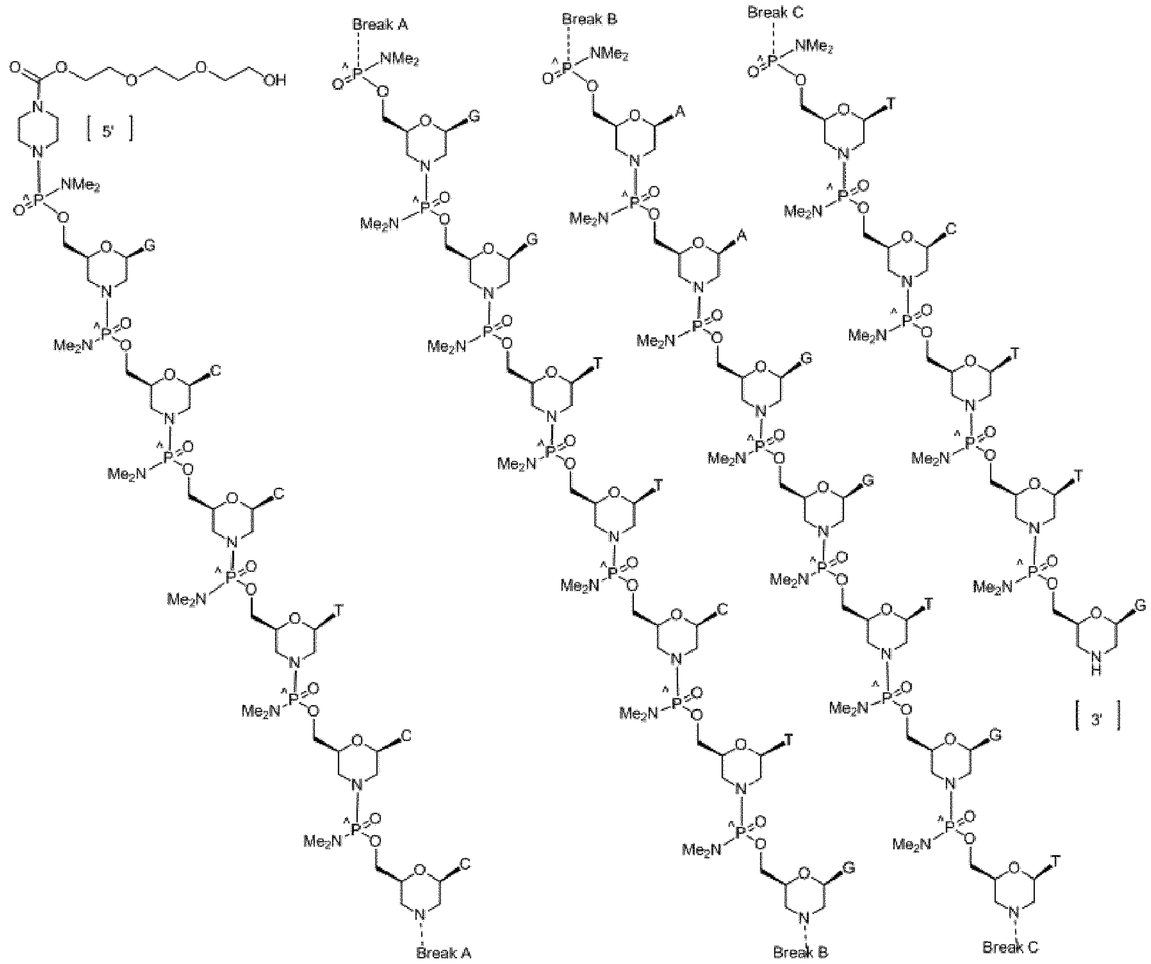

H53A(+33+57),

wherein

A= 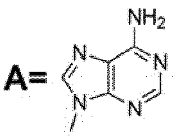, C= 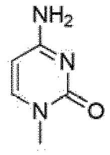, G= 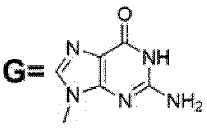, and T=m$^5$U= 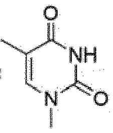,

and
wherein ^ = the stereochemistry of the phosphorous center is not defined.

17. A composition comprising the antisense oligomer of any of the preceding paragraphs and a pharmaceutically acceptable carrier.

18. The composition of paragraph 17 for use in the treatment of muscular dystrophy.
81

19. The composition of paragraph 18, wherein the muscular dystrophy is Duchenne's muscular dystrophy (DMD).

20. The composition of paragraph 18, wherein the muscular dystrophy is Becker's muscular dystrophy (BMD).

## Claims

1. An antisense oligomer of 23-27 nucleotides in length capable of binding a selected target to induce exon skipping in the human dystrophin gene, wherein said antisense oligomer comprises a sequence of bases that specifically hybridizes to an exon 53 target region, wherein the target region is H53A(+22+46), wherein the base sequence comprises at least 22 bases of TGAAGGTGTTCTTGTACTTCATCCC (SEQ ID NO:8), wherein the bases of said oligomer are linked to morpholino ring structures, and wherein said morpholino ring structures are joined by phosphorus-containing intersubunit linkages joining a morpholino nitrogen of one ring structure to a 5' exocyclic carbon of an adjacent ring structure.

2. An antisense oligomer of 23-27 nucleotides in length capable of binding a selected target to induce exon skipping in the human dystrophin gene, wherein said antisense oligomer comprises a sequence of bases that specifically hybridizes to an exon 53 target region, wherein the target region is H53A(+23+47), wherein the base sequence comprises at least 22 bases of CTGAAGGTGTTCTTGTACTTCATCC (SEQ ID NO:9), wherein the bases of said oligomer are linked to morpholino ring structures, and wherein said morpholino ring structures are joined by phosphorus-containing intersubunit linkages joining a morpholino nitrogen of one ring structure to a 5' exocyclic carbon of an adjacent ring structure.

3. A pharmaceutical composition comprising the antisense oligomer of claim 1 or claim 2 and a pharmaceutically acceptable carrier.

4. An antisense oligomer according to claim 1 or claim 2, for use in treating a patient with Duchenne muscular dystrophy (DMD) in need thereof.

# FIG. 1A

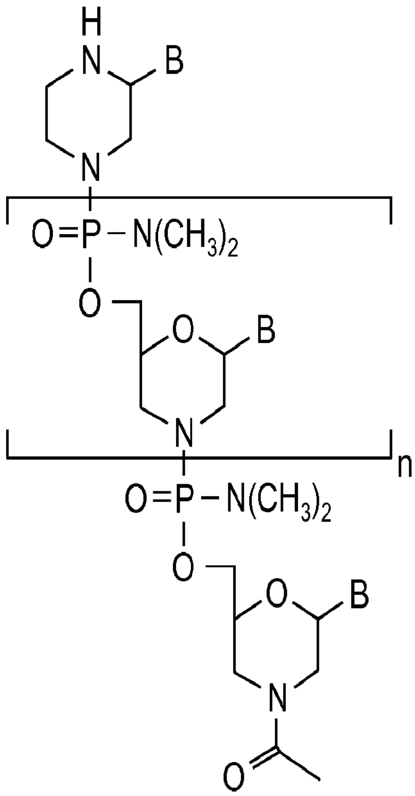

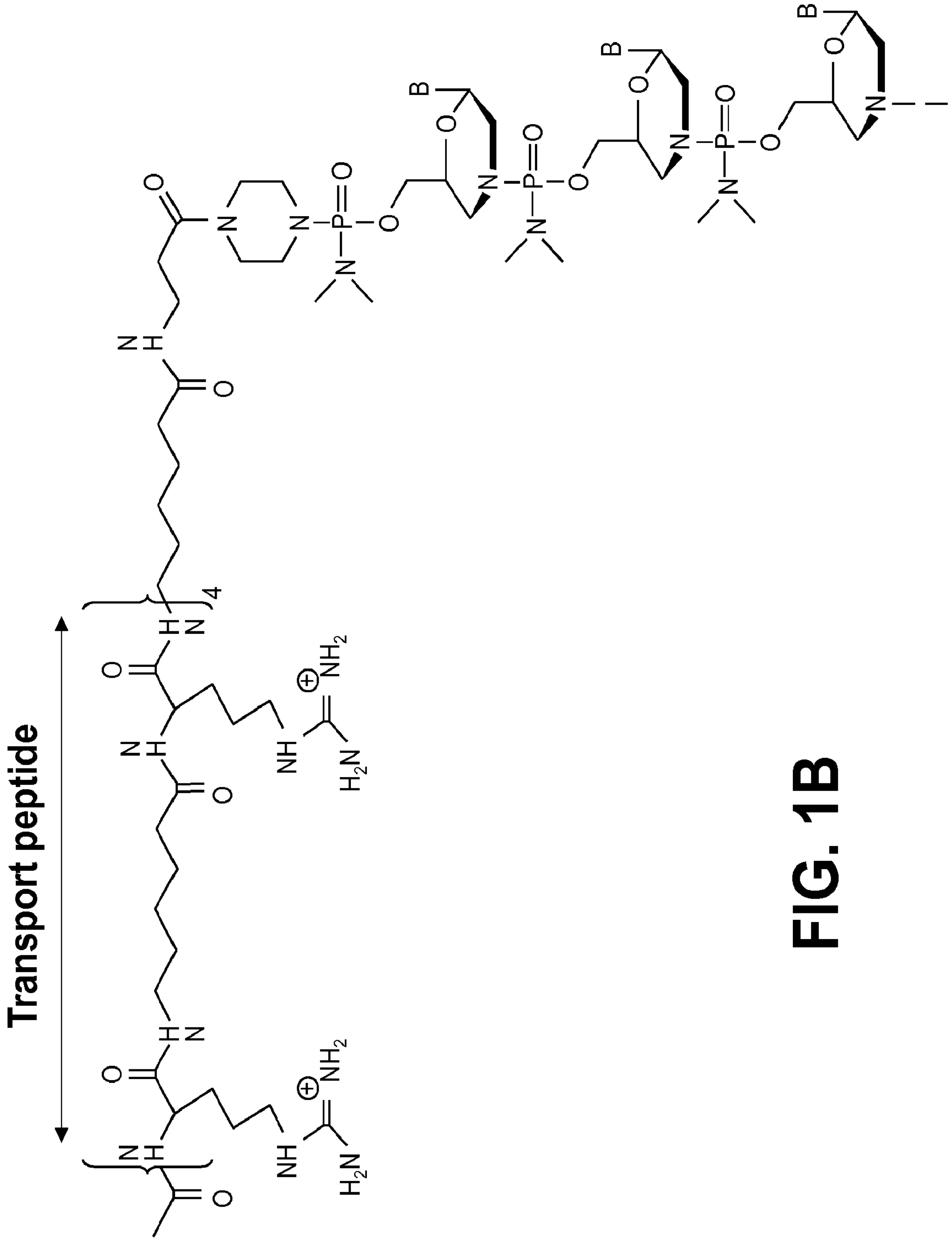


FIG. 1B

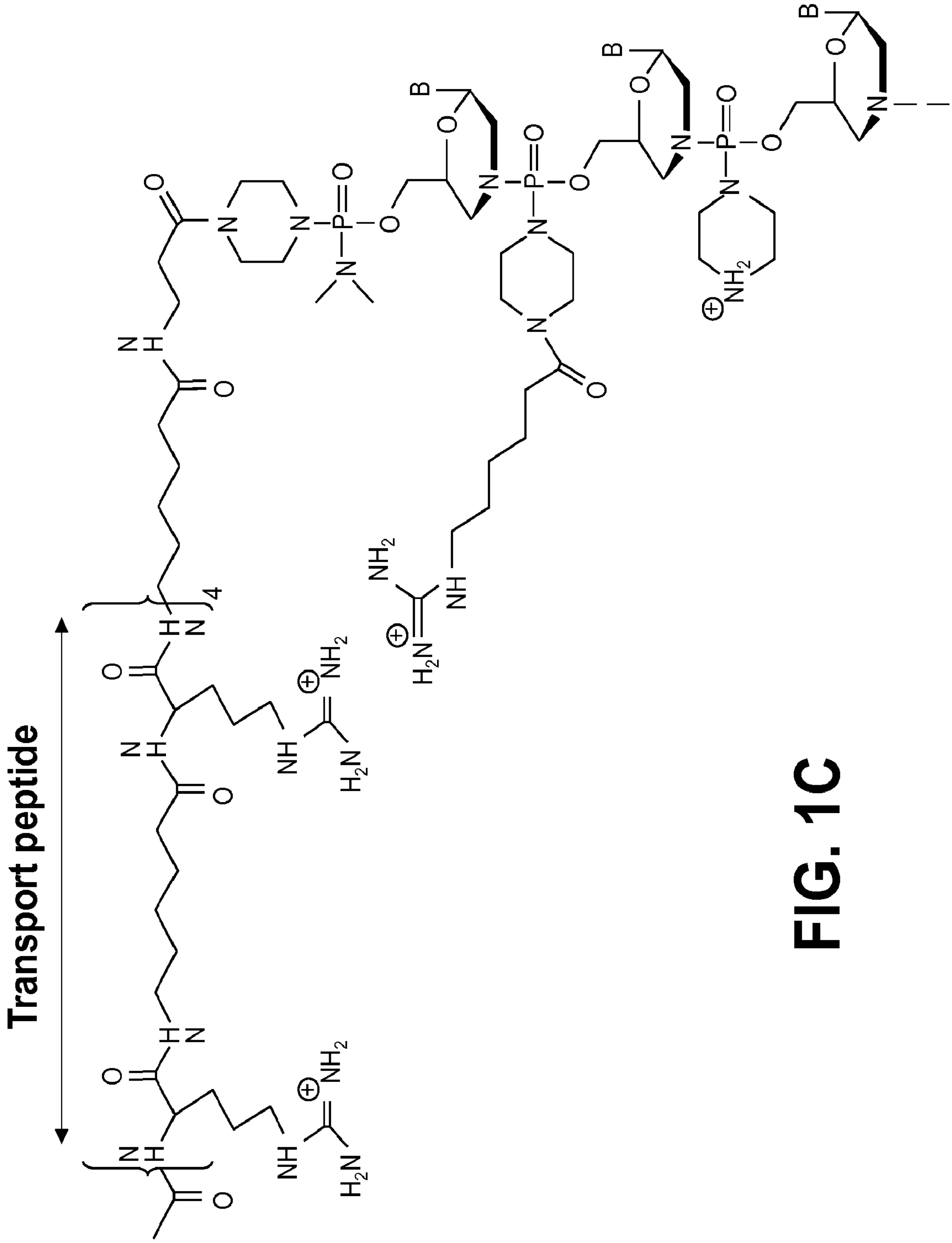


FIG. 1C

# FIG. 1D

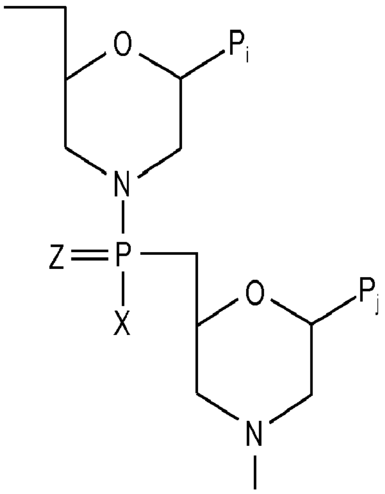

# FIG. 1E

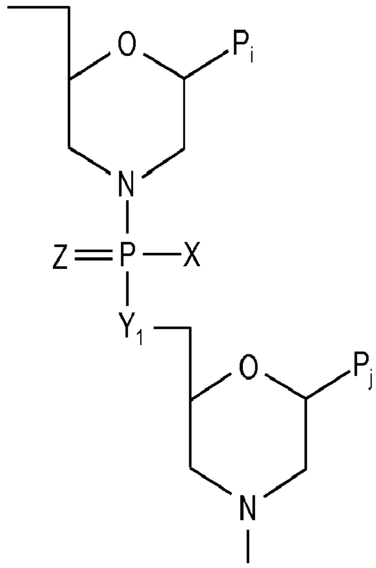

# FIG. 1F

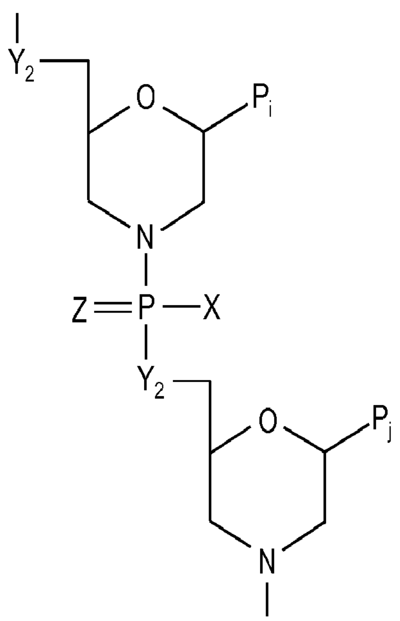

# FIG. 1G

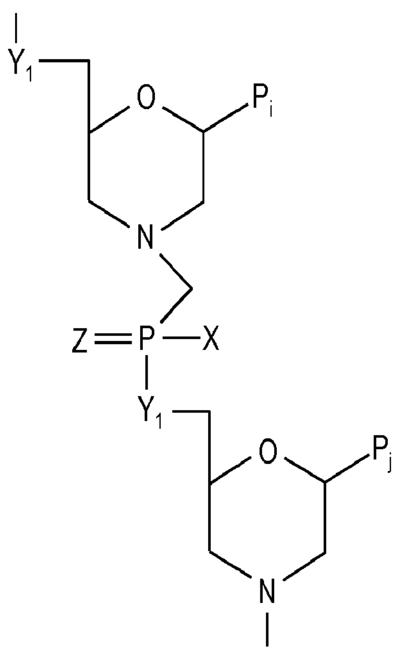

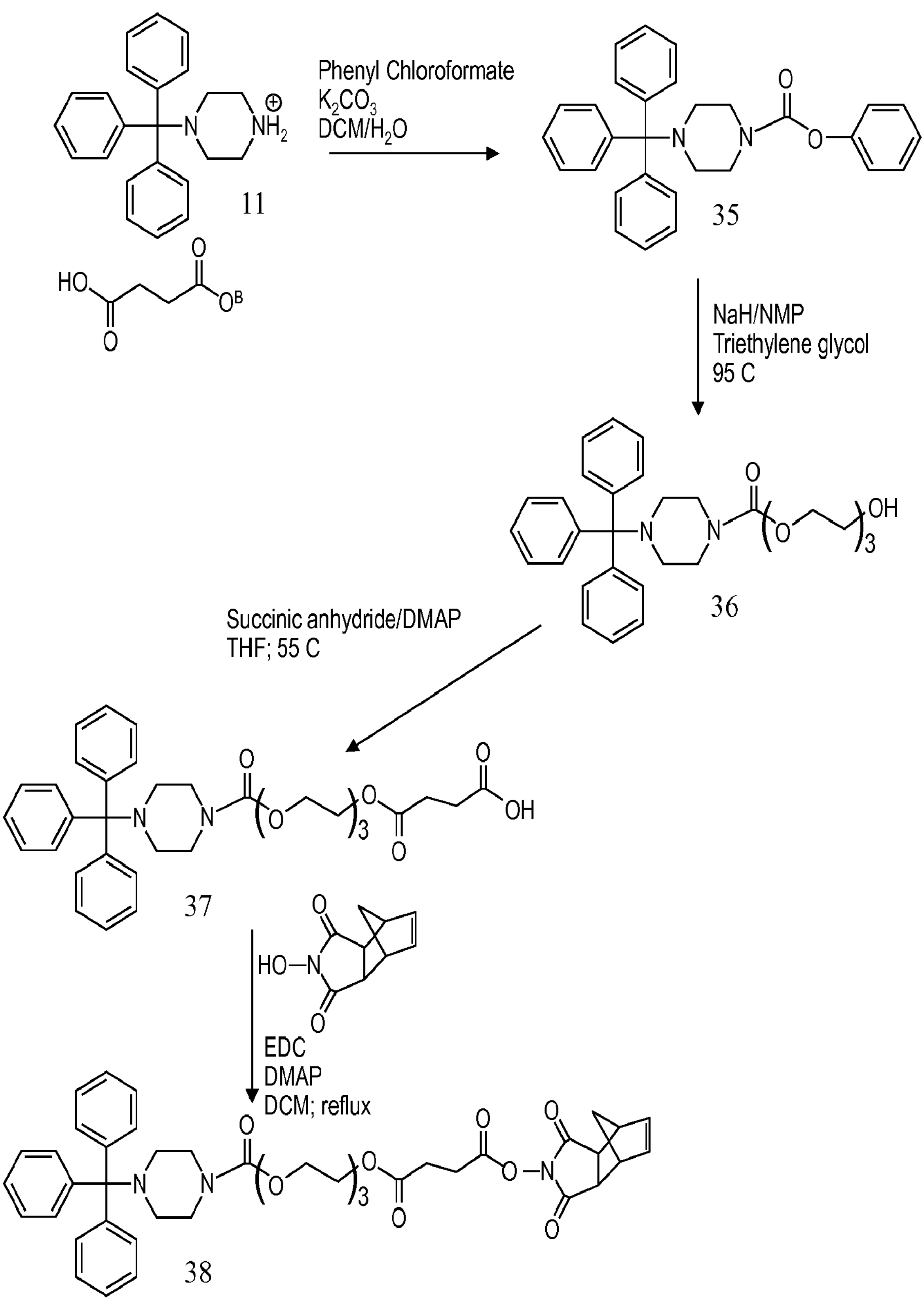
Phenyl Chloroformate
$K_2CO_3$
$DCM/H_2O$
11
35
NaH/NMP
Triethylene glycol
95 C
36
Succinic anhydride/DMAP
THF; 55 C
37
EDC
DMAP
DCM; reflux
38

**FIG. 2A**

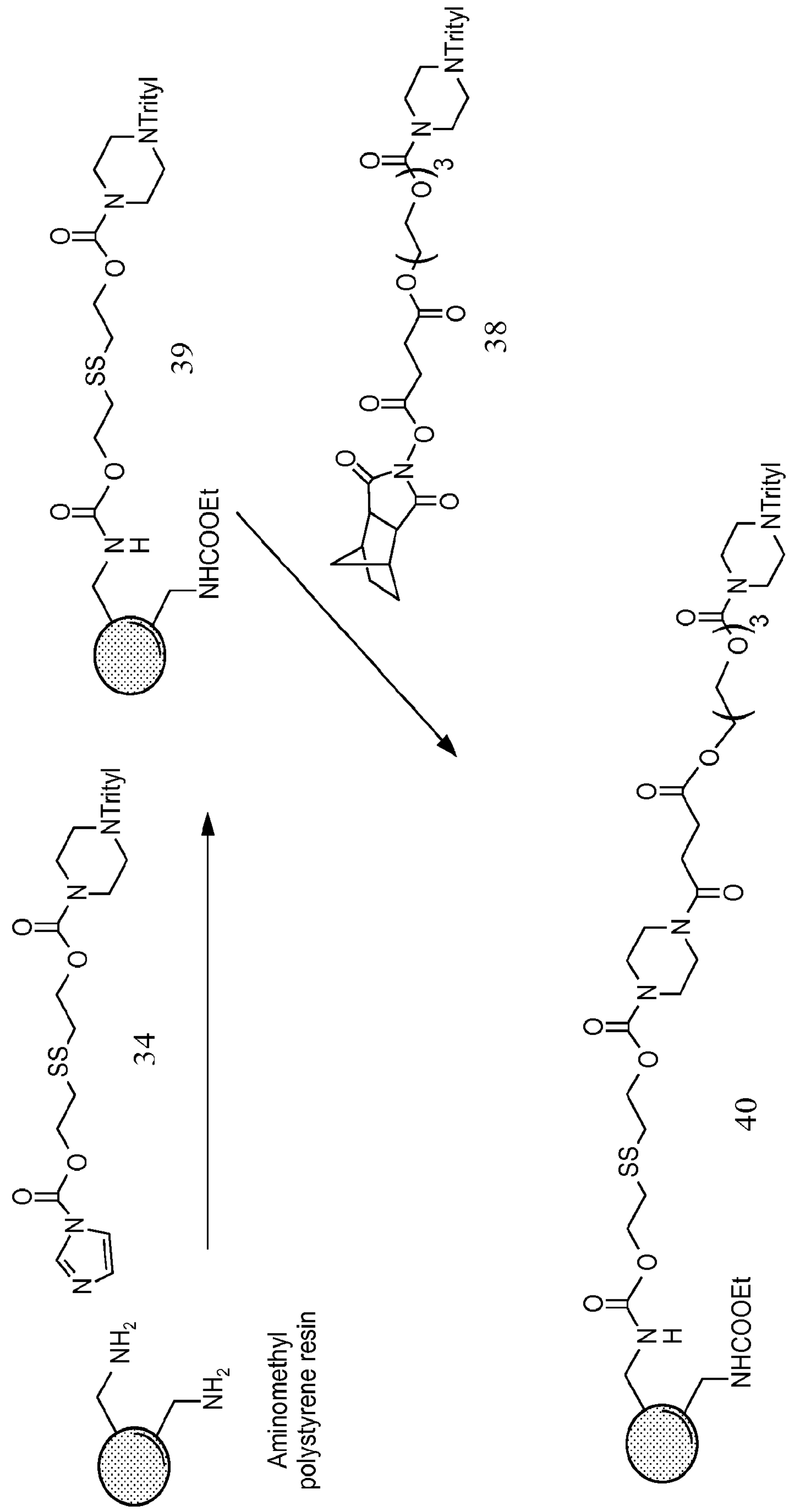


34

39

38

40

FIG. 2B

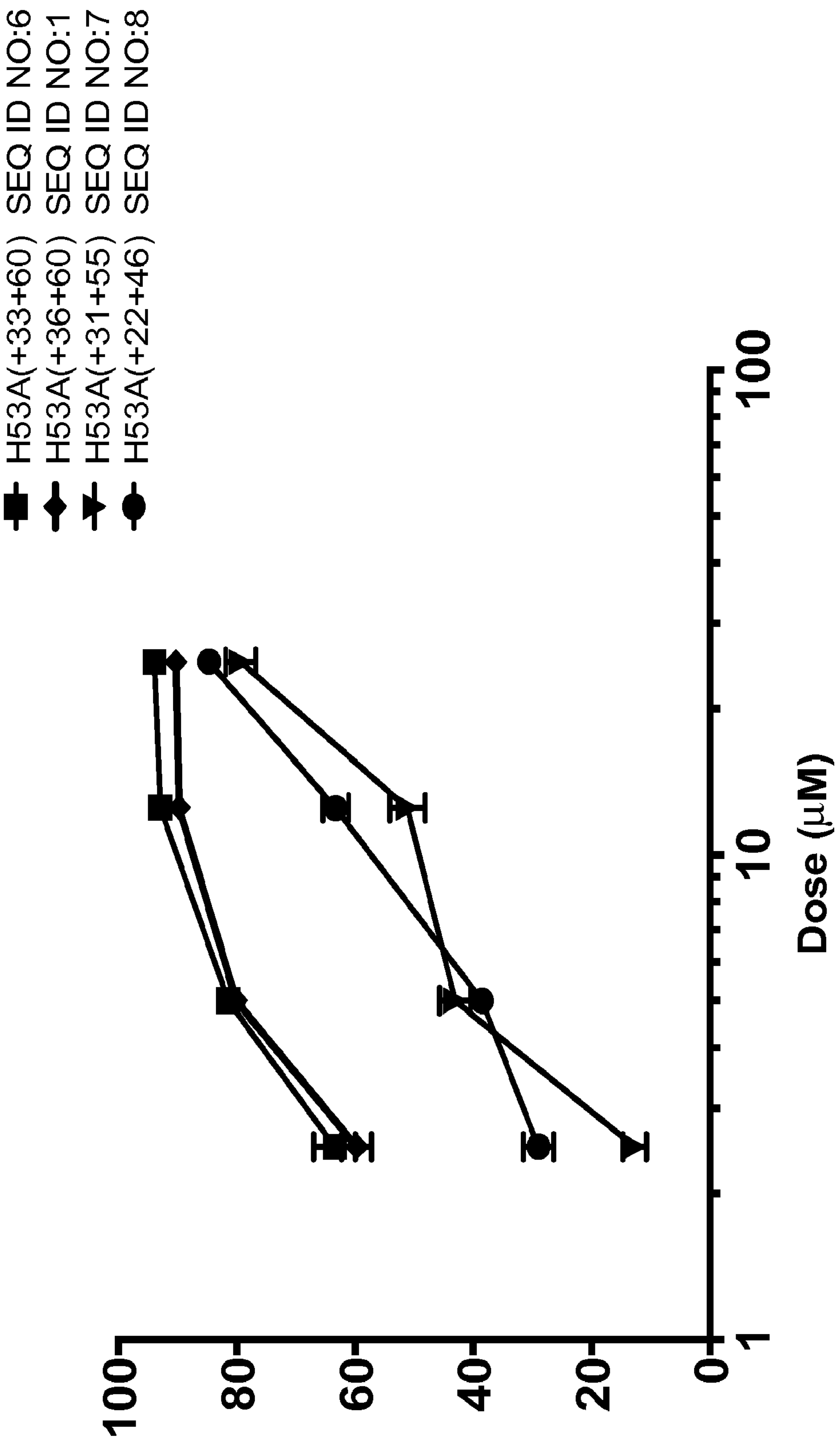
H53A(+33+60) SEQ ID NO:6
H53A(+36+60) SEQ ID NO:1
H53A(+31+55) SEQ ID NO:7
H53A(+22+46) SEQ ID NO:8
% Exon 53 Skipping
0
20
40
60
80
100
1
10
100
Dose (μM)

FIG. 3

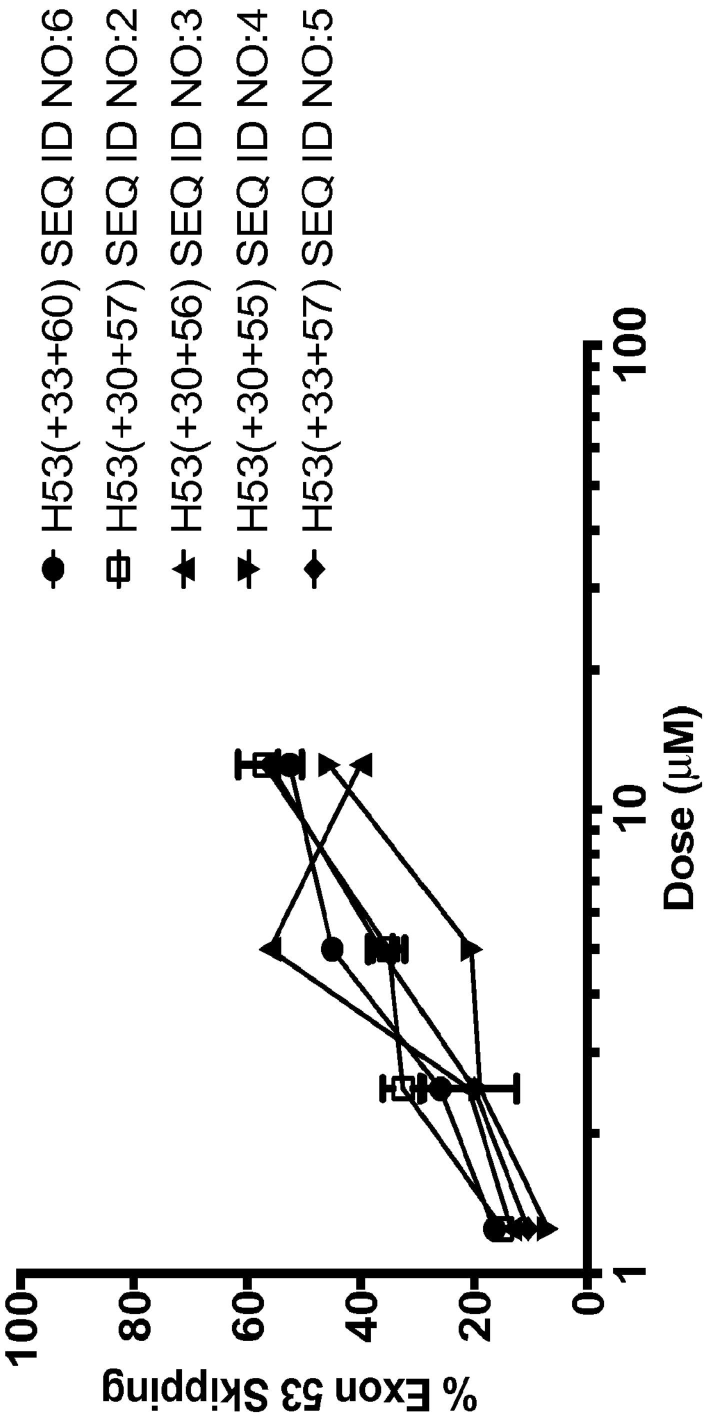
% Exon 53 Skipping
100
80
60
40
20
0
1
10
100
Dose (μM)
H53(+33+60) SEQ ID NO:6
H53(+30+57) SEQ ID NO:2
H53(+30+56) SEQ ID NO:3
H53(+30+55) SEQ ID NO:4
H53(+33+57) SEQ ID NO:5

FIG. 4

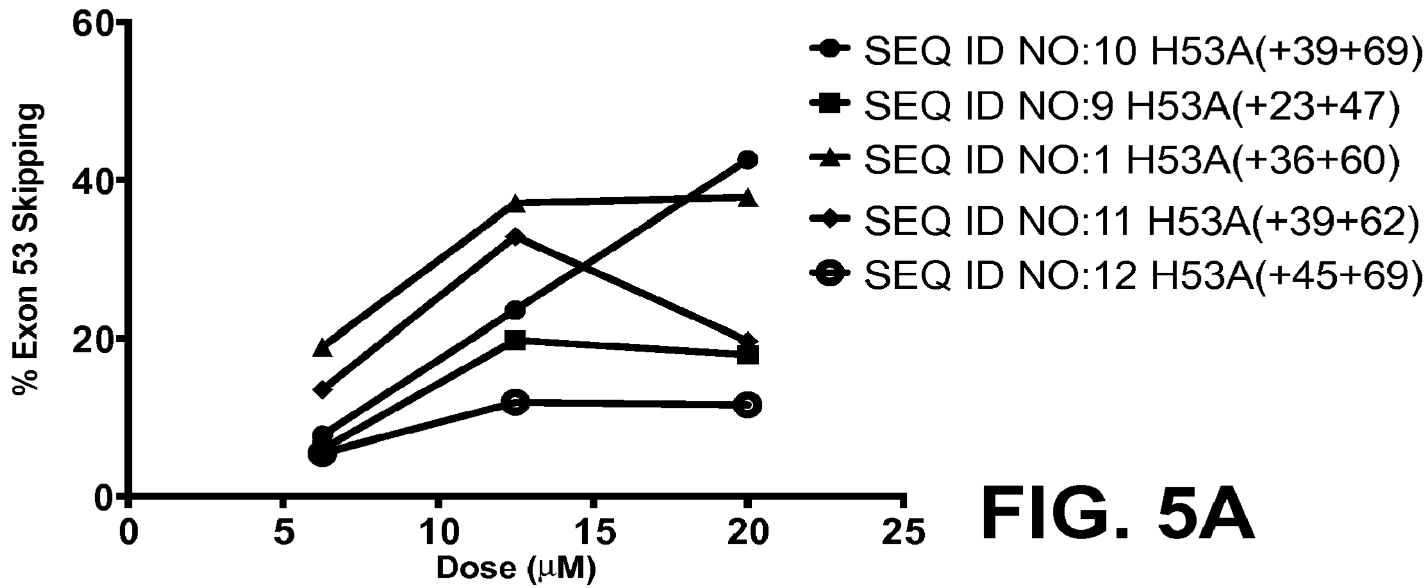
60
40
20
0
% Exon 53 Skipping
0
5
10
15
20
25
Dose (μM)
SEQ ID NO:10 H53A(+39+69)
SEQ ID NO:9 H53A(+23+47)
SEQ ID NO:1 H53A(+36+60)
SEQ ID NO:11 H53A(+39+62)
SEQ ID NO:12 H53A(+45+69)

FIG. 5A

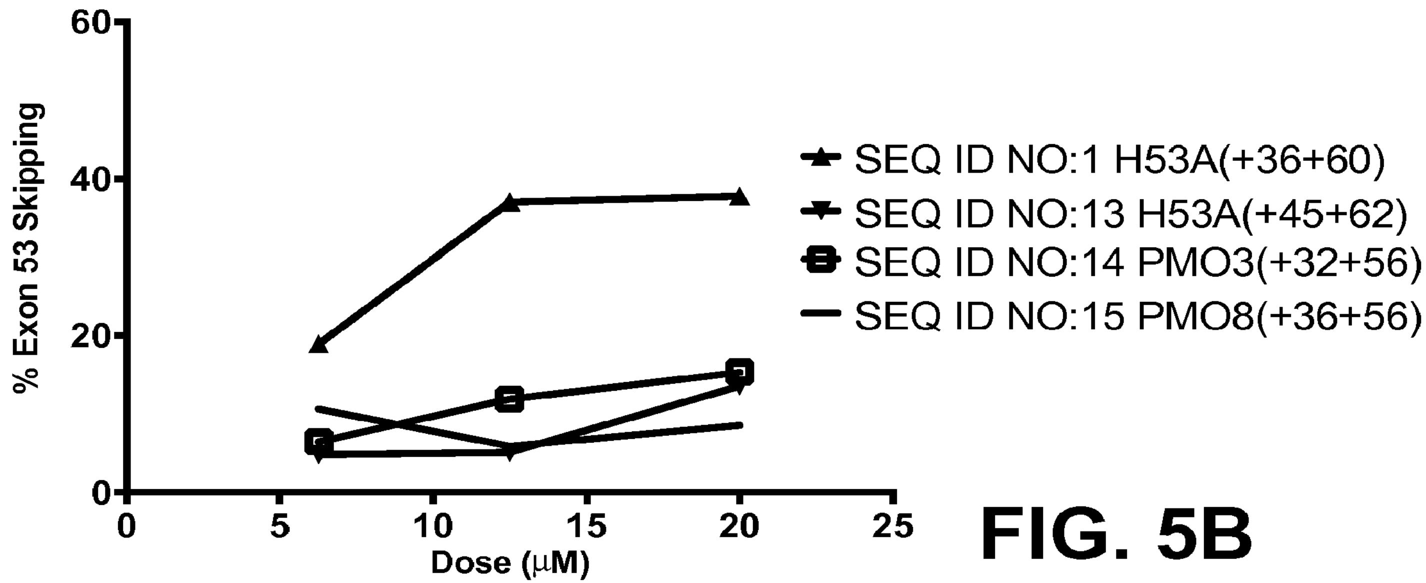
60
40
20
0
% Exon 53 Skipping
0
5
10
15
20
25
Dose (μM)
SEQ ID NO:1 H53A(+36+60)
SEQ ID NO:13 H53A(+45+62)
SEQ ID NO:14 PMO3(+32+56)
SEQ ID NO:15 PMO8(+36+56)

FIG. 5B

Europäisches Patentamt
European Patent Office
Office européen des brevets

# EUROPEAN SEARCH REPORT

Application Number
EP 20 18 2238

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | WO 2010/048586 A1 (AVI BIOPHARMA INC [US]; SAZANI PETER [US]; KOLE RYSZARD [US]) 29 April 2010 (2010-04-29) * page 41 - page 47; claims 36,39,48; sequence 429 * ----- | 1-4 | INV. C12N15/113 A61K31/7088 A61P21/00 |
| X | WO 2011/150408 A2 (AVI BIOPHARMA INC [US]; HANSON GUNNAR J [US] ET AL.) 1 December 2011 (2011-12-01) * page 95; claim 1; sequence 34 * ----- | 1-4 | |
| X | WO 2012/150960 A1 (AVI BIOPHARMA INC [US]; HANSON GUNNAR J [US]) 8 November 2012 (2012-11-08) * page 112; claims 1,35,60,63,65; sequence 34 * ----- | 1-4 | |
| X,P | WO 2013/142087 A1 (SAREPTA THERAPEUTICS INC [US]) 26 September 2013 (2013-09-26) * page 43; claims 1,2; sequence 17 * ----- | 1-4 | |
| E | WO 2014/100714 A1 (SAREPTA THERAPEUTICS INC [US]) 26 June 2014 (2014-06-26) * page 4 - page 8; example 1; sequences 2,7 * ----- | 1-4 | TECHNICAL FIELDS SEARCHED (IPC) C12N A61K A61P |
| X | WO 2012/029986 A1 (NIPPON SHINYAKU CO LTD [JP]; NAT CT OF NEUROLOGY AND PSYCHIATRY [JP];) 8 March 2012 (2012-03-08) * figure 16; sequence 47 * -& EP 2 612 917 A1 (NIPPON SHINYAKU CO LTD [JP]; NAT CT NEUROLOGY & PSYCHIATRY [JP]) 10 July 2013 (2013-07-10) * figure 16; sequence 47 * ----- -/-- | 1-4 | |

The present search report has been drawn up for all claims

1

| Place of search | Date of completion of the search | Examiner |
|---|---|---|
| The Hague | 23 November 2020 | Bucka, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 18 2238

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | POPPLEWELL L J ET AL: "Comparative analysis of antisense oligonucleotide sequences targeting exon 53 of the human DMD gene: Implications for future clinical trials", NEUROMUSCULAR DISORDERS, PERGAMON PRESS, GB, vol. 20, no. 2, 15 January 2010 (2010-01-15), pages 102-110, XP026878306, ISSN: 0960-8966 [retrieved on 2010-01-15] * table 1 * | 1-4 | |
| A | US 2010/168212 A1 (POPPLEWELL LINDA [GB] ET AL) 1 July 2010 (2010-07-01) * paragraphs [0033], [0054], [0055]; claims 1,7,11; table 1; sequences 22,23 * | 1-4 | |
| A | FOSTER H ET AL: "Genetic therapeutic approaches for Duchenne muscular dystrophy", HUMAN GENE THERAPY, MARY ANN LIEBERT, NEW YORK ,NY, US, vol. 23, no. 7, 30 May 2012 (2012-05-30), pages 676-687, XP002688790, ISSN: 1043-0342, DOI: 10.1089/HUM.2012.099 [retrieved on 2012-05-30] * page 679, right-hand column - page 681, left-hand column * | 1-4 | TECHNICAL FIELDS SEARCHED (IPC) |

-/--

The present search report has been drawn up for all claims

1

| Place of search | Date of completion of the search | Examiner |
|---|---|---|
| The Hague | 23 November 2020 | Bucka, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 18 2238

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VAN VLIET LAURA ET AL: "Assessment of the feasibility of exon 45-55 multiexon skipping for duchenne muscular dystrophy", BMC MEDICAL GENETICS, BIOMED CENTRAL, LONDON, GB, vol. 9, no. 1, 1 December 2008 (2008-12-01), page 105, XP021042784, ISSN: 1471-2350, DOI: 10.1186/1471-2350-9-105 * figure 1 * ----- | 1-4 | |
| A | JERRY R. MENDELL ET AL: "Gene therapy for muscular dystrophy: Lessons learned and path forward", NEUROSCIENCE LETTERS, vol. 527, no. 2, 17 May 2012 (2012-05-17), pages 90-99, XP055128775, ISSN: 0304-3940, DOI: 10.1016/j.neulet.2012.04.078 * page 91, right-hand column * ----- | 1-4 | TECHNICAL FIELDS SEARCHED (IPC) |
| A | MOULTON H M ET AL: "Morpholinos and their peptide conjugates: Therapeutic promise and challenge for Duchenne muscular dystrophy", BIOCHIMICA ET BIOPHYSICA ACTA (BBA) - BIOMEMBRANES, ELSEVIER, AMSTERDAM, NL, vol. 1798, no. 12, 17 February 2010 (2010-02-17), pages 2296-2303, XP027430152, ISSN: 0005-2736, DOI: 10.1016/J.BBAMEM.2010.02.012 [retrieved on 2010-02-17] * the whole document * ----- | 1-4 | |

1

The present search report has been drawn up for all claims

| Place of search | Date of completion of the search | Examiner |
|---|---|---|
| The Hague | 23 November 2020 | Bucka, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 20 18 2238

This annex lists the patent family members relating to the patent documents cited in the above-mentioned European search report. The members are as contained in the European Patent Office EDP file on
The European Patent Office is in no way liable for these particulars which are merely given for the purpose of information.

23-11-2020

| Patent document cited in search report | Publication date | Patent family member(s) | Publication date |
|---|---|---|---|
| WO 2010048586 A1 | 29-04-2010 | AU 2009308167 A1 | 29-04-2010 |
| | | BR PI0920276 A2 | 06-09-2016 |
| | | CA 2740328 A1 | 29-04-2010 |
| | | CA 3066050 A1 | 29-04-2010 |
| | | CN 102203253 A | 28-09-2011 |
| | | CN 105779453 A | 20-07-2016 |
| | | DK 3133160 T3 | 01-04-2019 |
| | | EP 2350281 A1 | 03-08-2011 |
| | | EP 2762567 A1 | 06-08-2014 |
| | | EP 3133160 A1 | 22-02-2017 |
| | | EP 3404100 A1 | 21-11-2018 |
| | | EP 3428278 A1 | 16-01-2019 |
| | | ES 2714787 T3 | 30-05-2019 |
| | | HK 1200868 A1 | 14-08-2015 |
| | | HR P20190387 T1 | 31-05-2019 |
| | | HU E042979 T2 | 29-07-2019 |
| | | IL 212386 A | 31-10-2016 |
| | | IL 246697 A | 31-12-2019 |
| | | JP 5864257 B2 | 17-02-2016 |
| | | JP 6406782 B2 | 17-10-2018 |
| | | JP 6429285 B2 | 28-11-2018 |
| | | JP 2012506703 A | 22-03-2012 |
| | | JP 2015017131 A | 29-01-2015 |
| | | JP 2016195616 A | 24-11-2016 |
| | | JP 2017086087 A | 25-05-2017 |
| | | JP 2018113989 A | 26-07-2018 |
| | | JP 2019178163 A | 17-10-2019 |
| | | KR 20110082576 A | 19-07-2011 |
| | | KR 20180011364 A | 31-01-2018 |
| | | KR 20180118828 A | 31-10-2018 |
| | | KR 20190079702 A | 05-07-2019 |
| | | LT 3133160 T | 10-04-2019 |
| | | PL 3133160 T3 | 28-06-2019 |
| | | PT 3133160 T | 18-03-2019 |
| | | SI 3133160 T1 | 31-05-2019 |
| | | TR 201902952 T4 | 21-03-2019 |
| | | TW 201018471 A | 16-05-2010 |
| | | TW 201534719 A | 16-09-2015 |
| | | TW 201712120 A | 01-04-2017 |
| | | TW 201831687 A | 01-09-2018 |
| | | US 2010130591 A1 | 27-05-2010 |
| | | US 2013190390 A1 | 25-07-2013 |
| | | US 2014094500 A1 | 03-04-2014 |
| | | US 2015152415 A1 | 04-06-2015 |
| | | US 2015376617 A1 | 31-12-2015 |
| | | US 2015376618 A1 | 31-12-2015 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 20 18 2238

This annex lists the patent family members relating to the patent documents cited in the above-mentioned European search report. The members are as contained in the European Patent Office EDP file on
The European Patent Office is in no way liable for these particulars which are merely given for the purpose of information.

23-11-2020

| Patent document cited in search report | Publication date | Patent family member(s) | Publication date |
|---|---|---|---|
| | | US 2016002633 A1 | 07-01-2016 |
| | | US 2016002634 A1 | 07-01-2016 |
| | | US 2016002637 A1 | 07-01-2016 |
| | | US 2017292125 A1 | 12-10-2017 |
| | | US 2018066259 A1 | 08-03-2018 |
| | | US 2019127738 A1 | 02-05-2019 |
| | | US 2019284556 A1 | 19-09-2019 |
| | | WO 2010048586 A1 | 29-04-2010 |
| WO 2011150408 A2 | 01-12-2011 | AU 2011257980 A1 | 10-01-2013 |
| | | AU 2016234953 A1 | 13-10-2016 |
| | | CA 2799501 A1 | 01-12-2011 |
| | | CN 103003288 A | 27-03-2013 |
| | | CN 107353317 A | 17-11-2017 |
| | | EP 2576574 A2 | 10-04-2013 |
| | | IL 223298 A | 27-02-2020 |
| | | IL 243467 A | 31-12-2017 |
| | | JP 6634424 B2 | 22-01-2020 |
| | | JP 2013530154 A | 25-07-2013 |
| | | JP 2016000059 A | 07-01-2016 |
| | | JP 2018002728 A | 11-01-2018 |
| | | JP 2020018299 A | 06-02-2020 |
| | | KR 20130118227 A | 29-10-2013 |
| | | KR 20180019751 A | 26-02-2018 |
| | | KR 20190057160 A | 27-05-2019 |
| | | KR 20200036041 A | 06-04-2020 |
| | | NZ 603606 A | 26-06-2015 |
| | | TW 201202262 A | 16-01-2012 |
| | | TW 201712023 A | 01-04-2017 |
| | | US 2012065169 A1 | 15-03-2012 |
| | | US 2015073140 A1 | 12-03-2015 |
| | | US 2017198287 A1 | 13-07-2017 |
| | | US 2019218551 A1 | 18-07-2019 |
| | | WO 2011150408 A2 | 01-12-2011 |
| WO 2012150960 A1 | 08-11-2012 | AU 2011367230 A1 | 05-12-2013 |
| | | AU 2017206179 A1 | 03-08-2017 |
| | | AU 2019204913 A1 | 25-07-2019 |
| | | CA 2834128 A1 | 08-11-2012 |
| | | CA 3092114 A1 | 08-11-2012 |
| | | CN 103619356 A | 05-03-2014 |
| | | CN 107693797 A | 16-02-2018 |
| | | EP 2704749 A1 | 12-03-2014 |
| | | IL 229227 A | 30-07-2020 |
| | | JP 6478632 B2 | 06-03-2019 |
| | | JP 2014515762 A | 03-07-2014 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 20 18 2238

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2020

| Patent document cited in search report | Publication date | Patent family member(s) | Publication date |
|---|---|---|---|
| | | JP 2016185991 A | 27-10-2016 |
| | | JP 2018135396 A | 30-08-2018 |
| | | JP 2020111607 A | 27-07-2020 |
| | | KR 20140028058 A | 07-03-2014 |
| | | KR 20190084351 A | 16-07-2019 |
| | | WO 2012150960 A1 | 08-11-2012 |
| WO 2013142087 A1 | 26-09-2013 | AU 2013235691 A1 | 16-10-2014 |
| | | AU 2017202883 A1 | 18-05-2017 |
| | | AU 2019201989 A1 | 11-04-2019 |
| | | BR 112014023347 A2 | 18-07-2017 |
| | | CA 2868174 A1 | 26-09-2013 |
| | | CN 104411831 A | 11-03-2015 |
| | | EA 201491726 A1 | 31-03-2015 |
| | | EP 2828395 A1 | 28-01-2015 |
| | | ES 2706198 T3 | 27-03-2019 |
| | | HK 1206064 A1 | 31-12-2015 |
| | | JP 6542662 B2 | 10-07-2019 |
| | | JP 2015512254 A | 27-04-2015 |
| | | KR 20140138995 A | 04-12-2014 |
| | | MX 358497 B | 23-08-2018 |
| | | NZ 700399 A | 29-07-2016 |
| | | US 2015080340 A1 | 19-03-2015 |
| | | WO 2013142087 A1 | 26-09-2013 |
| WO 2014100714 A1 | 26-06-2014 | AU 2013364158 A1 | 09-07-2015 |
| | | AU 2019253778 A1 | 07-11-2019 |
| | | CA 2894899 A1 | 26-06-2014 |
| | | CN 106414739 A | 15-02-2017 |
| | | CN 111440796 A | 24-07-2020 |
| | | EA 201591178 A1 | 30-11-2015 |
| | | EP 2935584 A1 | 28-10-2015 |
| | | HK 1216902 A1 | 09-12-2016 |
| | | JP 2016502858 A | 01-02-2016 |
| | | JP 2018110601 A | 19-07-2018 |
| | | JP 2019050834 A | 04-04-2019 |
| | | KR 20150099804 A | 01-09-2015 |
| | | US 2015361428 A1 | 17-12-2015 |
| | | US 2017369875 A1 | 28-12-2017 |
| | | US 2020339985 A1 | 29-10-2020 |
| | | WO 2014100714 A1 | 26-06-2014 |
| WO 2012029986 A1 | 08-03-2012 | AU 2011296882 A1 | 18-04-2013 |
| | | CA 2809637 A1 | 08-03-2012 |
| | | CN 103154245 A | 12-06-2013 |
| | | CY 1117367 T1 | 26-04-2017 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 20 18 2238

This annex lists the patent family members relating to the patent documents cited in the above-mentioned European search report. The members are as contained in the European Patent Office EDP file on
The European Patent Office is in no way liable for these particulars which are merely given for the purpose of information.

23-11-2020

| Patent document cited in search report | Publication date | Patent family member(s) | Publication date |
|---|---|---|---|
| | | DK 2612917 T3 | 18-04-2016 |
| | | DK 3018211 T3 | 14-10-2019 |
| | | EP 2612917 A1 | 10-07-2013 |
| | | EP 3018211 A1 | 11-05-2016 |
| | | EP 3543341 A1 | 25-09-2019 |
| | | EP 3581655 A1 | 18-12-2019 |
| | | ES 2567411 T3 | 22-04-2016 |
| | | ES 2750748 T3 | 27-03-2020 |
| | | HR P20160336 T1 | 22-04-2016 |
| | | HR P20191770 T1 | 27-12-2019 |
| | | HU E027321 T2 | 28-10-2016 |
| | | HU E046364 T2 | 28-02-2020 |
| | | JP 5363655 B2 | 11-12-2013 |
| | | JP 6141728 B2 | 07-06-2017 |
| | | JP 6193343 B2 | 06-09-2017 |
| | | JP 6465932 B2 | 06-02-2019 |
| | | JP 6647430 B2 | 14-02-2020 |
| | | JP 2014054250 A | 27-03-2014 |
| | | JP 2016104021 A | 09-06-2016 |
| | | JP 2018027083 A | 22-02-2018 |
| | | JP 2019062913 A | 25-04-2019 |
| | | JP 2020072724 A | 14-05-2020 |
| | | JP WO2012029986 A1 | 31-10-2013 |
| | | KR 20130069762 A | 26-06-2013 |
| | | LT 3018211 T | 25-10-2019 |
| | | PL 2612917 T3 | 29-07-2016 |
| | | PL 3018211 T3 | 28-02-2020 |
| | | PT 3018211 T | 28-10-2019 |
| | | RU 2013114396 A | 10-10-2014 |
| | | SI 3018211 T1 | 29-11-2019 |
| | | SM T201600111 B | 01-07-2016 |
| | | TW 201215408 A | 16-04-2012 |
| | | US 2013211062 A1 | 15-08-2013 |
| | | US 2015166995 A1 | 18-06-2015 |
| | | US 2017320903 A1 | 09-11-2017 |
| | | US 2019211049 A1 | 11-07-2019 |
| | | US 2019211050 A1 | 11-07-2019 |
| | | US 2019218244 A1 | 18-07-2019 |
| | | US 2019315796 A1 | 17-10-2019 |
| | | US 2020102343 A1 | 02-04-2020 |
| | | US 2020109162 A1 | 09-04-2020 |
| | | US 2020115411 A1 | 16-04-2020 |
| | | WO 2012029986 A1 | 08-03-2012 |
| EP 2612917 A1 | 10-07-2013 | AU 2011296882 A1 | 18-04-2013 |
| | | CA 2809637 A1 | 08-03-2012 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 20 18 2238

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2020

| Patent document cited in search report | Publication date | Patent family member(s) | Publication date |
|---|---|---|---|
| | | CN 103154245 A | 12-06-2013 |
| | | CY 1117367 T1 | 26-04-2017 |
| | | DK 2612917 T3 | 18-04-2016 |
| | | DK 3018211 T3 | 14-10-2019 |
| | | EP 2612917 A1 | 10-07-2013 |
| | | EP 3018211 A1 | 11-05-2016 |
| | | EP 3543341 A1 | 25-09-2019 |
| | | EP 3581655 A1 | 18-12-2019 |
| | | ES 2567411 T3 | 22-04-2016 |
| | | ES 2750748 T3 | 27-03-2020 |
| | | HR P20160336 T1 | 22-04-2016 |
| | | HR P20191770 T1 | 27-12-2019 |
| | | HU E027321 T2 | 28-10-2016 |
| | | HU E046364 T2 | 28-02-2020 |
| | | JP 5363655 B2 | 11-12-2013 |
| | | JP 6141728 B2 | 07-06-2017 |
| | | JP 6193343 B2 | 06-09-2017 |
| | | JP 6465932 B2 | 06-02-2019 |
| | | JP 6647430 B2 | 14-02-2020 |
| | | JP 2014054250 A | 27-03-2014 |
| | | JP 2016104021 A | 09-06-2016 |
| | | JP 2018027083 A | 22-02-2018 |
| | | JP 2019062913 A | 25-04-2019 |
| | | JP 2020072724 A | 14-05-2020 |
| | | JP WO2012029986 A1 | 31-10-2013 |
| | | KR 20130069762 A | 26-06-2013 |
| | | LT 3018211 T | 25-10-2019 |
| | | PL 2612917 T3 | 29-07-2016 |
| | | PL 3018211 T3 | 28-02-2020 |
| | | PT 3018211 T | 28-10-2019 |
| | | RU 2013114396 A | 10-10-2014 |
| | | SI 3018211 T1 | 29-11-2019 |
| | | SM T201600111 B | 01-07-2016 |
| | | TW 201215408 A | 16-04-2012 |
| | | US 2013211062 A1 | 15-08-2013 |
| | | US 2015166995 A1 | 18-06-2015 |
| | | US 2017320903 A1 | 09-11-2017 |
| | | US 2019211049 A1 | 11-07-2019 |
| | | US 2019211050 A1 | 11-07-2019 |
| | | US 2019218244 A1 | 18-07-2019 |
| | | US 2019315796 A1 | 17-10-2019 |
| | | US 2020102343 A1 | 02-04-2020 |
| | | US 2020109162 A1 | 09-04-2020 |
| | | US 2020115411 A1 | 16-04-2020 |
| | | WO 2012029986 A1 | 08-03-2012 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 20 18 2238

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2020

| Patent document cited in search report | Publication date | Patent family member(s) | Publication date |
|---|---|---|---|
| US 2010168212 A1 | 01-07-2010 | US 2010168212 A1 | 01-07-2010 |
| | | US 2012065244 A1 | 15-03-2012 |
| | | US 2012108652 A1 | 03-05-2012 |
| | | US 2012108653 A1 | 03-05-2012 |
| | | US 2013289096 A1 | 31-10-2013 |
| | | US 2014057964 A1 | 27-02-2014 |
| | | US 2016024500 A1 | 28-01-2016 |
| | | US 2017204413 A1 | 20-07-2017 |
| | | US 2019112601 A1 | 18-04-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61782706
- US 6210892 B, Bennett
- US 5627274 A
- US 5916808 A
- US 5976879 A
- US 5665593 A
- US 13118298 B
- US 5698685 A
- US 5217866 A
- US 5142047 A
- US 5034506 A
- US 5166315 A
- US 5521063 A
- US 5506337 A
- US 8076476 B
- US 8299206 B
- US 7943762 B
- US 2011038459 W
- WO 2011150408 A
- US 5149797 A
- US 6683173 A
- WO 2009127230 A
- US 4458066 A
- US 5185444 A
- US 118298
- US 20100016215 A1
- WO 2012150960 A
- US 20110212529 A
- WO 9402595 A, Sullivan
- WO 9610391 A, Choi
- WO 9610390 A, Ansell
- WO 9610392 A, Holland
- US 6692911 B
- US 7163695 B
- US 7070807 B
- US 3453259 A, Parmeter (I)
- US 3459731 A, Gramera
- US 3453257 A, Parmeter (II)
- US 3420788 A, Solms
- US 3426011 A, Parmeter (III)
- US 5134127 A, Stella
- US 4235871 A
- WO 9614057 A
- US 4737323 A
- US 8232384 B
- WO 2004083446 A
- WO 2012029986 A
- WO 2007058894 A


### Non-patent literature cited in the description


- **BROWN et al.** *Journal of Cell Science.,* 1999, vol. 112, 209-216
- **COLLINS ; MORGAN.** *Int J Exp Pathol,* 2003, vol. 84, 165-172
- **DELLORUSSO et al.** *Proc Natl Acad Sci USA,* 2002, vol. 99, 12979-12984
- **MANN et al.** *J Gen Med,* 2002, vol. 4, 644-654
- **PEACOCK H. et al.** 133. *J. Am. Chem. Soc.,* 2011, 9200
- **BEAUCAGE et al.** *Tetrahedron Letters,* 1981, vol. 22, 1859-1862
- **TALBOT et al.** *Molecular Therapy,* 2010, vol. 18 (3), 601-608
- **WANG et al.** *Gene Therapy,* 2008, vol. 15 (22), 1489-99
- **COULON et al.** *Journal of Biological Chemistry,* 2007, vol. 282 (45), 33192-33200
- **AKHTAR et al.** *Trends Cell Bio.,* 1992, vol. 2, 139
- **EMERICH, D F et al.** *Cell Transplant,* 1999, vol. 8, 47-58
- *Prog Neuropsychopharmacol Biol Psychiatry,* 1999, vol. 23, 941-949
- **LASIC et al.** *Chem. Rev.,* 1995, vol. 95, 2601-2627
- **ISHIWATA et al.** *Chem. Pharm. Bull.,* 1995, vol. 43, 1005-1011
- **LASIC et al.** *Science,* 1995, vol. 267, 1275-1276
- **OKU et al.** *Biochim. Biophys. Acta,* 1995, vol. 1238, 86-90
- **LIU et al.** *J. Biol. Chem.,* 1995, vol. 42, 24864-24870
- **BERGE et al.** Pharmaceutical Salts. *J. Pharm. Sci.,* 1977, vol. 66, 1-19
- **DORDUNOO, S. K. et al.** *Drug Development and Industrial Pharmacy,* 1991, vol. 17 (12), 1685-1713
- **SHEEN, P. C. et al.** *J Pharm Sci,* 1991, vol. 80 (7), 712-714
- **VAN UDEN et al.** *Plant Cell Tiss. Org. Cult.,* 1994, vol. 38, 1-3, 113

- **WENZ.** *Agnew. Chem. Int. Ed. Engl.,* 1994, vol. 33, 803-822
- Liposomes: A practical approach. New RRC. IRL Press, 1990, 33-104
- Liposomes from physics to applications. Lasic DD. Elsevier Science Publishers BV, 1993
- **FRIEDMANN.** *Science,* 1989, vol. 244, 1275-1280
- **ROSENBERG.** *Cancer Research,* 1991, vol. 51 (18), 5074S-5079S
- **ROSENFELD et al.** *Cell,* 1992, vol. 68, 143-155
- **ROSENFELD et al.** *Science,* 1991, vol. 252, 431-434
- **BRIGHAM et al.** *Am. J. Med. Sci.,* 1989, vol. 298, 278-281
- **NABEL et al.** *Science,* 1990, vol. 249, 1285-1288
- **HAZINSKI et al.** *Am. J. Resp. Cell Molec. Biol.,* 1991, vol. 4, 206-209
- **WANG ; HUANG.** *Proc. Natl. Acad. Sci. (USA),* 1987, vol. 84, 7851-7855
- **WU ; WU.** *J. Biol. Chem.,* 1988, vol. 263, 14621-14624
- **WOLFF et al.** *Science,* 1990, vol. 247, 1465-1468
- *Clinical Research,* 1991, 39
- **ANDERSON.** *Science,* 1992, vol. 256, 808-813
- **AARTSMA-RUS, A. ; A. A. JANSON et al.** Antisense-induced multiexon skipping for Duchenne muscular dystrophy makes more sense. *Am J Hum Genet,* 2004, vol. 74 (1), 83-92
- **CIRAK, S. ; V. ARECHAVALA-GOMEZA et al.** Exon skipping and dystrophin restoration in patients with Duchenne muscular dystrophy after systemic phosphorodiamidate morpholino oligomer treatment: an open-label, phase 2, dose-escalation study. *Lancet,* 2011, vol. 378 (9791), 595-605
- **DUNCKLEY, M. G. ; I. C. EPERON et al.** Modulation of splicing in the DMD gene by antisense oligoribonucleotides. *Nucleosides & Nucleotides,* 1997, vol. 16 (7-9), 1665-1668
- **DUNCKLEY, M. G. ; M. MANOHARAN et al.** Modification of splicing in the dystrophin gene in cultured Mdx muscle cells by antisense oligoribonucleotides. *Hum Mol Genet,* 1998, vol. 7 (7), 1083-90
- **ERRINGTON, S. J. ; C. J. MANN et al.** Target selection for antisense oligonucleotide induced exon skipping in the dystrophin gene. *J Gene Med,* 2003, vol. 5 (6), 518-27
- **GOEMANS, N. M. ; M. TULINIUS et al.** Systemic Administration of PRO051 in Duchenne's Muscular Dystrophy. *N Engl J Med.,* 2011
- **JEARAWIRIYAPAISARN, N. ; H. M. MOULTON et al.** Sustained Dystrophin Expression Induced by Peptide-conjugated Morpholino Oligomers in the Muscles of mdx Mice. *Mol Ther.,* 2008
- **KINALI, M. ; V. ARECHAVALA-GOMEZA et al.** Local restoration of dystrophin expression with the morpholino oligomer AVI-4658 in Duchenne muscular dystrophy: a single-blind, placebo-controlled, dose-escalation, proof-of-concept study. *Lancet Neurol,* 2009, vol. 8 (10), 918-28
- **LU, Q. L. ; C. J. MANN et al.** Functional amounts of dystrophin produced by skipping the mutated exon in the mdx dystrophic mouse. *Nat Med,* 2003, vol. 9 (8), 1009-14
- **MANN, C. J. ; K. HONEYMAN et al.** Improved antisense oligonucleotide induced exon skipping in the mdx mouse model of muscular dystrophy. *J Gene Med,* 2002, vol. 4 (6), 644-54
- **MARSHALL, N. B. ; S. K. ODA et al.** Arginine-rich cell-penetrating peptides facilitate delivery of antisense oligomers into murine leukocytes and alter pre-mRNA splicing. *Journal of Immunological Methods,* 2007, vol. 325 (1-2), 114-126
- **MATSUO, M. ; T. MASUMURA et al.** Exon skipping during splicing of dystrophin mRNA precursor due to an intraexon deletion in the dystrophin gene of Duchenne muscular dystrophy kobe. *J Clin Invest,* 1991, vol. 87 (6), 2127-31
- **MONACO, A. P. ; C. J. BERTELSON et al.** An explanation for the phenotypic differences between patients bearing partial deletions of the DMD locus. *Genomics,* 1988, vol. 2 (1), 90-5
- **PRAMONO, Z. A. ; Y. TAKESHIMA et al.** Induction of exon skipping of the dystrophin transcript in lymphoblastoid cells by transfecting an antisense oligodeoxynucleotide complementary to an exon recognition sequence. *Biochem Biophys Res Commun,* 1996, vol. 226 (2), 445-9
- **SIERAKOWSKA, H. ; M. J. SAMBADE et al.** Repair of thalassemic human beta-globin mRNA in mammalian cells by antisense oligonucleotides. *Proc Natl Acad Sci U S A,* 1996, vol. 93 (23), 12840-4
- **SUMMERTON, J. ; D. WELLER.** Morpholino antisense oligomers: design, preparation, and properties. *Antisense Nucleic Acid Drug Dev,* 1997, vol. 7 (3), 187-95
- **TAKESHIMA, Y. ; H. NISHIO et al.** Modulation of in vitro splicing of the upstream intron by modifying an intra-exon sequence which is deleted from the dystrophin gene in dystrophin Kobe. *J Clin Invest,* 1995, vol. 95 (2), 515-20
- **VAN DEUTEKOM, J. C. ; M. BREMMER-BOUT et al.** Antisense-induced exon skipping restores dystrophin expression in DMD patient derived muscle cells. *Hum Mol Genet,* 2001, vol. 10 (15), 1547-54
- **VAN DEUTEKOM, J. C. ; A. A. JANSON et al.** Local dystrophin restoration with antisense oligonucleotide PRO051. *N Engl J Med,* 2007, vol. 357 (26), 2677-86

- **WILTON, S. D. ; A. M. FALL et al.** Antisense oligonucleotide-induced exon skipping across the human dystrophin gene transcript. *Mol Ther,* 2007, vol. 15 (7), 1288-96
- **WILTON, S. D. ; F. LLOYD et al.** Specific removal of the nonsense mutation from the mdx dystrophin mRNA using antisense oligonucleotides. *Neuromuscul Disord,* 1999, vol. 9 (5), 330-8
- **WU, B. ; H. M. MOULTON et al.** Effective rescue of dystrophin improves cardiac function in dystrophin-deficient mice by a modified morpholino oligomer. *Proc Natl Acad Sci U S A,* 2008, vol. 105 (39), 14814-9
- **YIN, H. ; H. M. MOULTON et al.** Cell-penetrating peptide-conjugated antisense oligonucleotides restore systemic muscle and cardiac dystrophin expression and function. *Hum Mol Genet,* 2008, vol. 17 (24), 3909-18

SEQUENCE LISTING

<110> Sarepta Therapeutics, Inc.

<120> Exon skipping compositions for treating muscular dystrophy

<130> P108026EPAA

<150> PCT/US2014/029766
<151> 2014-03-14

<150> US 61/782,706
<151> 2013-03-14

<160> 35

<170> PatentIn version 3.5

<210> 1
<211> 25
<212> DNA
<213> Artificial Sequence

<220>
<223> Synthetic oligonucleotide H53A(+36+60)

<400> 1
gttgcctccg gttctgaagg tgttc 25

<210> 2
<211> 28
<212> DNA
<213> Artificial Sequence

<220>
<223> Synthetic oligonucleotide H53A(+30+57)

<400> 2
gcctccggtt ctgaaggtgt tcttgtac 28

<210> 3
<211> 27
<212> DNA
<213> Artificial Sequence

<220>
<223> Synthetic oligonucleotide H53A(+30+56)

<400> 3
cctccggttc tgaaggtgtt cttgtac 27

<210> 4
<211> 26
<212> DNA
<213> Artificial Sequence

<220>
<223> Synthetic oligonucleotide H53A(+30+55)

<400> 4

ctccggttct gaaggtgttc ttgtac 26

<210> 5
<211> 25
<212> DNA
<213> Artificial Sequence

<220>
<223> Synthetic oligonucleotide H53A(+33+57)

<400> 5
gcctccggtt ctgaaggtgt tcttg 25

<210> 6
<211> 28
<212> DNA
<213> Artificial Sequence

<220>
<223> Synthetic oligonucleotide H53A(+33+60)

<400> 6
gttgcctccg gttctgaagg tgttcttg 28

<210> 7
<211> 25
<212> DNA
<213> Artificial Sequence

<220>
<223> Synthetic oligonucleotide H53A(+31+55)

<400> 7
ctccggttct gaaggtgttc ttgta 25

<210> 8
<211> 25
<212> DNA
<213> Artificial Sequence

<220>
<223> Synthetic oligonucleotide H53A(+22+46)

<400> 8
tgaaggtgtt cttgtacttc atccc 25

<210> 9
<211> 25
<212> DNA
<213> Artificial Sequence

<220>
<223> Synthetic oligonucleotide H53A(+23+47)

<400> 9
ctgaaggtgt tcttgtactt catcc 25

<210> 10
<211> 31
<212> DNA
<213> Artificial Sequence

<220>
<223> Synthetic oligonucleotide H53A(+39+69)

<400> 10
cattcaactg ttgcctccgg ttctgaaggt g 31

<210> 11
<211> 24
<212> DNA
<213> Artificial Sequence

<220>
<223> Synthetic oligonucleotide H53A(+39+62)

<400> 11
ctgttgcctc cggttctgaa ggtg 24

<210> 12
<211> 25
<212> DNA
<213> Artificial Sequence

<220>
<223> Synthetic oligonucleotide H53A(+45+69)

<400> 12
cattcaactg ttgcctccgg ttctg 25

<210> 13
<211> 18
<212> DNA
<213> Artificial Sequence

<220>
<223> Synthetic oligonucleotide H53A(+45+62)

<400> 13
ctgttgcctc cggttctg 18

<210> 14
<211> 25
<212> DNA
<213> Artificial Sequence

<220>
<223> Synthetic oligonucleotide PMO3(+32+56)

<400> 14
cctccggttc tgaaggtgtt cttgt 25

<210> 15
<211> 21
<212> DNA

<213>  Artificial Sequence

<220>
<223>  Synthetic oligonucleotide PMO8(+36+56)

<400>  15
cctccggttc tgaaggtgtt c                                    21

<210>  16
<211>  9
<212>  PRT
<213>  Artificial Sequence

<220>
<223>  Synthetic peptide rTAT

<400>  16

Arg Arg Arg Gln Arg Arg Lys Lys Arg
1               5

<210>  17
<211>  9
<212>  PRT
<213>  Artificial Sequence

<220>
<223>  Synthetic peptide Tat

<400>  17

Arg Lys Lys Arg Arg Gln Arg Arg Arg
1               5

<210>  18
<211>  11
<212>  PRT
<213>  Artificial Sequence

<220>
<223>  Synthetic peptide R9F2

<400>  18

Arg Arg Arg Arg Arg Arg Arg Arg Arg Phe Phe
1               5                   10

<210>  19
<211>  11
<212>  PRT
<213>  Artificial Sequence

<220>
<223>  Synthetic peptide R5F2R4

<400>  19

Arg Arg Arg Arg Arg Phe Phe Arg Arg Arg Arg
1               5                   10

<210> 20
<211> 4
<212> PRT
<213> Artificial Sequence

<220>
<223> Synthetic peptide R4

<400> 20

Arg Arg Arg Arg
1

<210> 21
<211> 5
<212> PRT
<213> Artificial Sequence

<220>
<223> Synthetic peptide R5

<400> 21

Arg Arg Arg Arg Arg
1 5

<210> 22
<211> 6
<212> PRT
<213> Artificial Sequence

<220>
<223> Synthetic peptide R6

<400> 22

Arg Arg Arg Arg Arg Arg
1 5

<210> 23
<211> 7
<212> PRT
<213> Artificial Sequence

<220>
<223> Synthetic peptide R7

<400> 23

Arg Arg Arg Arg Arg Arg Arg
1 5

<210> 24
<211> 8
<212> PRT
<213> Artificial Sequence

<220>
<223> Synthetic peptide R8

<400> 24

Arg Arg Arg Arg Arg Arg Arg Arg
1               5

<210> 25
<211> 9
<212> PRT
<213> Artificial Sequence

<220>
<223> Synthetic peptide R9

<400> 25

Arg Arg Arg Arg Arg Arg Arg Arg Arg
1               5

<210> 26
<211> 16
<212> PRT
<213> Artificial Sequence

<220>
<223> Synthetic peptide (RX)8

<220>
<221> misc_feature
<222> (2)..(2)
<223> Xaa can be any naturally occurring amino acid

<220>
<221> misc_feature
<222> (4)..(4)
<223> Xaa can be any naturally occurring amino acid

<220>
<221> misc_feature
<222> (6)..(6)
<223> Xaa can be any naturally occurring amino acid

<220>
<221> misc_feature
<222> (8)..(8)
<223> Xaa can be any naturally occurring amino acid

<220>
<221> misc_feature
<222> (10)..(10)
<223> Xaa can be any naturally occurring amino acid

<220>
<221> misc_feature
<222> (12)..(12)
<223> Xaa can be any naturally occurring amino acid

<220>

<221> misc_feature
<222> (14)..(14)
<223> Xaa can be any naturally occurring amino acid

<220>
<221> misc_feature
<222> (16)..(16)
<223> Xaa can be any naturally occurring amino acid

<400> 26

Arg Xaa Arg Xaa Arg Xaa Arg Xaa Arg Xaa Arg Xaa Arg Xaa Arg Xaa
1 5 10 15

<210> 27
<211> 12
<212> PRT
<213> Artificial Sequence

<220>
<223> Synthetic peptide RAhxR)4; (P007)

<220>
<221> MOD_RES
<222> (2)..(2)
<223> Acp

<220>
<221> MOD_RES
<222> (5)..(5)
<223> Acp

<220>
<221> MOD_RES
<222> (8)..(8)
<223> Acp

<220>
<221> MOD_RES
<222> (11)..(11)
<223> Acp

<400> 27

Arg Xaa Arg Arg Xaa Arg Arg Xaa Arg Arg Xaa Arg
1 5 10

<210> 28
<211> 15
<212> PRT
<213> Artificial Sequence

<220>
<223> Synthetic peptide (RAhxR)5; (CP04057)

<220>
<221> MOD_RES
<222> (2)..(2)
<223> Acp

<220>
<221> MOD_RES
<222> (5)..(5)
<223> Acp

<220>
<221> MOD_RES
<222> (8)..(8)
<223> Acp

<220>
<221> MOD_RES
<222> (11)..(11)
<223> Acp

<220>
<221> MOD_RES
<222> (14)..(14)
<223> Acp

<400> 28

Arg Xaa Arg Arg Xaa Arg Arg Xaa Arg Arg Xaa Arg Arg Xaa Arg
1 5 10 15

<210> 29
<211> 12
<212> PRT
<213> Artificial Sequence

<220>
<223> Synthetic peptide (RAhxRRBR)2; (CP06062)

<220>
<221> MOD_RES
<222> (2)..(2)
<223> Acp

<220>
<221> MOD_RES
<222> (5)..(5)
<223> bAla

<220>
<221> MOD_RES
<222> (8)..(8)
<223> Acp

<220>
<221> MOD_RES
<222> (11)..(11)
<223> bAla

<400> 29

Arg Xaa Arg Arg Xaa Arg Arg Xaa Arg Arg Xaa Arg
1 5 10

<210> 30

<211> 14
<212> PRT
<213> Artificial Sequence

<220>
<223> Synthetic peptide (RAR)4F2

<400> 30

Arg Ala Arg Arg Ala Arg Arg Ala Arg Arg Ala Arg Phe Phe
1 5 10

<210> 31
<211> 14
<212> PRT
<213> Artificial Sequence

<220>
<223> Synthetic peptide (RGR)4F2

<400> 31

Arg Gly Arg Arg Gly Arg Arg Gly Arg Arg Gly Arg Phe Phe
1 5 10

<210> 32
<211> 23
<212> DNA
<213> Artificial Sequence

<220>
<223> Primer, outer forward

<400> 32
cttggacaga acttaccgac tgg 23

<210> 33
<211> 23
<212> DNA
<213> Artificial Sequence

<220>
<223> Primer, outer reverse

<400> 33
gtttcttcca aagcagcctc tcg 23

<210> 34
<211> 21
<212> DNA
<213> Artificial Sequence

<220>
<223> Primer, inner forward

<400> 34
gcaggatttg gaacagaggc g 21

```
<210>  35
<211>  23
<212>  DNA
<213>  Artificial Sequence

<220>
<223>  Primer, inner reverse

<400>  35
catctacatt tgtctgccac tgg                                    23
```